# FASCICULE DE BREVET EUROPEEN

(11) **EP 2 004 759 B1**
(45) Date de publication et mention de la délivrance du brevet: **11.05.2016**
(21) Numéro de dépôt: 07731820.2
(22) Date de dépôt: 23.03.2007
(51) Int. Cl.: C09B 23/14, C09B 49/12, A61Q 5/08, A61K 8/49, A61Q 5/10

(54) **PROCEDE DE COLORATION ET D'ECLAIRCISSEMENT DES MATIERES KERATINIQUES EN PRESENCE D'UN AGENT REDUCTEUR COMPRENANT UN COLORANT DISULFURE FLUORESCENT**
VERFAHREN ZUR FÄRBUNG UND AUFHELLUNG VON KERATINMATERIALIEN BEI VORHANDENSEIN EINES REDUKTIONSMITTELS MIT EINEM DISULFID-LEUCHTFARBSTOFF
METHOD OF DYEING AND LIGHTENING KERATIN MATERIALS IN THE PRESENCE OF A REDUCING AGENT COMPRISING A FLUORESCENT DISULPHIDE DYE

(30) Priorité: 24.03.2006 FR 0651035; 19.04.2006 US 792941 P
(43) Date de publication de la demande: 24.12.2008
(60) Demande divisionnaire: 14167009.1
(73) Titulaire: L'Oréal, 75008 Paris (FR)
(72) Inventeur: DAUBRESSE, Nicolas, F-78170 La Celles St Cloud (FR); GREAVES, Andrew, F-77144 Montevrain (FR); SAMAIN, Henri, F-91570 Bievres (FR)
(74) Mandataire: Fevrier, Murielle Françoise E.
(86) Numéro de dépôt international: PCT/FR2007/051008
(87) Numéro de publication internationale: WO 2007/110542

(56) Documents cités:
- EP-A- 1 647 580
- WO-A-03/028685
- WO-A-2006/134043
- WO-A2-2004/091473
- WO-A2-2005/097051
- US-A- 2 904 385
- TSUBOI K ET AL: "Formation of Merocyanine Self-Assembled Monolayer and Its Nonlinear Optical Properties Probed by Second-Harmonic Generation and Surface Plasmon Resonance" JAPANESE JOURNAL OF APPLIED PHYSICS, JAPAN SOCIETY OF APPLIED PHYSICS, TOKYO, JP, vol. 42, no. 2A, février 2003 (2003-02), pages 607-613, XP002355458 ISSN: 0021-4922
- NARAOKAA R ET AL: "Nonlinear optical property of hemicyanine self-assembled monolayers on gold and its adsorption kinetics probed by optical second-harmonic generation and surface plasmon resonance spectroscopy" CHEMICAL PHYSICS LETTERS, NORTH-HOLLAND, AMSTERDAM, NL, vol. 362, no. 1-2, 13 août 2002 (2002-08-13), pages 26-30, XP002355459 ISSN: 0009-2614
- KAJIKAWA K ET AL: "PREPARATION AND OPTICAL CHARACTERIZATION OF HEMICYANINE SELF-ASSEMBLED MONOLAYER ON AU SUBSTRATE" MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, vol. 370, 2001, pages 277-283, XP008056026 ISSN: 1058-725X

## Description

L'invention a pour objet un procédé de coloration et d'éclaircissement des matières kératiniques en présence d'un agent réducteur.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader les fibres et d'altérer leurs propriétés cosmétiques. Les fibres ont en effet tendance à devenir rêches, plus difficilement démêlables et plus fragiles. Enfin, l'éclaircissement ou la décoloration de fibres kératiniques à partir d'agent oxydant est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans le document FR 2830189 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques. Sont ainsi décrits certains colorants portant des fonctions sels de Bunte et isothiuroniums, ou d'autres groupements protecteurs de thiols. Cependant, l'obtention de la forme réactive du colorant nécessite en général l'utilisation de milieux fortement basiques. De plus, les fonctions thiols sont généralement générées en excès ce qui rend nécessaire une étape de post neutralisation à la suite de la coloration.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un pré-traitement réducteur du cheveu. Cependant, ces colorants peuvent occasionner des virages de couleur lors de l'application. Par ailleurs le document WO 03/028685 divulgue des colorants fluarescents directs utilisés pour éclaircir les fibres kératiniques foncées.

Enfin, le document WO 2005/097051 décrit des colorants disulfures azaimidazoliums pour la coloration directe de fibres kératiniques.

Le but de la présente invention est de fournir de nouveaux systèmes de coloration avec effet éclaircissant de matières kératiniques en particulier les fibres kératiniques humaines, notamment les cheveux, qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs et qui ne dégradent pas les fibres kératiniques et qui n'altèrent pas leurs propriétés cosmétiques.

Ce but est atteint avec la présente invention qui a pour objet un procédé de coloration de matières kératiniques foncées, consistant à appliquer sur les matières kératiniques, une composition tinctoriale, comprenant dans un milieu cosmétique approprié, au moins un colorant disulfure fluorescent par exemple choisi parmi les colorants de formules (I) et (II) suivantes :

A- (X)ₚ - Cₛₐₜ - S-S - C'ₛₐₜ - (X')ₚ' - A' (I)

A- (X)ₚ - Cₛₐₜ - S-S - C'ₛₐₜ - (X')ₚ' - D (II)

leurs sels d'acide organique ou minéral, isomères optiques, isomères géométriques, et les solvates tels que hydrates ;
formules dans lesquelles :
- A, et A', identiques ou différents, représentent un radical contenant au moins un chromophore fluorescent cationique ou non;
- X et X', identiques ou différents, représentent une chaîne hydrocarbonée en C₁-C₃₀, linéaire ou ramifiée, saturée ou insaturée, éventuellement interrompue et/ou éventuellement terminée à l'une ou deux de ses extrémités par un ou plusieurs groupes divalents ou leur combinaisons choisis parmi :
   - -N(R)-, -N⁺(R)(R')-, -O-, -S-, -CO-, -SO₂- avec R, R', identiques ou différents, sont choisis parmi un hydrogène, un radical alkyle en C₁-C₄, hydroxyalkyle et aminoalkyle
   - un radical (hétéro)cyclique aromatique ou non, saturé ou insaturé, condensé ou non comprenant éventuellement un ou plusieurs hétéroatomes identiques ou non, éventuellement substitué ;
- le coefficient p, et p', identiques ou différents représentent un entier égal à 0 ou 1 ;
- Cₛₐₜ, C'ₛₐₜ, identiques ou différents représentent une chaîne alkylène en C₁-C₁₈, linéaire ou ramifiée, éventuellement substituée, éventuellement cyclique ;
- D correspond à un radical choisi parmi les radicaux hydroxy, hydroxyalkyle, alcoxy, carboxyliques, carboxylates, amino, alkylamino, ou dialkylamino,
cette application est réalisée en présence d'un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol.

Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Le procédé de l'invention permet d'obtenir un éclaircissement des matières kératiniques foncées. Notamment le procédé de l'invention permet d'obtenir un éclaircissement des fibres kératiniques telle que les cheveux, très tenaces aux shampooings, aux agressions courantes (soleil, transpiration), et aux autres traitements capillaires sans dégrader la fibre kératinique.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la « hauteur de ton » qui caractérise le degré ou le niveau d'éclaircissement. La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond clair clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit, après application sur des cheveux, par exemple châtains, amener aux résultats ci-dessous.
- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 450 à 700 nanomètres supérieure à la courbe correspondant aux cheveux non traités.
- Cela signifie que, dans la gamme de longueur d'onde allant de 450 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 450 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 450 à 650 nanomètres, et de préférence dans la gamme de longueur d'onde allant de 450 à 620 nanomètres.

Au sens de la présente invention, et à moins qu'une indication différente ne soit donnée :
- un colorant disulfure fluorescent est un composé fluorescent comportant au moins un chromophore fluorescent tel que défini ci après, et comprenant une ou plusieurs liaisons disulfures S-S entre deux atomes de carbones, directement ou indirectement reliées au(x) chromophore(s) fluorescent(s) du composé, préférentiellement la liaison est susceptible d'être réduite dans un milieu cosmétiquement acceptable ;
- les radicaux « aryles » ou « hétéroaryles » ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄ , éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxyle ;
   - un radical alcoxy en C₁-C₂ ; un radical (poly)-hydroxyalcoxy en C₂-C₄ ;
   - un radical amino ;
   - un radical héterocycloalkyle à 5 ou 6 chaînons ;
   - un radical hétéroaryle à 5 ou 6 chaînons éventuellement cationique, préférentiellement imidazolium, et éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupement hydroxyle,
      ii) un groupement amino éventuellement substitué par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
      iii) un groupement ammonium quaternaire -N⁺R'R"R"', M⁻ pour lequel R', R", R"', identiques ou différents représentent un atome d'hydrogène, ou un groupement alkyle en C₁-C₄; et M représente le contre-ion de l'acide organique, minéral ou de l'halogénure correspondant,
      iv) ou un radical hétéroaryle à 5 ou 6 chaînons éventuellement cationique, préférentiellement imidazolium, et éventuellement substitué par un radical (C₁-C₄) alkyle, préférentiellement méthyle ;

   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfonylamino (R'SO₂-NR- dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂- dans lequel les radicaux R, identiques ou non, représentent un atome d'hydrogène, un radical alkyle en C₁-C₄ éventuellement porteur d'au moins un groupement hydroxyle,
   - un radical carboxylique sous forme acide ou salifiée (de préférence avec un métal alcalin ou un ammonium, substitué ou non) ;
   - un groupement cyano (CN) ;
   - un groupement polyhalogénoalkyle, préférentiellement le trifluorométhyle (CF₃) ;
- la partie cyclique ou hétérocyclique d'un radical non aromatique peut être substituée par au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkylcarbonyloxy ((RCO-O-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkcoxycarbonyle ((RO-CO-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- un radical cyclique, hétérocyclique, ou une partie non aromatique d'un radical aryle ou hétéroaryle, peut également être substitué par un ou plusieurs groupements oxo ;
- une chaîne hydrocarbonée est insaturée lorsqu'elle comporte une ou plusieurs liaisons doubles et/ou une ou plusieurs liaisons triples ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un « radical cyclique » est un radical cycloalkyle non aromatique, mono ou polycyclique, condensé ou non, contenant de 5 à 22 atomes de carbone, pouvant comporter de 1 à plusieurs insaturations ;
- un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en C₁-C₁₆, linéaire ou ramifié, de préférence en C₁-C₈,
- l'expression « éventuellement substitué » attribué au radical alkyle sous entend que ledit radical alkyle peut être substitué par un ou plusieurs radicaux choisis parmi les radicaux i) hydroxy, ii) alcoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire -N⁺R'R"R"', M pour lequel R', R", R"', identiques ou différents représentent un atome d'hydrogène, ou un groupement alkyle en C₁-C₄, ou alors -N⁺R'R"R"' forme un hétéroaryle tel que imidazolium éventuellement substitué par un groupement C₁-C₄ alkyle, et M représente le contre-ion de l'acide organique, minéral ou de l'halogénure correspondant,
- un « radical alcoxy » est un radical alkyl-oxy pour lequel le radical alkyle est un radical hydrocarboné, linéaire ou ramifié, en C₁-C₁₆ préférentiellement en C₁-C₈ ; lorsque le groupement alcoxy est éventuellement substitué, cela sous entend que le groupe alkyle est éventuellement substitué tel que défini supra.

De plus, sauf indication contraire, les bornes délimitant l'étendue d'une plage de valeurs sont comprises dans cette plage de valeurs.

Selon la présente invention, on entend par « chromophore fluorescent» un radical issu d'un composé fluorescent. Un composé fluorescent est un composé qui est capable d'absorber dans le rayonnement UV ou visible à une longueur d'onde λ_{abs} comprise entre 250 et 800 nm et capable de réémettre dans le domaine du visible à une longueur d'onde d'émission λ_{ém} comprise entre 400 et 800 nm.

De préférence les composés fluorescents sont des colorants capables d'absorber dans le visible λ_{abs} comprise entre 400 et 800 nm et de réémettre dans le visible λ_{ém} comprise entre 400 et 800 nm. Plus préférentiellement les colorants fluorescents sont des colorants capables d'absorber à une λ_{abs} comprise entre 420 nm et 550 nm et de réémettre dans le visible à une λ_{ém} comprise entre 470 et 600 nm.

### I. Colorants de formules (I) et (II)

Les radicaux A et A' des formules (I) et (II) peuvent contenir un ou plusieurs chromophores fluorescents, identiques ou différents.

### I.1. Chromophores

Au sens de la présente invention, les chromophores sont dits différents lorsqu'ils diffèrent par leur structure chimique. De tels chromophores peuvent être des chromophores issus de familles différentes ou d'une même famille à la condition de présenter des structures chimiques différentes. Par exemple, les chromophores peuvent être choisis dans la famille des colorants (poly)méthines mais différer par la structure chimique des radicaux le constituant ou par la position respective de ces radicaux.

A titre de chromophores fluorescents utiles dans la présente invention, on peut citer les radicaux issus des colorants acridines, acridones, benzanthrones, benzimidazoles, benzimidazolones, benzindoles, benzoxazoles, benzopyranes, benzothiazoles, coumarines, difluoro{2-[(2*H*-pyrrol-2-ylidene-kN)methyl]-1H-pyrrolato-kN}bores (BODIPY ®), dipyrrinones, dicétopyrrolopyrroles, fluorindines, (poly)méthines (notamment cyanines et styryles/hémicyanines), naphthalimides, naphthanilides, naphthylamine (comme les dansyles), oxadiazoles, oxazines, périlones, périnones, pérylènes, polyènes/caroténoides, squaranes, stilbènes, xanthènes.

On peut également citer les colorants fluorescents décrits dans les documents EP 1133975, WO 03/029359, EP 860636, WO 95/01772, WO 95/15144, EP 714954 et ceux listés dans l'encyclopédie *"*The chemistry of synthetic dye" de K. VENKATARAMAN, 1952, Academic press vol 1 à 7, dans l'encyclopédie *"*Kirk Othme" "Chemical technology", chapitre "dyes and Dye intermediate", 1993, Wiley and sons, et dans divers chapitres de l'encyclopédie *"*ULLMANN's ENCYCLOPEDIA of Industrial chemistry" 7th édition, Wiley and sons, dans The Handbook - A Guide to Fluorescent Probes and Labeling Technologies, 10th Ed Molecular Probes/Invitrogen - Oregon 2005 diffusé par Internet ou dans les éditions précédentes imprimées.

De préférence, les chromophores sont choisis parmi ceux issus de colorants de type coumarines, (poly)méthines (notamment cyanines et styryles/hémicyanines) et naphthalimides.

Selon une variante, les radicaux A, et A' des formules (I) ou (II) contiennent au moins un radical cationique porté par ou inclus dans au moins un des chromophores.

De préférence, le radical cationique est un ammonium quaternaire.

Ces radicaux cationiques sont par exemple un radical alkylammonium, acridinium, benzimidazolium, benzobistriazolium, benzopyrazolium, benzopyridazinium, benzoquinolium, benzothiazolium, benzotriazolium, benzoxazolium, bi-pyridinium, bis-tétrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phénazinium, phénooxazolium, pyrazinium, pyrazolium, pyrazoyltriazolium, pyridinium, pyridinoimidazolium, pyrrolium, pyrylium , quinolium, tétrazolium, thiadiazolium, thiazolium, thiazolopyridinium, thiazoylimidazolium, thiopyrylium, triazolium ou xanthylium.

### I.1 Cₛₐₜ et C'ₛₐₜ :

Comme indiqué auparavant, dans les formules (I) ou (II), Cₛₐₜ et C'ₛₐₜ, indépendamment l'un de l'autre, représentent une chaîne alkylène en C₁-C₁₈, linéaire ou ramifiée, éventuellement substituée, éventuellement cyclique. A titre de substituant, on peut citer les groupements amino, (C₁-C₄)alkylamino, (C₁-C₄)dialkylamino, ou le groupement R^{a}-Z^{a}-C(Z^{b})- (dans laquelle Z^{a}, Z^{b}, identiques ou différents, représentent un atome d'oxygène, de soufre, ou un groupe NR^{a,}, et R^{a}, représente un métal alcalin, un atome d'hydrogène, ou un groupement C₁-C₄ alkyle et R^{a,} représente un atome d'hydrogène ou un groupement C₁-C₄ alkyle) présents de préférence sur le carbone en position béta ou gamma des atomes de soufre.

De préférence, dans le cas des formules (I) ou (II), Cₛₐₜ et C'ₛₐₜ, représentent une chaîne -(CH₂)ₖ- avec k entier, compris inclusivement entre 1 et 8.

### 1. 3. X et X':

Conformément à un mode de réalisation particulier de l'invention, dans les formules (I) ou (II), précitées, lorsque p est égal à 1, X et X', identiques ou différents, représentent la séquence suivante :

-(T)ₜ-(Z)_{z}-(T')_{t'}-

ladite séquence étant reliée dans les formules (I) ou (II) de façon symétrique comme suit :

- Cₛₐₜ (ou C'ₛₐₜ)-(T)ₜ-(Z)_{z}-(A ou A') ;

dans laquelle
**T** et **T',** identiques ou différents, représentent un ou plusieurs radicaux ou leurs combinaisons choisis parmi : -SO₂- -O- ; -S- ; -N(R)- ; -N⁺(R)(R°)- ; -CO- ; avec R, R°, identiques ou différents, représentant un atome d'hydrogène, un radical alkyle en C₁-C₄, hydroxyalkyle en C₁-C₄ ou un aryl(C₁-C₄)alkyle ; et un radical hétérocycloalkyle ou hétéroaryle, cationique ou non, préférentiellement monocyclique, contenant préférentiellement deux hétéroatomes (plus préférentiellement deux atomes d'azote) et comportant préférentiellement de 5 à 7 chaînons, plus préférentiellement imidazolium ;
les indices **t** et **t',** identiques ou différents, valent 0 ou 1 ;
**Z** représente :
   - -(CH₂)ₘ- avec m entier compris entre 1 et 8
   - -(CH₂CH₂O)_{q}- ou -(OCH₂CH₂)_{q}- dans lesquelles q est un entier compris entre 1 et 15
   - un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C₁-C₁₈ éventuellement porteurs d'au moins un hydroxyle
**z** vaut 0 ou 1.

Par ailleurs, selon un mode de réalisation particulier de l'invention, Z représente :

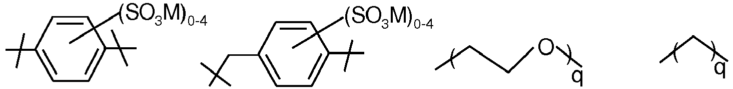

### I.4. Colorants fluorescents disulfures:

Selon une variante préférée de l'invention, le colorant disulfure est un colorant fluorescent cationique comprenant au moins un radical ammonium quaternaire et tel que, dans la formule (I) avec p égal à 1 :
**A** et **A',** identiques ou différents, plus préférentiellement identiques, représentent W-C(R^{c})=C(R^{d})-Ar- ou -W-C(R^{c})=C(R^{d})-Ar, avec W représentant un hétérocycle ou un hétéroaryle, comprenant un ammonium quaternaire ; Ar représente un radical (hétéro)aryle à 5 ou 6 chaînons de type phényle ou pyridium, ou un bicycle (hétéro)aromatique de type naphtyle, benzopyrydinium, indolinyle, ou benzoindolinyle, éventuellement substitués par un ou plusieurs atomes d'halogène, de préférence chlore, fluor ; par un ou plusieurs groupements alkyle, de préférence en C₁-C₄ ; par un ou plusieurs groupements hydroxyle ; par un ou plusieurs groupements alcoxy, par un ou plusieurs groupements hydroxyalkyle, par un ou plusieurs groupements amino ou (di)alkylamino, de préférence avec la partie alkyle en C₁-C₄, par un ou plusieurs groupements acylamino ; par un ou plusieurs groupements hétérocycloalkyle ou hétéroaryle à 5 ou 6 chaînons préférentiellement choisi parmi pyrrolidinyle, pypérazinyle, pypéridinyle et imidazolinyle; R^{c}, R^{d}, identiques ou différents, représentent un atome d'hydrogène ou un groupement alkyle en C₁-C₄.

Selon une variante préférée p, p'=1 ; z=0 ; t'=0 et t=1 et T représente -N(R)-, de préférence en position para sur Ar par rapport à la fonction oléfine -C(R^{c})=C(R^{d})-.

Selon une autre variante préférée p, p'=1 ; z=0 ; t, t'=1 ; T représente - N(R)-, de préférence en position para sur Ar par rapport à la fonction oléfine - C(R^{c})=C(R^{d})- et T' représente un groupement -N(R)-, -N⁺(R)(R°)- ou un imidazolium.

De préférence, W est un imidazolium, pyridinium, benzopyridinium, benzimidazolium, quinolinium, et pyrazolium, éventuellement substitués par un ou plusieurs radicaux alkyles, identiques ou non, en C₁-C₄.

Selon une autre variante préférée, le colorant disulfure est un colorant fluorescent cationique comprenant au moins un radical ammonium quaternaire et tel que, dans la formule (I) avec p égal à 1 :
A représentant un radical naphthalimidyle de formule : 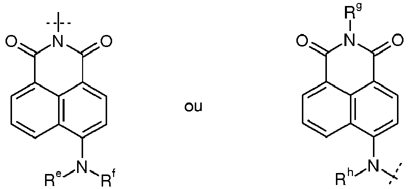 avec 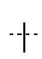 représentant la liaison avec le groupement X ou X', Cₛₐₜ ou C'ₛₐₜ
   dans lequel R^{e}, R^{f}, R^{g},et R^{h}, identiques ou différents, représentent un atome d'hydrogène ou un groupement alkyle en C₁-C₆ éventuellement substitué.

Plus préférentiellement, le colorant disulfure est un colorant fluorescent choisi parmi : 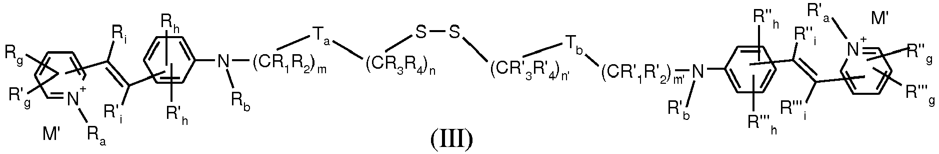 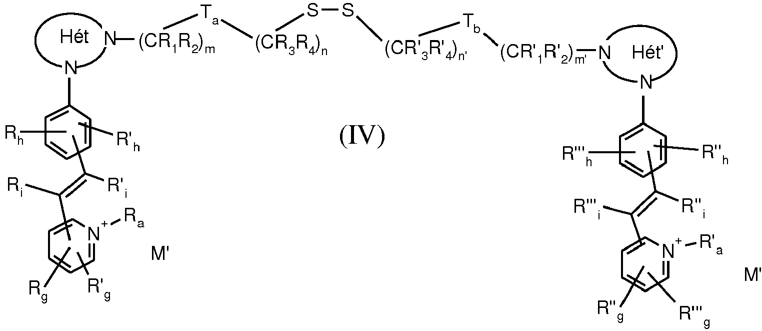 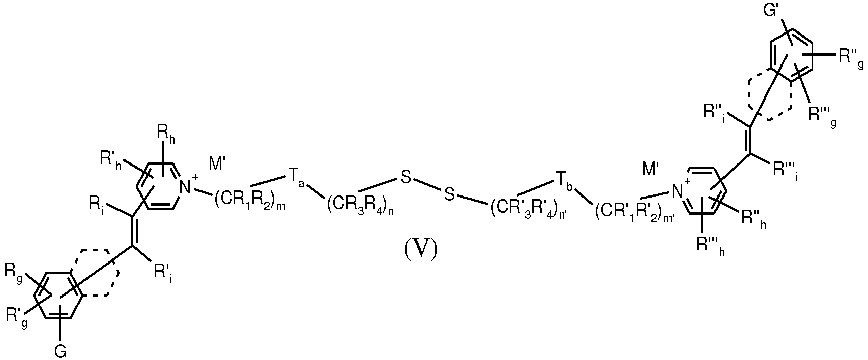 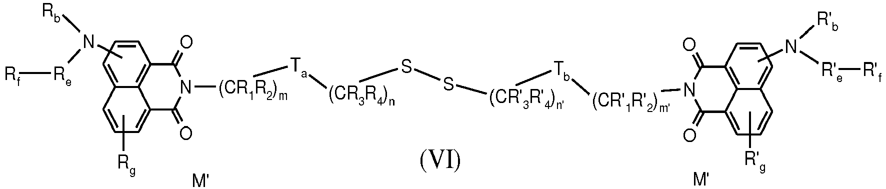 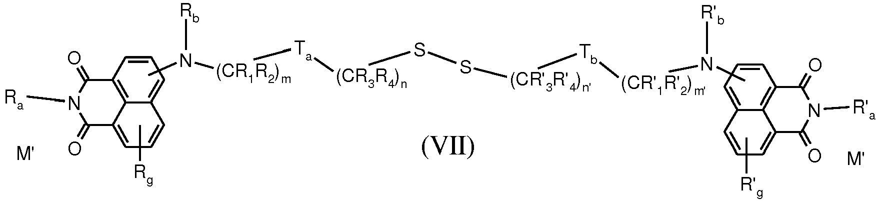 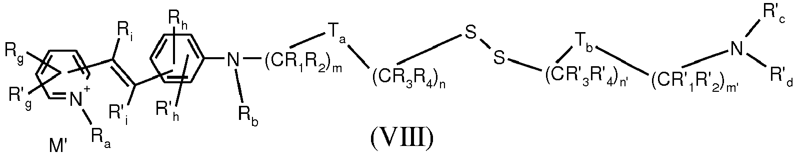 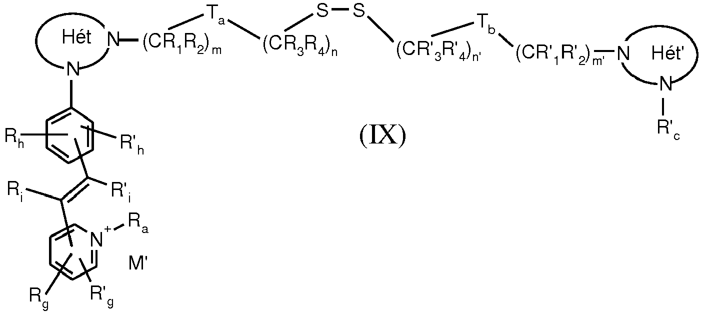 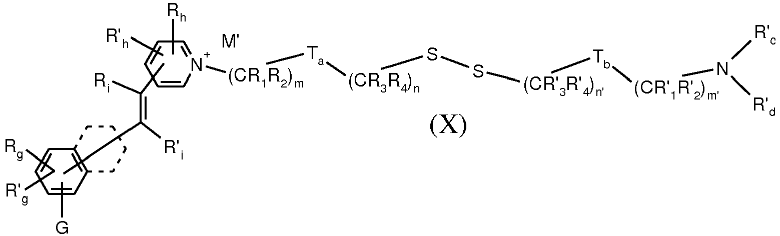 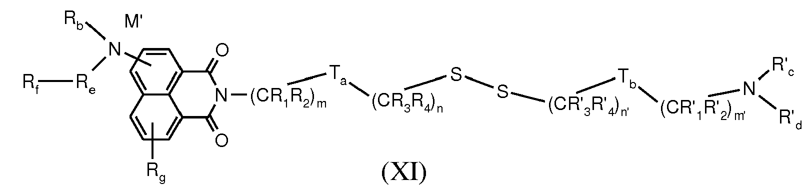 et 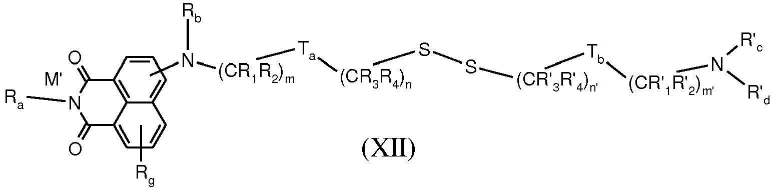 dans lequel
- G et G', identiques ou différents, représentent un groupement -NR_{c}R_{d},-NR'_{c}R'_{d}, ou C₁-C₆ alkoxy éventuellement substitué, préférentiellement non substitué ; préférentiellement G et G' représentent un groupement -NR_{c}R_{d} et-NR'_{c}R'_{d} respectivement ;
- Rₐ et R'ₐ, identiques ou différents, un groupement aryl(C₁-C₄)alkyle ou un groupement C₁-C₆ alkyle éventuellement substitué par un groupement hydroxyle ou amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement Rₐ et R'ₐ représentent un groupement C₁-C₃ alkyle éventuellement substitué par un groupement hydroxy, ou un groupement benzyle ;
- R_{b}, R'_{b}, identiques ou différents, représentent un atome d'hydrogène, un groupement aryl(C₁-C₄)alkyle ou un groupement C₁-C₆ alkyle éventuellement substitué; préférentiellement R_{b}, R'_{b} représentent un atome d'hydrogène ou un groupement C₁-C₃ alkyle ou benzyle ;
- R_{c}, R'_{c}, R_{d} et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ; R_{c}, R'_{c}, R_{d} et R'_{d} représentent préférentiellement un atome d'hydrogène, un groupement hydroxy, C₁-C₃ alcoxy, amino, C₁-C₃ (di)alkylamino, ou un groupement C₁-C₃ alkyle éventuellement substitué par i) un groupement hydroxy, ii) amino, iii) C₁-C₃ (di)alkylamino, ou iv) ammonium quaternaire (R")(R"')(R"")N⁺-;
   ou alors deux radicaux adjacents R_{c} et R_{d}, R'_{c} et R'_{d} portés par le même atome d'azote forment ensemble un groupe hétérocyclique ou hétéroaryle ; préférentiellement l'hétérocycle ou l'hétéroaryle est monocyclique et comprend entre 5 et 7 chaînons ; plus préférentiellement les groupements sont choisis parmi l'imidazolyle et le pyrrolidinyle ;
- Rₑ et R'ₑ, identiques ou différents, représentent une chaîne hydrocarbonée en C₁-C₆ alkylènyle divalente, linéaire ou ramifiée, éventuellement insaturée ;
- R_{f} et R'_{f}, identiques ou différents, représentent un groupement ammonium quaternaire (R")(R"')(R"")N⁺- où R", R'" et R"", identiques ou différents, représentent un atome d'hydrogène ou un groupement C₁-C₄ alkyle ou alors (R")(R"')(R"")N⁺- représente un groupement hétéroaryle cationique éventuellement substitué préférentiellement un groupement imidazolinium éventuellement substitué par un groupement C₁-C₃ alkyle ;
- R_{g}, R'_{g}, R"_{g}, R'"_{g} Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement R_{g}, R'_{g}, R"_{g}, R'"_{g}, Rₕ, R'ₕ, R"ₕ, et R"'ₕ représentent un atome d'hydrogène, d'halogène ou un groupement C₁-C₃ alkyle ;
- ou alors deux groupements R_{g} et R'_{g} ; R"_{g} et R'"_{g} ; Rₕ, et R'ₕ ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par : un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote; préférentiellement R_{g} et R'_{g}; R"_{g} et R'"_{g} forment ensemble un groupement benzo ;
- ou alors lorsque G représente -NR_{c}R_{d} et G' représente -NR'_{c}R'_{d} deux groupements R_{c} et R'_{g} ; R'_{c} et R"_{g} ; R_{d} et R_{g} ; R'_{d} et R"'_{g} forment ensemble un hétéroaryle ou hétérocycle saturé, éventuellement substitué par un ou plusieurs groupement C₁-C₆ alkyle, préférentiellement un l'hétérocycle contenant un ou deux hétéroatomes choisis parmi l'azote et l'oxygène et comprenant entre 5 et 7 chaînons ; plus préférentiellement l'hétérocycle est choisi parmi les groupement morpholinyle, pypérazinyle, pypéridinyle et pyrrolidinyle ;
- Rᵢ, R'ᵢ, R"ᵢ, et R"'ᵢ, identiques ou différents, représentent un atome d'hydrogène, ou un groupement C₁-C₄ alkyle ;
- R₁, R₂, R₃, R₄, R'₁, R'₂, R'₃, et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement R₁, R₂, R₃, R₄, R'₁, R'₂, R'₃, et R'₄ sont des atomes d'hydrogène ou un groupement amino ; plus préférentiellement R₁, R₂, R₃, R₄, R'₁, R'₂, R'₃, et R'₄ représentent un atome d'hydrogène;
- Tₐ, T_{b}, identiques ou différents, représentent i) soit une liaison covalente σ, ii) soit un ou plusieurs radicaux ou leurs combinaisons choisis parmi -SO₂- -O-, -S-, -N(R)-, -N⁺(R)(R°)-, -CO-, avec R, R^{o}, identiques ou différents, représentant un atome d'hydrogène, un radical alkyle en C₁-C₄, hydroxyalkyle en C₁-C₄; ou un aryl(C₁-C₄)alkyle, préférentiellement Tₐ est identique à T_{b} et représentent une liaison covalente σ ou un groupement choisi parmi -N(R)-, -C(O)-N(R)-, - N(R)-C(O)-, -O-C(O)-, -C(O)-O- et -N⁺(R)(R°)-, avec R, R° identiques ou différents représentant un atome d'hydrogène ou un groupement C₁-C₄ alkyle ; plus préférentiellement Tₐ et T_{b} représentent une liaison σ ; iii) soit un radical hétérocycloalkyle ou hétéroaryle, cationique ou non, préférentiellement monocycliques, préférentiellement identiques, contenant préférentiellement deux hétéroatomes (plus préférentiellement deux atomes d'azote) et comportant préférentiellement de 5 à 7 chaînons tel que l'imidazolium;
- 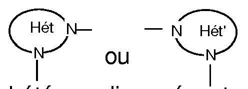 identiques ou différents, représentent une groupement hétérocyclique éventuellement substitué ; preterentiellement les hétérocycles sont identiques, monocycliques, saturés, et comprennent au total deux atomes d'azote et de 5 à 8 chaïnons ;
- 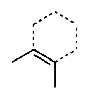 représentent un groupement aryle ou hétéroaryle fusionné au cycle phényle ; ou alors est absent du cycle phényle; préférentiellement lorsque le cycle est présent le cycle est un benzo ;
- m, m', n et n', identiques ou différents, représentent un entier compris inclusivement entre 0 et 6 avec m+n, m'+n', identiques ou différents, représente un entier compris inclusivement entre 1 et 10; préférentiellement m+n=m'+n'=un entier compris inclusivement entre 2 et 4 ; plus préférentiellement m+n=m'+n'= un entier égal à 2;

M' représentant contre-ion ou un sel d'acide organique ou minéral.

A titre d'exemple de colorants disulfures fluorescents, on peut citer notamment les composés suivants : 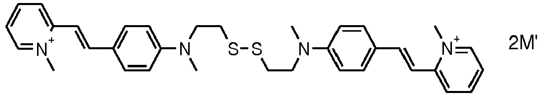 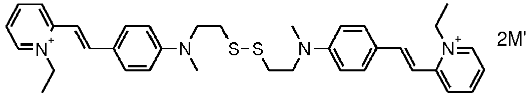 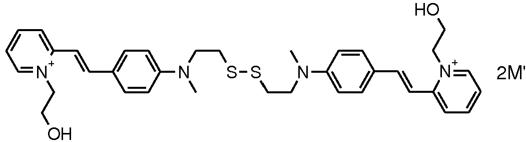 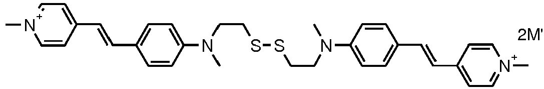 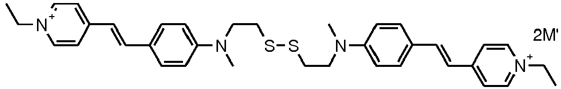 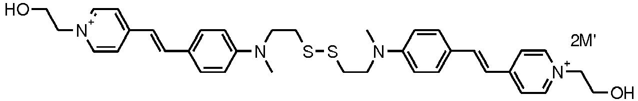 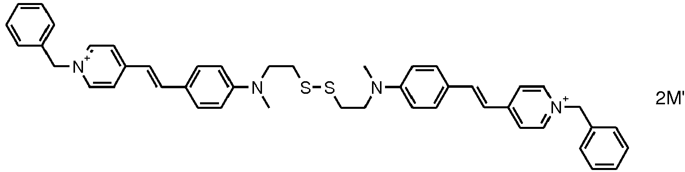 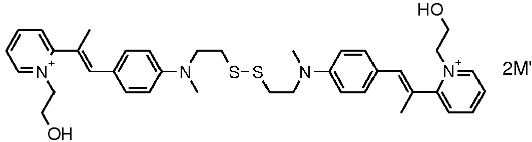 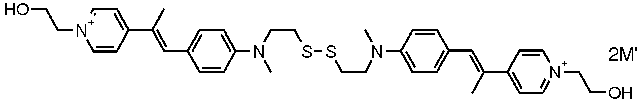 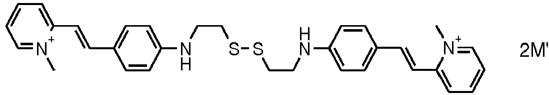 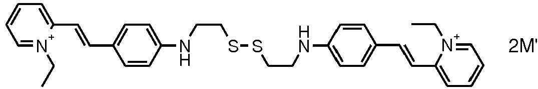 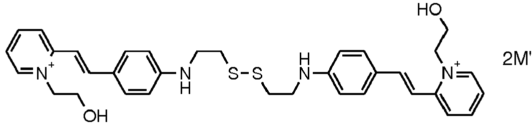 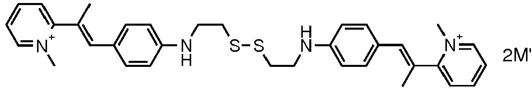 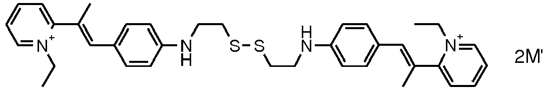 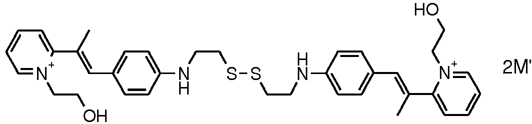 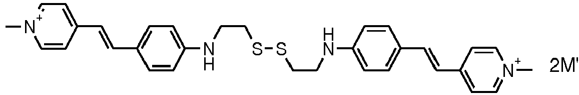 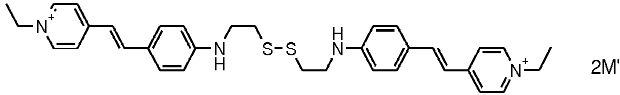 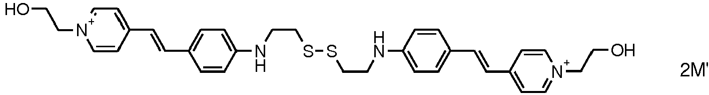 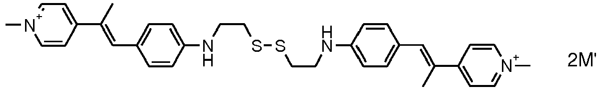 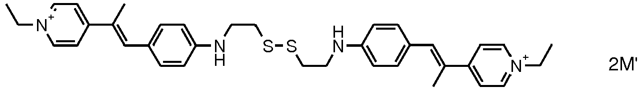 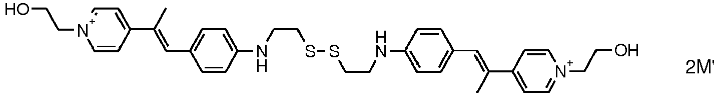 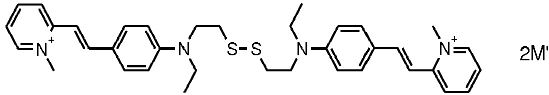 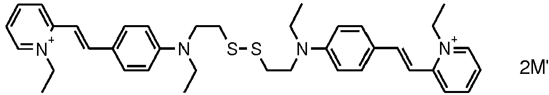 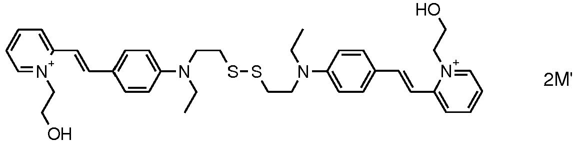 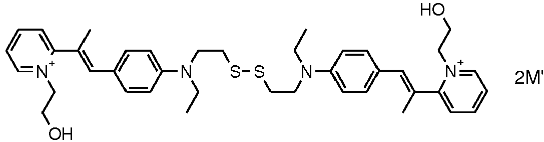 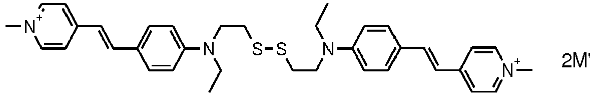 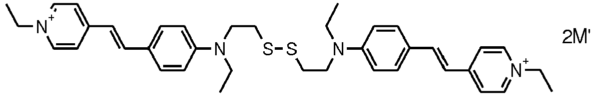 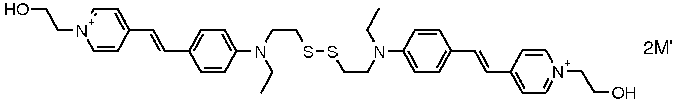 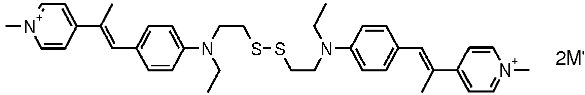 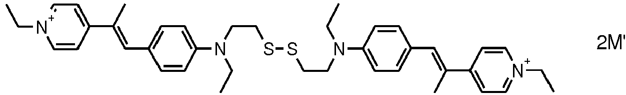 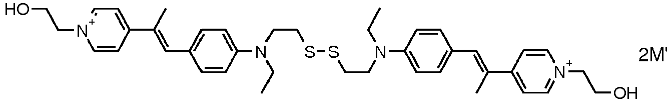 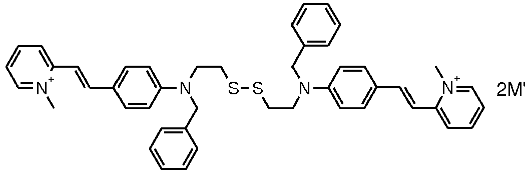 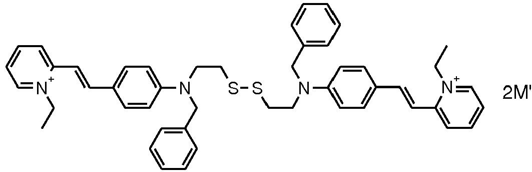 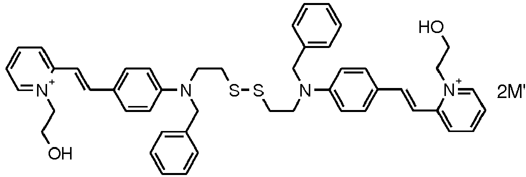 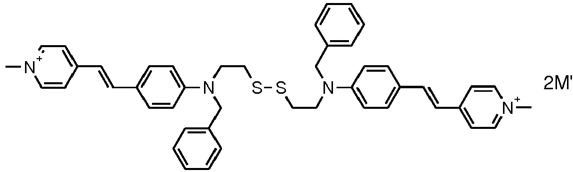 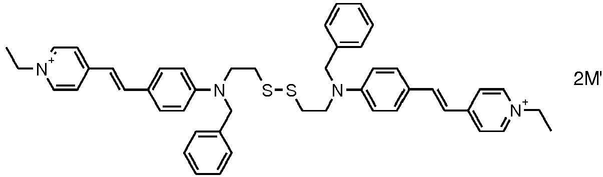 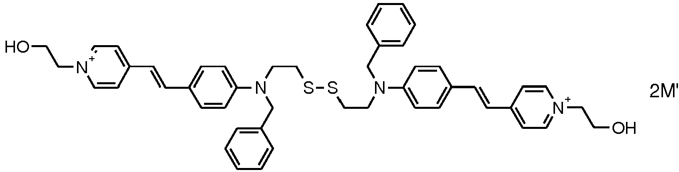 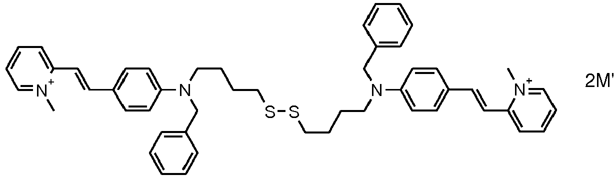 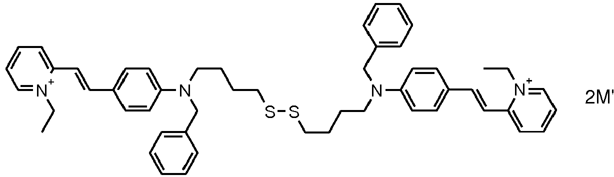 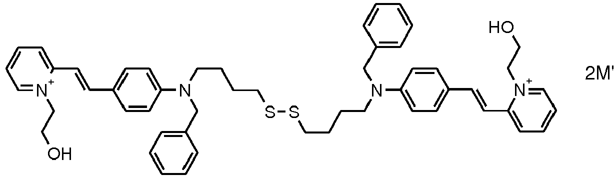 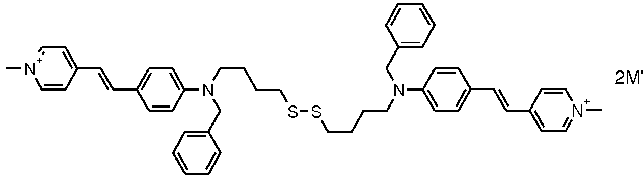 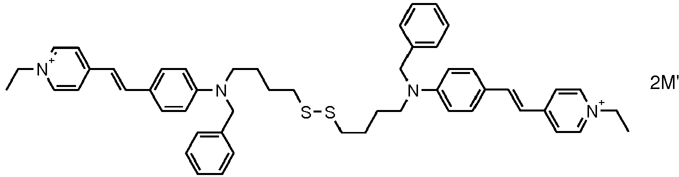 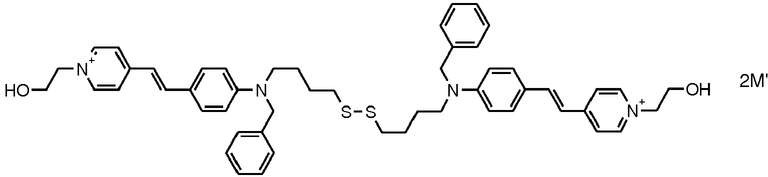 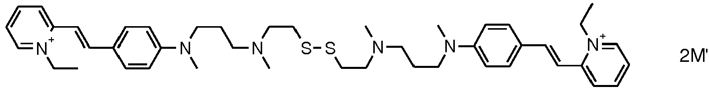 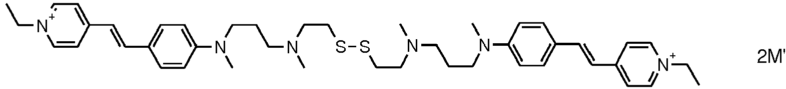 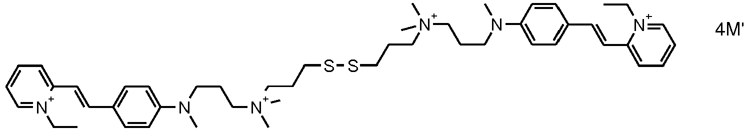 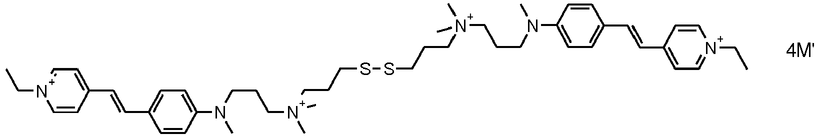 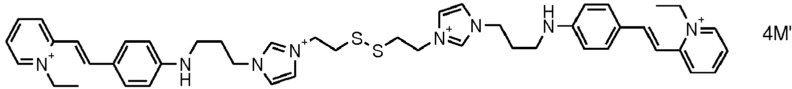 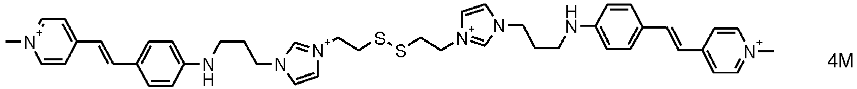 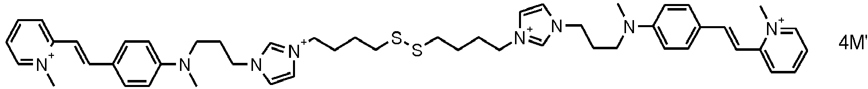 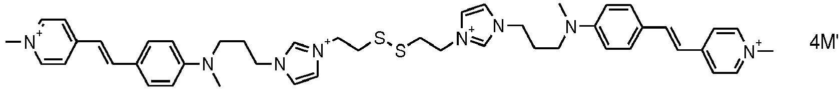 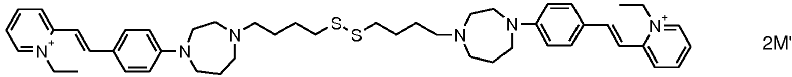 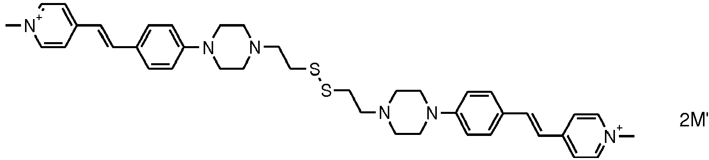 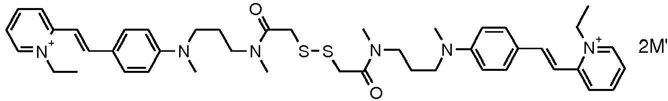 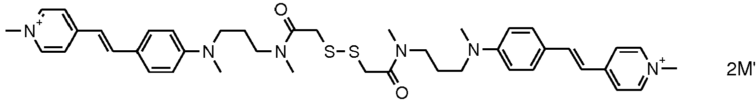 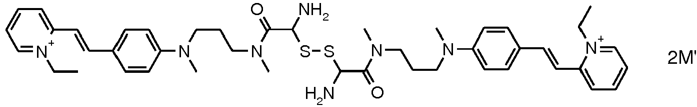 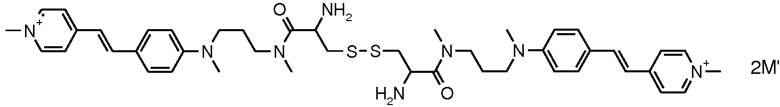 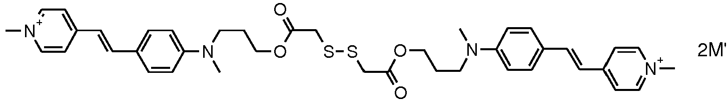 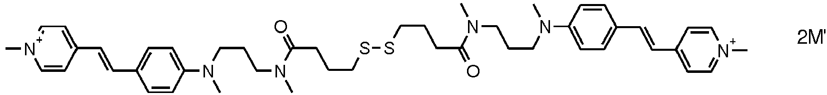 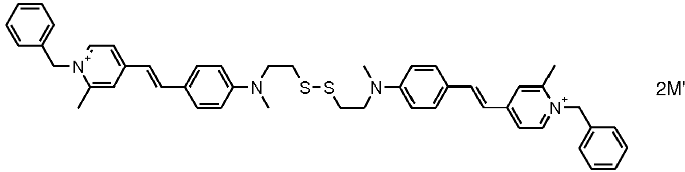 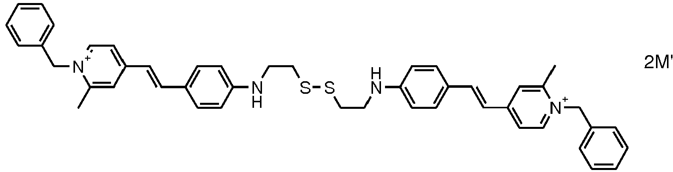 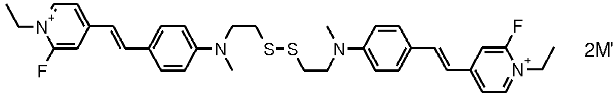 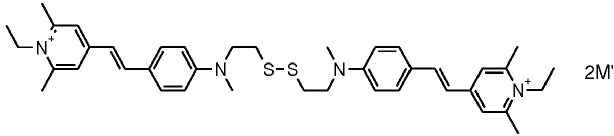 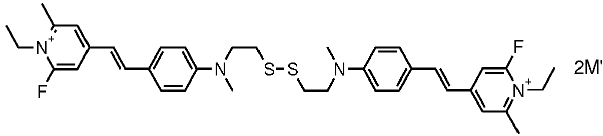 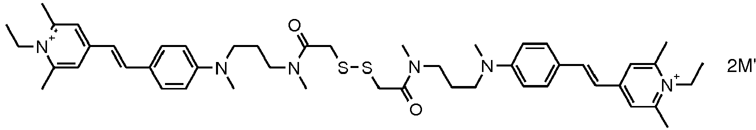 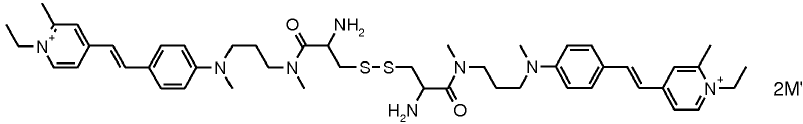 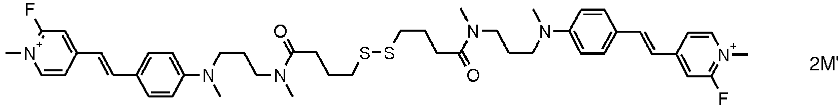 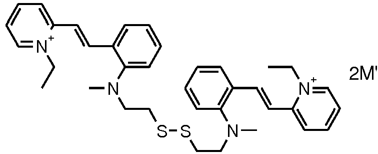 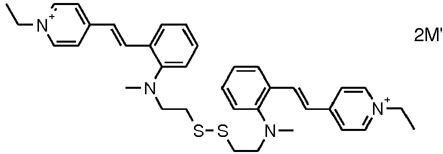 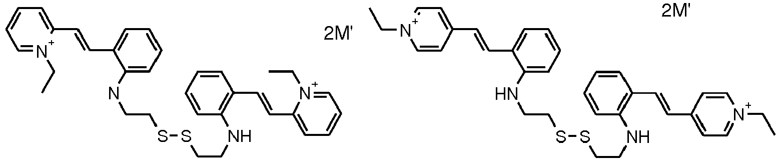 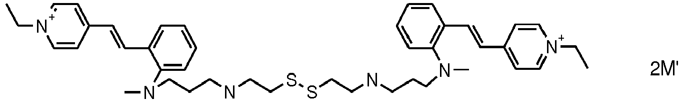 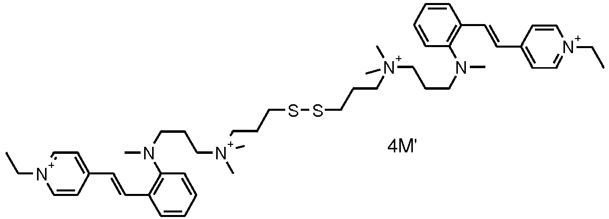 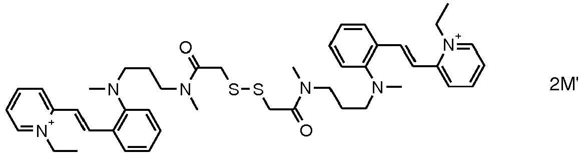 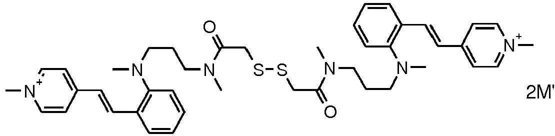 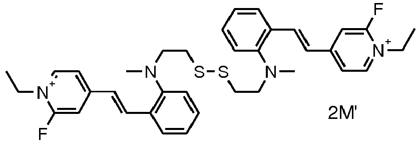 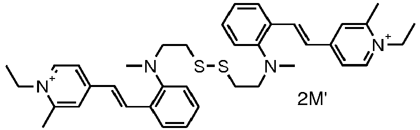 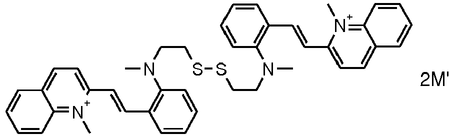 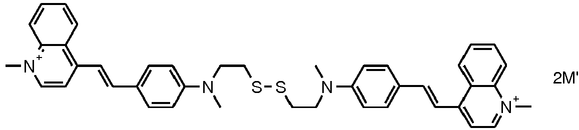 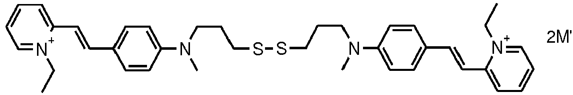 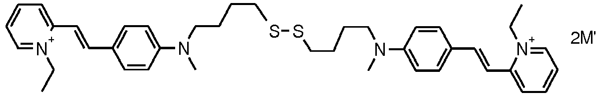 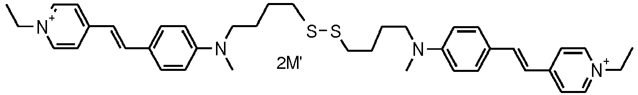 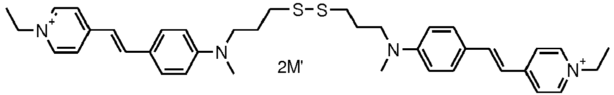 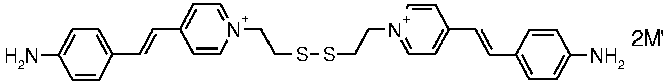 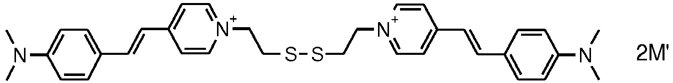 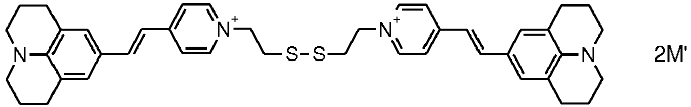 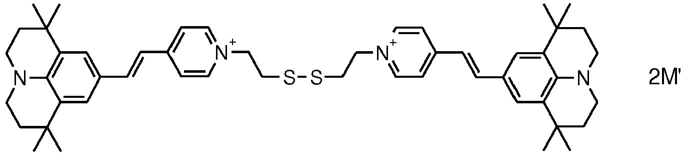 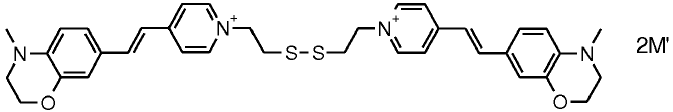 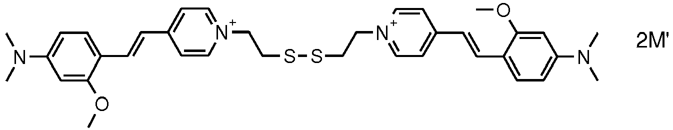 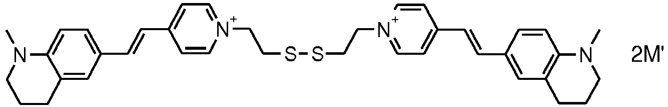 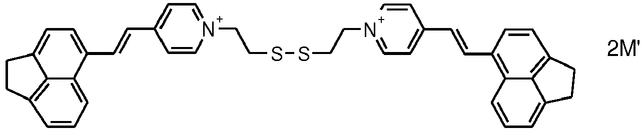 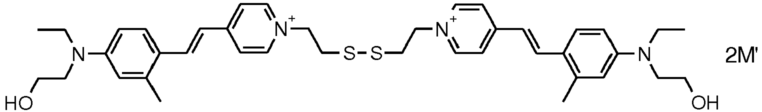 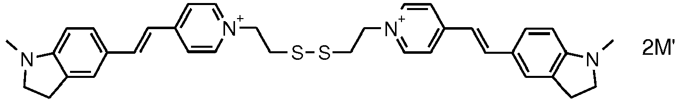 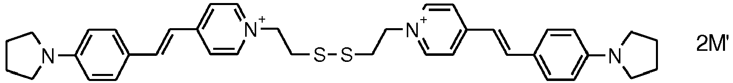 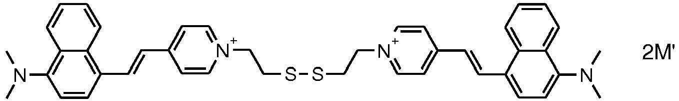 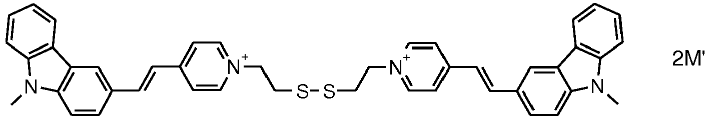 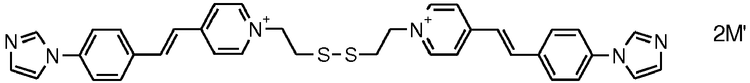 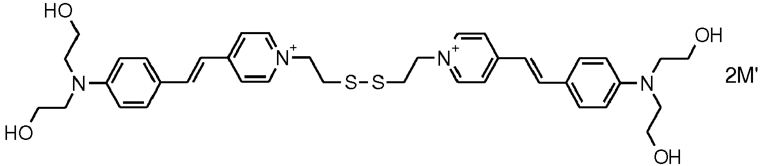 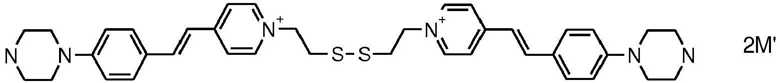 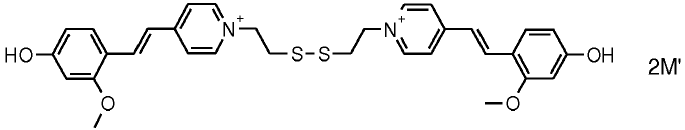 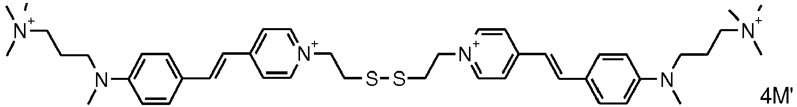 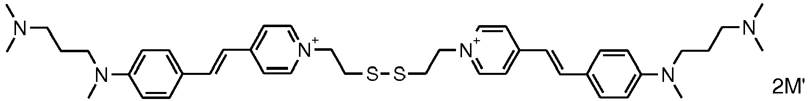 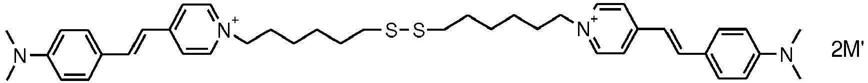 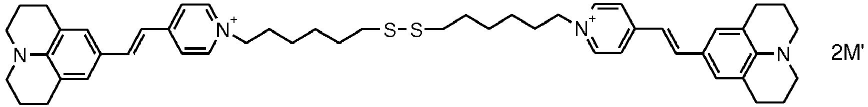 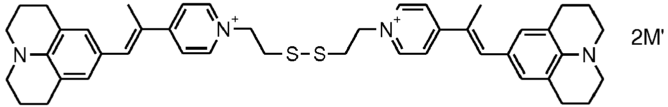 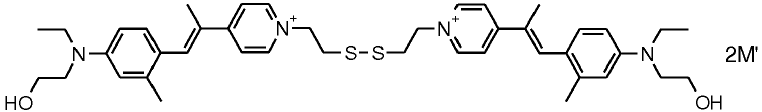 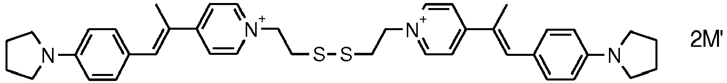 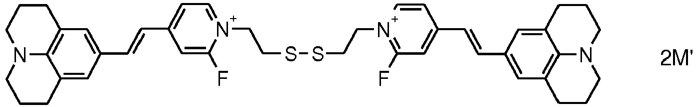 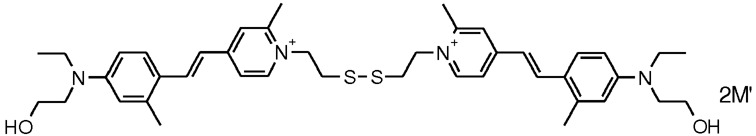 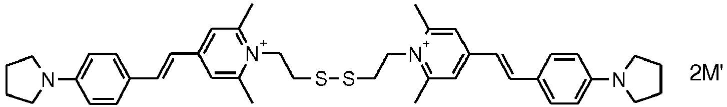 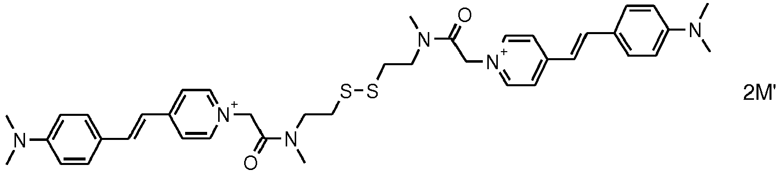 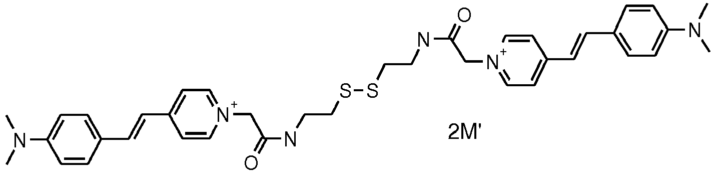 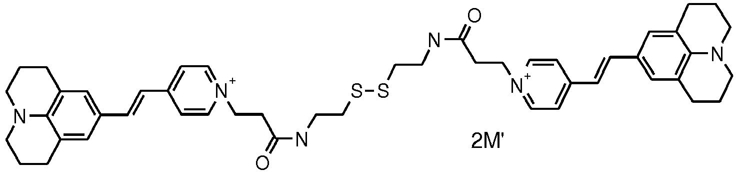 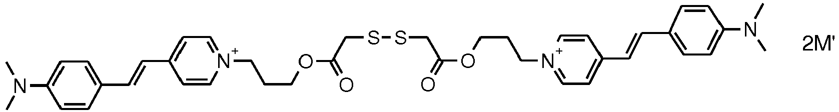 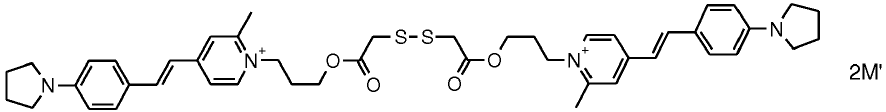 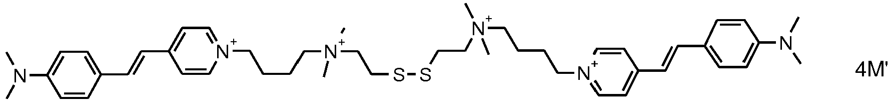 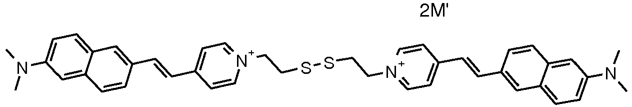 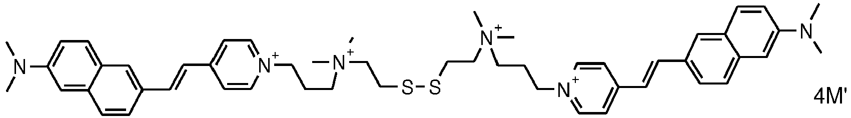 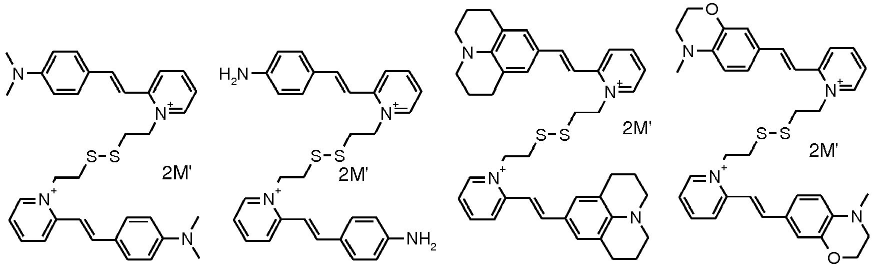 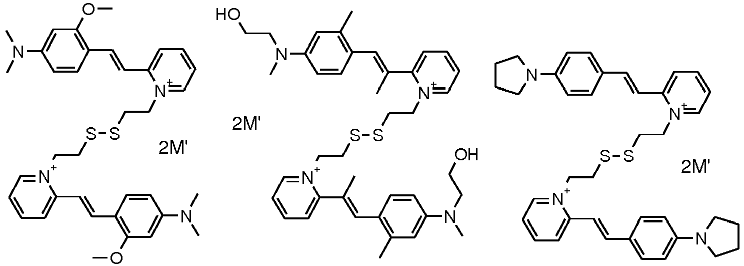 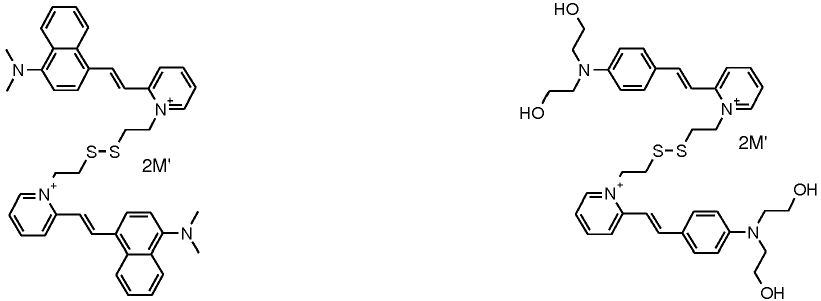 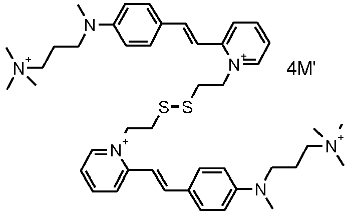 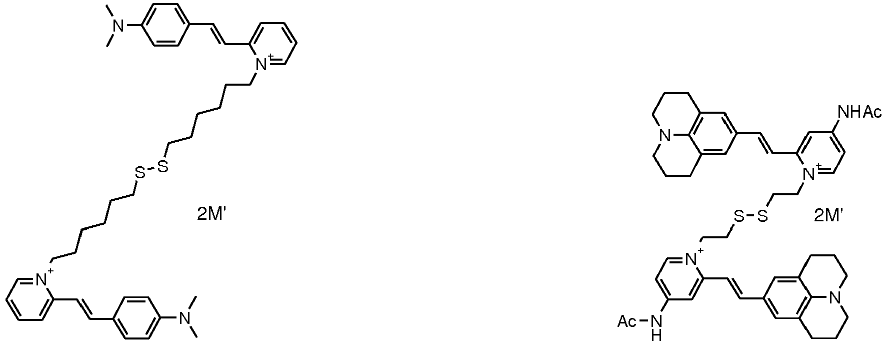 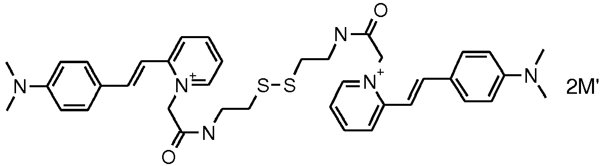 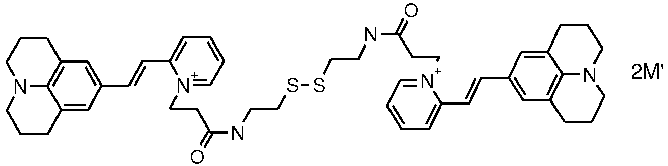 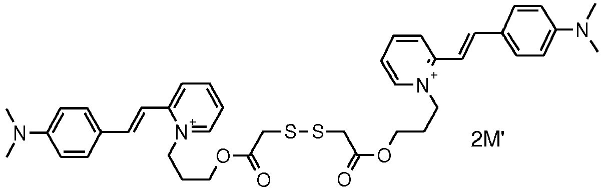 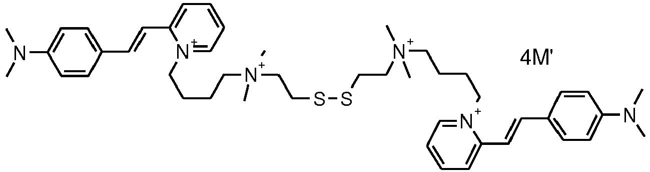 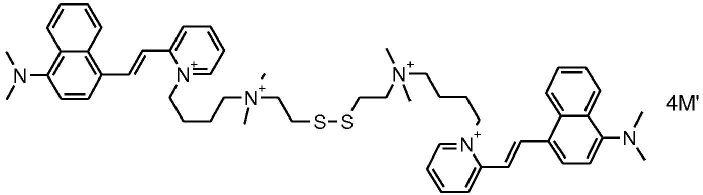 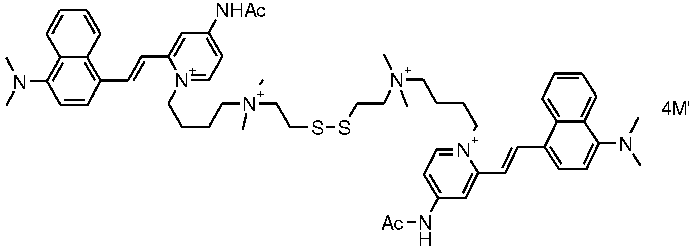 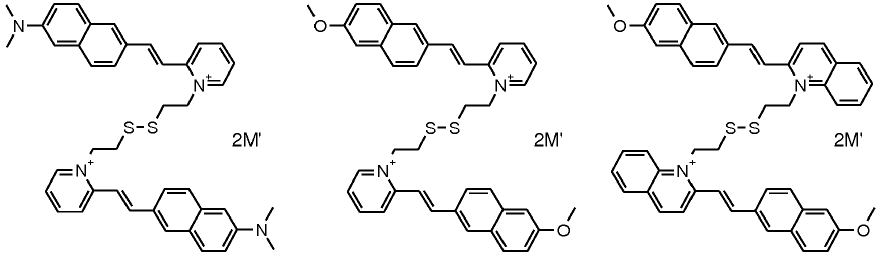 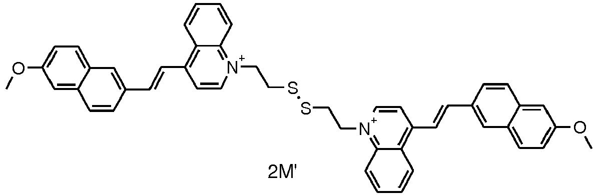 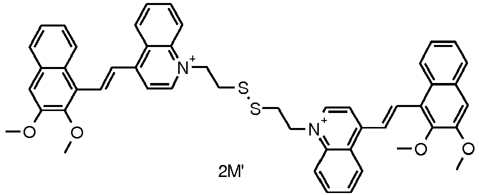 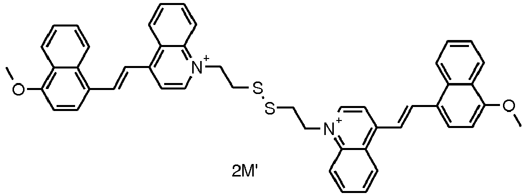 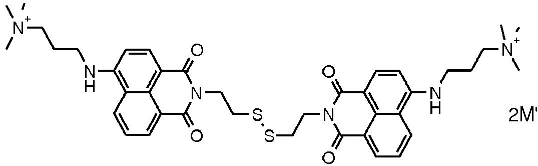 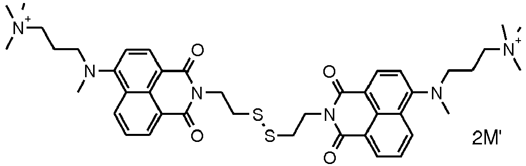 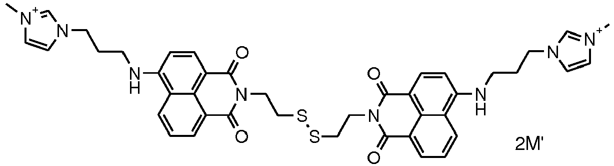 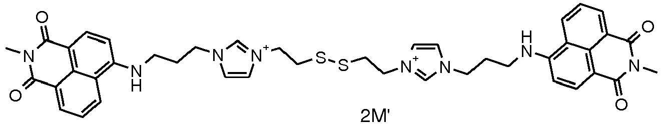 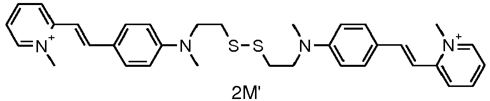 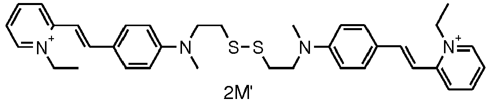 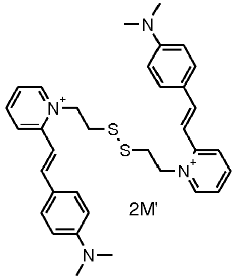 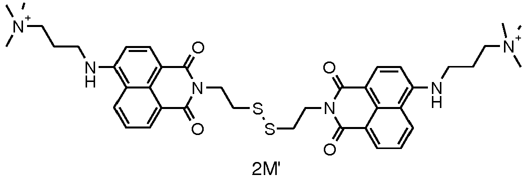 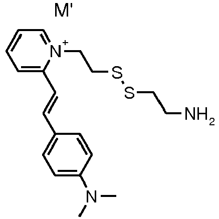 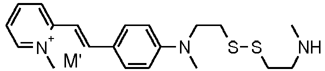 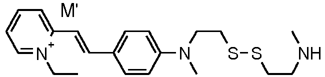 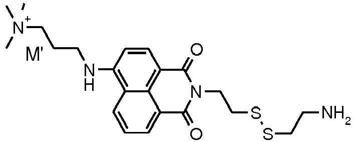 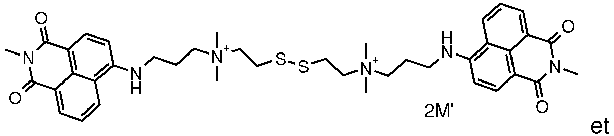 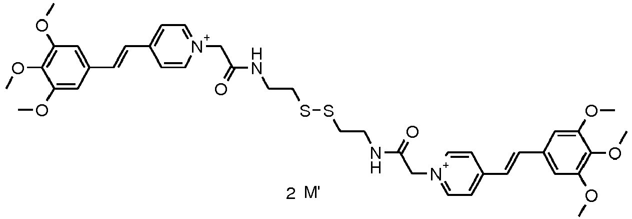 avec M' un contre-ion anionique .

### I.5. Le sel organique et contre-ion:

Un « sel d'acide organique ou minéral » est plus particulièrement choisi parmi un sel dérivé i) d'acide chlorhydrique HCl, ii) d'acide bromhydrique HBr, iii) d'acide sulfurique H₂SO₄, iv) d'acides alkylsulfoniques : Alk-S(O)₂OH tels que d'acide méthylsulfonique et d'acide éthylsulfonique ; v) d'acides arylsulfoniques : Ar-S(O)₂OH tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-0-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique H₃PO₄; xiii) d'acide acétique CH₃COOH ; xiv) d'acide triflique CF₃SO₃H et xv) d'acide tétrafluoroborique HBF₄.

Un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les C₁-C₆ alkylsulfonates : Alk-S(O)₂O- tels que le méthylsulfonate ou mésylate et l'éthylsulfonate ; iv) les arylsulfonates : ArS(O)₂O- tel que le benzènesulfonate et le toluènesulfonate ou tosylate ; v) le citrate ; vi) le succinate ; vii) le tartrate ; viii) le lactate ; ix) les alkylsulfites : Alk-O-S(O)O⁻ tels que le méthysulfite et l'éthylsulfite ; x) les arylsulfites : Ar-O-S(O)O⁻ tels que le benzènesulfite et le toluènesulfite ; xi) les alkylsulfates : Alk-O-S(O)₂O⁻ tel que le méthyl sulfate et l'éthylsulfate ; xii) les arylsulfates : Ar-O-S(O)₂O⁻, xiii) le phosphate ; xiv) l'acétate ; xv) le triflate ; et xvi) les borates tels que le tétrafluoroborate.

### I.6. Préparation des colorants disulfures fluorescents :

Les colorants disulfures fluorescents peuvent être préparés selon des méthodes connues de l'homme de l'art.

Selon une première possibilité, on peut faire réagir un composé disulfure comprenant deux fonctions amine, de préférence primaire ou secondaire avec une quantité suffisante d'un "chromophore fluorescent réactif" ou d'un composé comprenant un tel "chromophore fluorescent réactif", en d'autres termes comprenant une fonction électrophile.

Parmi les "chromophores fluorescent réactifs", on peut citer les colorants réactifs comportant notamment une fonction vinylsulfone, sulfatoéthylsulfone, mono- di-chlorotriazine, mono- di-chloro pyrimidine, difluoro chloro pyrimidine, dichloroquinoxaline, bromo-vinylsulfone.

Conviennent aussi, en tant que chromophores réactifs, les composés chromophores fluorescents comprenant au moins un groupement susceptible de réagir avec une fonction amine pour donner un groupement sulfamide (-SO₂-NR-) amide (-CO-NR-). Par exemple, on peut mentionner les groupes -SO₃W', -COOW' (avec W' représentant un atome d'hydrogène, un métal alcalin, comme le sodium, le potassium, un groupement ammonium, un groupement ammonium substitué par un ou plusieurs groupement alkyle, identiques ou non, linéaires ou ramifiés, en C₁-C₁₀, éventuellement porteurs d'au moins un hydroxyle), que l'on peut activer préalablement, selon des méthodes connues, respectivement en groupement -SO₂Cl, -COCl.

Il est ainsi envisageable de mettre en oeuvre, à titre de chromophore fluorescent réactif, les colorants acides du Colour Index répertoriés comme tels.

On pourra se référer notamment à l'ouvrage Advanced Organic Chemistry, March, 4^{ème} Ed, pour avoir plus de détails sur les conditions opératoires mises en oeuvre.

Toujours dans le cadre de cette première possibilité, on peut mettre en oeuvre des chromophores fluorescents comprenant un groupement labile directement lié ou non au chromophore fluorescent et susceptible d'être substitué par un groupement amine, tel que Cl, Br, F, O-alkyle (par exemple O-Me), O-aryle, O-alkylaryle (par exemple O-benzyle).

Les colorants fluorescents disulfures peuvent aussi être obtenus, dans le cadre de cette possibilité, en utilisant des chromophores possédant une fonction acrylate (-OCO-C=C-) sur laquelle on effectue une réaction d'addition.

Conformément à une autre possibilité, les colorants fluorescents disulfures peuvent être obtenus en faisant réagir un composé disulfure avec un composé portant deux fonctions acide carboxylique activées selon les méthodes classiques (par exemple réaction avec un carbodiimide ou avec le chlorure de thionyle). Le produit résultant est ensuite mis à réagir avec un chromophore fluorescent porteur d'une fonction nucléophile, par exemple de type amine primaire ou secondaire, ou de type alcool aliphatique ou aromatique comme le phénol.

Là encore, on pourra se référer à l'ouvrage Advanced Organic Chemistry, March, 4^{ème} Ed, pour avoir plus de détails sur les conditions opératoires mises en oeuvre.

Conformément à une troisième possibilité, les colorants fluorescents disulfures peuvent être obtenus par réaction d'un composé comprenant un groupement disulfure et deux groupements hydroxyle activés préalablement en groupements partants (par exemple mésylate, tosylate) avec un chromophore fluorescent portant une fonction nucléophile, avantageusement de type amine primaire, secondaire ou tertiaire, hétéroaromatique ou non, par exemple de type pyridine, imidazole, benzimidazole.

Conformément à une quatrième possibilité, les colorants fluorescents disulfures peuvent être obtenus par oxydation ménagée de colorants portant une fonction SH.

Conformément à une cinquième possibilité, et notamment pour la préparation des composés répondant à la formule (II), les colorants fluorescents disulfures peuvent être obtenus par une variante des possibilités une, deux ou trois décrites ci-dessus, en utilisant une quantité molaire de réactif disulfure supérieure ou égale à la quantité molaire de réactif contenant le groupement chromophore.

La préparation des colorants fluorescents disulfures répondant à la formule (I) pour lesquels A et A' sont identiques est en revanche facilitée par l'emploi d'une quantité molaire de réactif contenant le groupement fluorescent chromophore de préférence supérieure ou égale à deux fois la quantité de réactifs disulfures.

Conformément à une sixième possibilité, et notamment pour la préparation des composés répondant à la formule (I) et dont les deux groupements A, A' d'une part et X et X' d'autre part, sont différents, les composés disulfures peuvent être obtenus à partir de composés fluorescents disulfures répondant à la formule (II).

### II. Composition tinctoriale:

### II.1. Colorants :

La composition tinctoriale utile dans l'invention contient en général une quantité de colorant fluorescent disulfure comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

Selon une variante, l'invention contient un agent réducteur capable de réduire les liaisons disulfures. Cet agent réducteur est tel que défini précédemment.

### 11.2. Adjuvants:

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

### Quantité d'adiuvants :

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

### Le pH:

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (a) suivante : 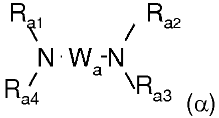 dans laquelle Wₐ est un reste propylène éventuellement substitué par un groupement hydroxyle ou un radical alkyle en C₁-C₄ ; Rₐ₁, Rₐ₂, Rₐ₃ et Rₐ₄, identiques ou différents, représentent un atome d'hydrogène, un radical alkyle en C₁-C₄ ou hydroxyalkyle en C₁-C₄.

### II.3. Formes de la composition :

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

### III. Procédé de coloration:

L'application de la composition tinctoriale est généralement effectuée à température ambiante. Elle peut cependant être réalisée à des températures variant de 20 à 180°C.

Dans un procédé particulier de coloration de l'invention, l'application du colorant fluorescent disulfure sur les matières kératiniques est réalisée en même temps qu'un agent réducteur. L'agent réducteur est tel que défini précédemment.

Selon une autre variante l'agent réducteur est ajouté à la composition tinctoriale contenant les colorants fluorescents disulfures (I) et (II) au moment de l'emploi.

Suivant une autre variante le procédé de l'invention peut être mis en oeuvre en présence d'un agent d'oxydation en post traitement lorsque la composition contient déjà un agent réducteur.

L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant les colorants fluorescents disulfures de formule (I) et (II).

### III. Dispositif ou « kit » de teinture:

L'invention a aussi pour objet un dispositif à plusieurs compartiments ou "kit" de teinture dans lequel un premier compartiment renferme une composition tinctoriale comprenant au moins un colorant fluorescent disulfure de formules (I) ou (II) et un deuxième compartiment renferme un agent réducteur capable de réduire la liaison disulfure du colorant et/ou les fonctions disulfures des matières kératiniques.

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent disulfure de formules (I) ou (II); un deuxième compartiment contient un agent réducteur capable de réduire la liaison disulfure du colorant ; un troisième compartiment contient un agent oxydant.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents disulfures des exemples ci-après ont été entièrement caractérisés par les méthodes spectroscopiques et spectrométriques classiques.

### EXEMPLES

### EXEMPLES DE SYNTHESE

### Exemple 1

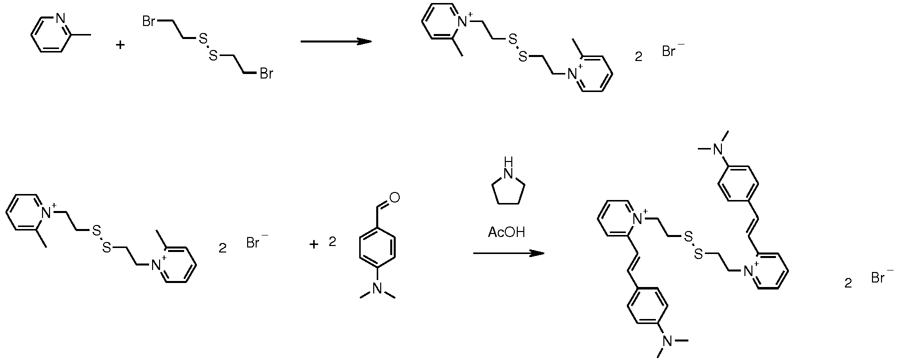

### Etape 1 : dibromure de 1,1'-(disulfanediyldiéthane-2,1-diyl)bis(2-méthyl-pyridinium)

Un mélange de 56 g de 1-bromo-2-[(2-bromoethyl)disulfanyl]ethane et de 15 mL de *N* Méthylpyrrolidone (NMP) est versé goutte à goutte sur 35 g de 2-picoline sous agitation, à 80°C. Le mélange (suspension blanche) est maintenu agité 30 min à 80°C, 100 mL d'acétonitrile sont ajoutés, l'agitation est maintenue à 80°C pendant 90 min. Après refroidissement, le solide obtenu est filtré, lavé par 100 mL d'acétonitrile puis séché.

On recueille 56,2 g de poudre brune. 45 g de cette poudre sont mis en suspension dans 300 mL d'isopropanol, à reflux. Une fois la température baissée à 40°C, le solide est filtré, lavé par 3 fois 100 mL d'isopropanol et séché sous vide. Produit beige clair, 40,56 g. Analyses en conformité avec la structure attendue.

### Etape 2 : dibromure de 1,1'-(disulfanediyldiéthane-2,1-diyl)bis(2-{(E)-2-[4-(diméthylamino)phényl]vinyl}pyridinium)

150 mg de pyrrolidine puis 129 mg d'acide acétique sont ajoutés à une solution de 297 mg de 4-diméthylaminobenzaldéhyde dans 2 mL de méthanol.

Après 18 h d'agitation à température ambiante, 495 mg de dibromure de 1'-(disulfanediyldiéthane-2,1-diyl)bis(2-méthylpyridinium) sont ajoutés au mélange et l'agitation est maintenue à température ambiante pendant 7 j. Après filtration, lavage par du méthanol et séchage sous vide, 312 mg de poudre orange sont recueillis. Analyses en conformité avec la structure attendue. RMN ¹H (400 MHz, MeOH-*d*₄) : 3.02 (s ,6H), 3.22 (t, 2H), 5 (t, 2H), 6.72 (m, 2H), 7.19 (d, 1 H), 7.63 (m, 3H), 7.76 (d, 1 H), 8.3 (m, 2H), 8.59 (m, 1 H).

### Exemple 2

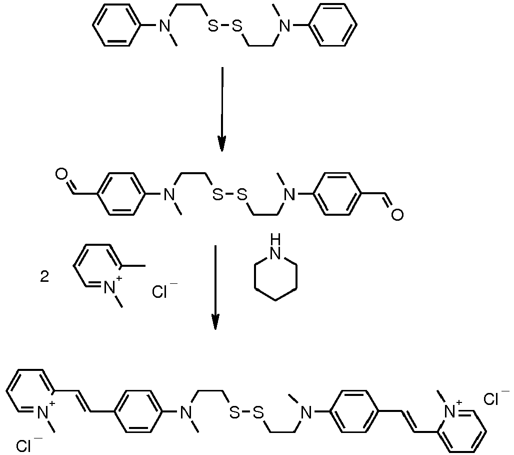

### Etape 1 :4,4'-{disulfanediylbis[éthane-2,1-diyl(méthylimino)]}dibenzaldéhyde

82,3 g d'oxychlorure de phosphore sont ajoutés à 500 mL de DMF à 0°C. Après 30 min d'agitation à 0°C une solution de 47g de *N*,*N*'-(disulfanediyldiéthane-2,1-diyl)bis(N-méthylaniline) sont ajoutés goutte à goutte. Le mélange est agité 90 min. à 0°C puis 75 min. à 10°C et 105 min. à 40°C. Il est ensuite versé sur 2,5 L d'eau glacée, 700 mL de soude 5N sont ajoutés. Le précipité jaune obtenu est filtré sur célite, solubilisé dans 200 mL de dichlorométhane et la solution obtenue est lavée par 200 mL de solution aqueuse saturée en chlorure de sodium. Après séchage sur sulfate de magnésium et évaporation du dichlorométhane, le résidu jaune (80g) est purifié par chromatographie sur gel de silice.

Après séchage, une poudre jaune clair est recueillie. Les analyses indiquent que le produit est conforme à la structure attendue.

### Etape 2 : dichlorure de 2,2'-{disulfanediylbis[éthane-2,1-diyl(méthylimino)-4,1-phénylène(E)éthène-2,1-diyl] }bis(1-méthylpyridinium)

25 g de 4,4'-{disulfanediylbis[éthane-2,1-diyl(méthylimino)]}dibenzaldehyde et 18.5 g de chlorure de N-méthylpicolinium sont dissous dans 300 mL de méthanol. 12,7 mL de pipéridine sont ajoutés au mélange. Le tout est chauffé, sous agitation à 55°C pendant 11 h. Le méthanol est éliminé sous vide à 40°C. Le solide est mélangé à 300 mL d'isopropanol. Après un nouveau séchage par évaporation on introduit 200 mL d'isopropanol. Le mélange prend en masse, il est étendu par addition de 100 mL d'iospropanol et essoré sur verre fritté. Le solide recueilli est lavé par de l'isopropanol puis de l'acétone, puis séché sous vide. Après séchage, 36,7g de poudre orange sont recueillis. Par recristallisation dans l'isopropanol, 27 g de poudre rouge orange de pureté supérieure sont recueillis. Les analyses indiquent que le produit est conforme et pur. RMN ¹H (400 MHz, MeOH-*d*₄) 2.99 (t, 4H), 3.81 (t, 4H), 4.31 (s, 6H), 6.86 (d, 4H), 7.22 (d, 2H), 7.63 (m, 2H), 7.69 (d, 4H), 7.83 (d, 2H), 8.29 (m, 2H), 8.36 (m, 2H), 8.61 (m, 2H).

### Exemple 3 :

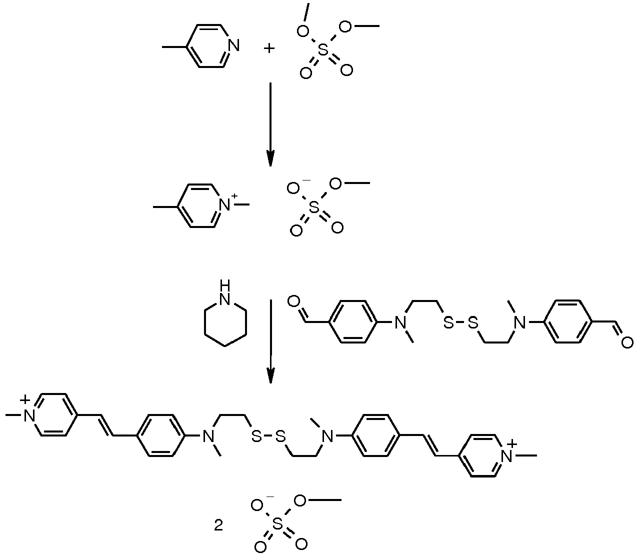

### Diméthoxysulfate de 4,4'-{disulfanediylbis[éthane-2,1-diyl(méthylimino)-4,1-phénylène(E)éthène-2,1-diyl]}bis(1-méthylpyridinium)

2,62 g de 4-picoline sont dilués dans 25 mL de dichlorométhane, 3 mL de sulfate de diméthyle sont ajoutés à la solution, dont la température s'élève au reflux (40°C). Après 40 min. d'agitation, 50 mL d'isopropanol sont ajoutés, et le mélange est concentré par distillation du dichlorométhane (mélange chauffé à 60°C). 1,83 g de pyrrolidine sont introduits dans le mélange suivis de 4,99 g de 4,4'-{disulfanediylbis[éthane-2,1-diyl(méthylimino)]}dibenzaldéhyde. Après 2 h d'agitation à 65°C, le mélange réactionnel est refroidi à température ambiante, le précipité formé est filtré, lavé par 3 fois 100 mL d'isopropanol. La pâte rouge obtenue est dispersée dans 200 mL d'isopropanol, le mélange ainsi obtenu est porté à reflux puis refroidi. Le précipité rouge formé est filtré puis séché. 8,94 g de poudre rouge sont recueillis. Les analyses indiquent que le produit est conforme et pur. RMN ¹H (400 MHz, DMSO-*d*₆) 2.96 (t, 4 H), 3.02 (s, 6 H), 3.36 (s, 6 H), 3.72 (t, 4 H), 4.16 (s, 6 H), 6.81 (d, 4 H), 7.15 (d, 2 H), 7.57 (d, 4 H), 7.87 (d, 2 H), 8.02 (d, 4 H), 8.66 (d, 4 H).

### Exemple 4

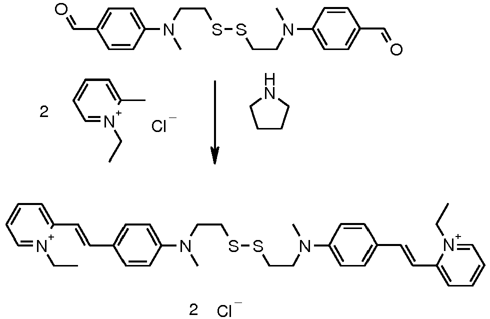

### Dichlorure de 2,2'-{disulfanediylbis[éthane-2,1-diyl(méthylimino)-4,1-phénylène(E)éthène-2,1-diyl]}bis(1-éthylpyridinium)

10 g de 4,4'-{disulfanediylbis[éthane-2,1-diyl(méthylimino)]}dibenzaldéhyde et 8,1 g de chlorure de N-éthylpicolinium sont dissous dans 100 mL d'isopropanol. 1,3 g de pipéridine sont ajoutés au mélange. Le tout est chauffé, sous agitation à reflux pendant 5 h. L'isopropanol est éliminé sous vide à 50°C. La gomme obtenue est triturée avec de l'acétone. 18 g de solide sont recueillis et traités au noir de charbon. 7,1 g de produit sont collectés et 4 g sont purifiés par chromatographie liquide liquide (eau/BuOH). Après séchage 1,65 g de poudre rouge sont recueillis. Les analyses indiquent que le produit est conforme et pur. RMN ¹H (400 MHz, MeOH-*d*₄) 1.57 (t, 6 H), 2.98 (t, 4 H), 3.11 (s, 6 H), 3.8 (t, 4 H), 4.73 (q, 4 H), 6.85 (m, 4 H), 7.23 (d, 2 H), 7.69 (m, 6 H), 7.85 (d, 2 H), 8.29 (m, 2 H), 8.38 (m, 2 H), 8.67 (m, 2 H).

### Exemple 5

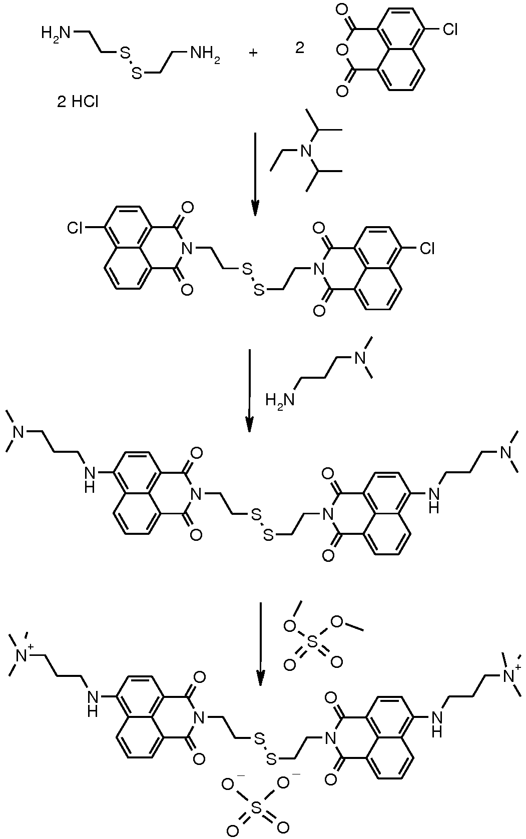

### Etape 1 : 2,2'-(disulfanediyldiéthane-2,1-diyl)bis(6-chloro-1H-benzo[de]-isoquinoline-1,3(2H)-dione)

9,30 g de 6-chloro-1 H,3H-benzo[de]isochromene-1,3-dione et 4,46 g de chlorhydrate de cystamine chlorhydratée sont mis en suspension dans 50ml de N-méthylpyrrolidone (NMP). 5,5 g de diisopropylethylamine sont ajoutés et le mélange est chauffé, sous agitation à 120°C. Après deux heures 50 mL de NMP sont ajoutés et le mélange est maintenu agité à 120°C pendant 3 h. Après refroidissement, le produit précipité est recueilli, la solution filtrée est étendue par ajout de 200 mL d'eau et un deuxième précipité est recueilli. Les précipités sont lavés à l'eau et séchés. 11,46 g de poudre blanche sont recueillis. Les analyses montrent que le produit est conforme à l'attendu

### Etape 2 : 2,2'-(disulfanediyldiéthane-2,1-diyl)bis[6-{[3-(diméthylamino)propyl]-amino}-1H-benzo[de]isoquinoline-1,3(2H)-dione]

4 g de (6-chloro-2-(2-{[2-(6-chloro-1,3-dioxo-1H-benzo[de]isoquinolin-2(3H)-yl)éthyl]-disulfanyl}éthyl)-1H-benzo[de]isoquinoline-1,3(2H)-dione sont mis en suspension 40 mL de N,N-dimethylpropane-1,3-diamine. Le mélange est chauffé sous agitation à 110°C pendant 12 h. Après refroidissement, un précipité jaune est collecté, 500 mL de mélange éthanol/eau : 1/1 sont ajoutés goutte à goutte au filtrat. La pâte jaune obtenue est isolée et triturée avec 200 mL d'acétone. Les solides obtenus sont lavés par 300 mL d'eau et séchés. 4,5 g de poudre jaune sont recueillis. Les analyses montrent que le produit est conforme à l'attendu.

### Etape 3 : sulfate de 3,3'-{disulfanediylbis[ethane-2,1-diyl(1,3-dioxo-1H-benzo[de]isoquinoline-2,6(3H)-diyl)imino]}bis(N,N,N-triméthylpropan-1-aminium)

4 g de 6-{[3-(diméthylamino)propyl]amino}-2-[2-({2-[6-{[3-(diméthylamino)propyl]-amino}-1,3-dioxo-1H-benzo[de]isoquinolin-2(3H)-yl]éthyl}disulfanyl)éthyl]-1H-benzo-[de]isoquinoline-1,3(2H)-dione sont mis en suspension dans 50 mL de diméthylformamide 4 mL de diméthylsulfate sont ajoutés et le mélange est maintenu sous agitation à température ambiante pendant 4 h. Le mélange réactionnel est versé dans 500 mL d'acétate d'éthyle. Le précipité est filtré, lavé par 4 fois 100 mL d'acétate d'éthyle et séché sous vide. On recueille 5,9 g de poudre jaune. Les analyses indiquent que le produit est conforme à l'attendu. RMN ¹H (400 MHz, DMSO-*d*₆) : 2.13 (m, 4 H), 3.06 (m, 4 H), 3.09 (s, 18 H), 3.46 (m, 4 H), 4.36 (m, 4 H), 6.85 (d, 2 H), 7.71 (m, 2 H), 7.82 (t, 2 H), 8.28 (d, 2 H), 8.29 (dd, 2 H), 8.45 (dd, 2 H).

### Exemple 6

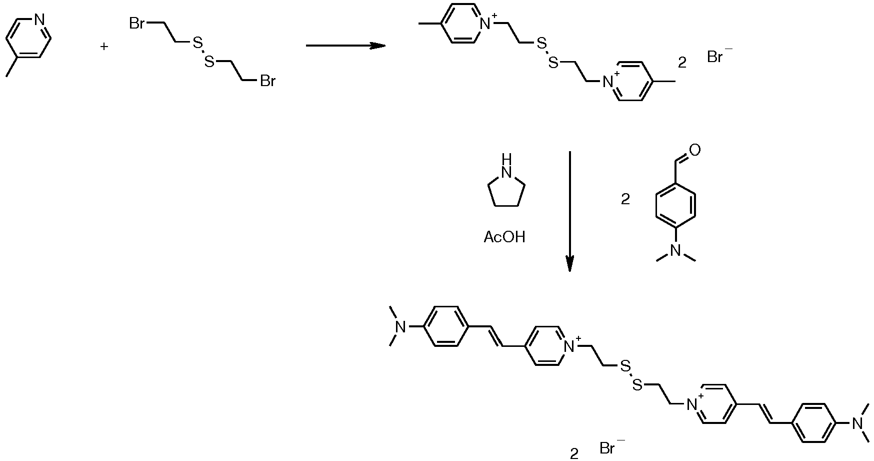

### Etape 1 : dibromure de 1,1'-(disulfanediyldiéthane-2,1-diyl)bis(4-méthyl-pyridinium)

67 g de 4-picoline sont dilués dans 100 mL d'acétonitrile et le mélange est porté à 80°C. Un mélange de 60 g de 1-bromo-2-[(2-bromoethyl)disulfanyl]ethane et 15 mL de N-méthylpyrrolidone (NMP) est ajouté en 5 min. Après 4 h d'agitation à 85°C, le mélange est refroidi. Le solide obtenue est filtré, rincé par 3 x 200 mL d'acétonitrile puis dissout dans 800 mL d'isopropanol (à reflux). Après refroidissement, 1 L d'éther éthylique est ajouté. Le précipité formé est filtré, rincé par 3 x 200 mL d'éther éthylique puis séché. La poudre blanc cassée obtenue (73,77g) contient très majoritairement (>90%) le produit attendu qui est utilisé tel quel pour l'étape suivante.

### Etape 2 : dibromure de 1,1'-(disulfanediyldiéthane-2,1-diyl)bis(4-{(E)-2-[4-(diméthylamino)phényl]vinyl}pyridinium)

13,2 g de 4-diméthylaminobenzaldéhyde sont mis en suspension dans 100 mL de méthanol. 6,2 g de pyrrolidine puis 5,3 g d'acide acétique dilués dans 20 mL de méthanol sont ajoutés au mélange (pH final 5/6). 20 g de dibromure de 1,1'-(disulfanediyldiéthane-2,1-diyl)bis(4-méthylpyridinium) obtenus à l'étape précédente, solubilisés dans 80 mL de méthanol sont introduits, puis le mélange réactionnel est dilué par ajout de 100 mL de méthanol. Après 21 h d'agitation à température ambiante, un premier précipité est recueilli, lavé par 3 x 100 mL d'éthanol puis 3 x 200 mL d'acétate d'éthyle et séché (poudre rouge, 7,4 g), puis un second précipité formé dans le filtrat est également recueilli et séché (poudre rouge, 11,44 g). Les analyses indiquent que les deux fractions sont conformes à la structure attendue. RMN ¹H (400 MHz, MeOH-*d*₄) : 3.02 (s, 12 H), 3.42 (t, 4 H), 4.74 (t, 4 H), 6.77 (d, 4 H), 7.19 (d, 2 H), 7.6 (d, 4 H), 7.97 (d, 2 H), 8.1 (d, 4 H), 8.79 (d, 4 H).

### Exemple 7

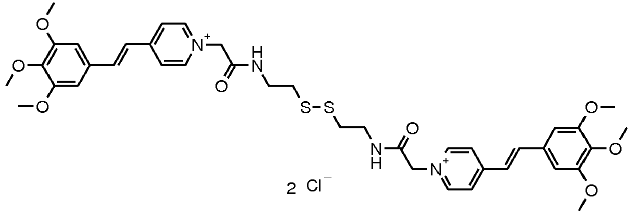

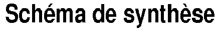

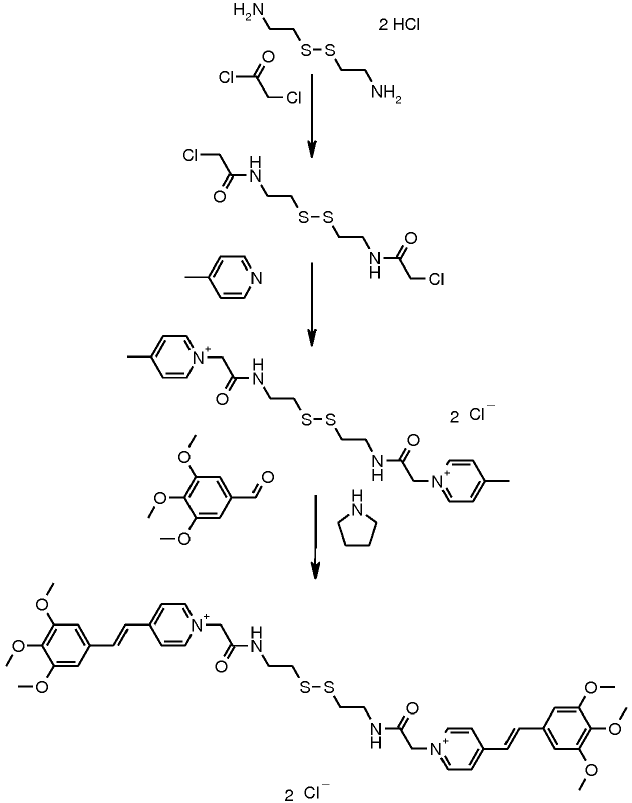

### Etape 1 : Synthèse du N,N'-(disulfanediyldiéthane-2,1-diyl)bis(2-chloroacétamide)

40,3 g de dichlorhydrate de cystamine sont dissous dans 100 mL d'eau, 32 mL de soude à 35 % sont ajoutés (pH 9.7) et la température est abaissée à 5 °C. 33,5 mL de chlorure de chloracétyle sont introduits goutte à goutte, en maintenant la température inférieure à 10 °C et en maintenant le pH entre 7,9 et 9,3 par addition de soude. Le milieu est maintenu agité à température ambiante pendant 2 H. Le précipité est filtré, lavé par 5 x 150 mL d'eau puis séché sous vide en présence de P₂O₅. 35,3 g de poudre blanche sont recueillis. les analyses indiquent que le produit est conforme.

### Etape 2 : Synthèse du dichlorure de 1,1'-{disulfanediylbis[éthane-2,1-diylimino(2-oxoéthane-2,1-diyl)]}bis(4-méthylpyridinium)

6,1 g N,N'-(disulfanediyldiéthane-2,1-diyl)bis(2-chloroacétamide) et 4,5 g de 4-picoline sont dissous dans 50 mL de NMP et portés à 80 °C pendant 19 h. Après refroidissement du mélange, par précipitations successives dans l'acétone et séchage sous vide, 9,2 g de sels sont recueillis. Les analyses montrent que le produit est conforme. RMN ¹H (400 MHz, D₂O) : 2.61 (s, 6 H), 2.82 (t, 4 H), 3.56 (t, 4 H), 5.31 (s, 4 H), 7.85 (d, 4 H), 8.51 (d, 4 H).

### Etape 3 : Synthèse du dichlorure de 1,1'-{disulfanediylbis[éthane-2,1-diylimino(2-oxoéthane-2,1-diyl)]}bis{4-[(E)-2-(3,4,5-triméthoxyphényl)vinyl]pyridinium} [4]

785 mg de 3,4,5-triméthoxy-benzaldéhyde, 328 µL de pyrrolidine, 232 µL d'acide acétique et 490 mg de dichlorure de 1,1'-{disulfanediylbis[éthane-2,1-diylimino(2-oxoéthane-2,1-diyl)]}bis(4-méthylpyridinium) sont mis en solution dans 10 mL d'isopropanol et maintenus agités à température ambiante 3 h 30 min. Le mélange est versé sur 50 mL de solution de dichlorométhane / acétone 1 :1. Un solide précipite. Il est filtré, lavé par trois fois 20 mL d'acétone et séché sous vide. 509 mg de poudre noire sont recueillis. Les analyses montrent que le produit est conforme (LCMS : 100% ; pic de masse m/z = 388, correspondant au dication).

### EXEMPLES DE COLORATION

### Préparation d'une composition A

| | |
|---|---|
| Colorant disulfure de formule (I) selon l'invention | 10⁻³ mol% |
| Alcool Benzylique | 4 g |
| Polyéthylèneglycol 6OE | 6 g |
| Hydroxyethylcellulose | 0,7 g |
| Alkylpolyglucoside en solution aqueuse à 65%MA | 4.5 g |
| Eau déminéralisée | qsp 100g |

### Préparation d'une composition B

| | |
|---|---|
| Acide thioglycolique | 1M |
| Hydroxyde de Sodium | qsp pH 8,5 |
| Eau déminéralisée | qsp 100g |

Au moment de l'emploi, on mélange les compositions **A** (9 volumes) et **B** (1 volume), puis on applique le mélange obtenu sur des cheveux foncés (hauteur de ton 4) pendant 30 minutes à température ambiante. Après rinçage à l'eau et séchage, on observe un éclaircissement optique des cheveux ainsi traités.

Les mèches ainsi traitées sont soumises à 30 shampooings selon un cycle qui comprend le mouillage des mèches à l'eau, le lavage aux shampooings, un rinçage à l'eau suivi d'un séchage.

La mèche est séchée avant l'application du shampooing suivant.

La couleur des mèches avant et après les 30 lavages a été évaluée dans le système L*a*b* au moyen d'un spectrophotomètre CM 2002 MINOLTA ®, (Illuminant D65).

Dans le système L* a* b*, les trois paramètres désignent respectivement l'intensité (L*), la nuance (a*) et la saturation (b*). Selon ce système, plus la valeur de L est élevée, plus la couleur est claire ou peu intense. Inversement, plus la valeur de L est faible, plus la couleur est foncée ou très intense. a* et b* indiquent deux axes de couleurs, a* indique l'axe de couleur vert/rouge et b* l'axe de couleur bleu/jaune.

| | **L** | **a*** | **b*** |
|---|---|---|---|
| Composé 1 | 24,85 | 8,08 | 7,68 |
| Composé 1 après 30 shampooings | 24,78 | 7,83 | 7,56 |
| Composé 2 | 26,32 | 6,38 | 10,01 |
| Composé 2 après 30 shampooings | 25,61 | 5,48 | 9,05 |
| Composé 3 | 25,49 | 10,56 | 9,96 |
| Composé 3 après 30 shampooings | 25,98 | 11,41 | 9,86 |
| Composé 4 | 25,87 | 6,06 | 9,60 |
| Composé 4 après 30 shampooings | 24,38 | 5,86 | 9,20 |
| Composé 5 | 25,08 | 1,19 | 7,51 |
| Composé 5 après 30 shampooings | 25,72 | 1,38 | 6,01 |

Les résultats du tableau ci-dessus montrent que la coloration évolue très peu même après 30 shampooings. Ainsi la coloration et l'effet éclaircissant sur les cheveux reste quasiment inchangés ce qui montre une très bonne résistance aux shampooings des colorants de l'invention.

## Revendications

1. Procédé de coloration de matières kératiniques foncée, **caractérisé en ce qu'**on applique sur les matières, au moins un colorant fluorescent disulfure en même temps qu'un agent réducteur.

2. Procédé selon la revendication précédente **caractérisé en ce qu'**on applique une composition tinctoriale, comprenant dans un milieu cosmétique approprié, au moins un colorant disulfure fluorescent en présence d'un agent réducteur.

3. Procédé selon une quelconque des revendications précédentes, **caractérisé en ce que** le colorant fluorescent disulfure est choisi parmi les colorants de formules (I) et (II) tels que définis ci-dessous :
A-(X)ₚ-Cₛₐₜ-S-S-C'ₛₐₜ-(X')_{p'}-A' (I)
A-(X)ₚ-Cₛₐₜ-S-S-C'ₛₐₜ-(X')_{p'}-D (II)
leurs sels d'acide organique ou minéral, isomères optiques, isomères géométriques, et les solvates tels que hydrates,
formules dans lesquelles :
• A, et A', identiques ou différents, représentent un radical contenant au moins un chromophore fluorescent cationique ou non;
• X et X', identiques ou différents, représentent une chaîne hydrocarbonée en C₁-C₃₀, linéaire ou ramifiée, saturée ou insaturée, éventuellement interrompue et/ou éventuellement terminée à l'une ou deux de ses extrémités par un ou plusieurs groupes divalents ou leur combinaisons choisis parmi :
- -N(R)-, -N⁺(R)(R)-, -O-, -S-, -CO-, -SO₂- avec R, identiques ou différents, choisi parmi un hydrogène, un radical alkyle en C₁-C₄, hydroxyalkyle, aminoalkyle
- un radical (hétéro)cyclique aromatique ou non, saturé ou insaturé, condensé ou non comprenant éventuellement un ou plusieurs hétéroatomes identiques ou non, éventuellement substitué ;
• les indices p et p', identiques ou différents, sont égaux à 0 ou 1 ;
• Cₛₐₜ, et C'ₛₐₜ, identiques ou différents, représentent une chaîne alkylène en C₁-C₁₈, linéaire ou ramifiée, éventuellement substituée, éventuellement cyclique ;
• D correspond à un radical choisi parmi les radicaux hydroxy, hydroxyalkyle, alcoxy, carboxyliques, carboxylates, amino, alkylamino et dialkylamino.

4. Procédé selon la revendication précédente **caractérisé en ce que** le colorant fluorescent disulfure de formule (I) ou (II) comprend un radical A ou A' identiques ou différents contiennant un chromophore styryle.

5. Procédé selon l'une quelconque des deux revendications précédentes, **caractérisé en ce que** X et X', identiques ou différents, représentent un groupement -N(R)-.

6. Procédé selon l'une quelconque des revendications 3 à 5, **caractérisé en ce que** A et A', identiques ou différents, plus préférentiellement identiques, représentent W-C(R^{c})=C(R^{d})-Ar- ou -W-C(R^{c})=C(R^{d})-Ar, avec W représentant un hétérocycle ou un hétéroaryle, comprenant un ammonium quaternaire ; Ar représente un radical (hétéro)aryle à 5 ou 6 chaînons de type phényle ou pyridium, ou un bicycle (hétéro)aromatique de type naphtyle, benzopyrydinium, indolinyle ou benzoindolinyle éventuellement substitués par un ou plusieurs atomes d'halogène; par un ou plusieurs groupements alkyle; par un ou plusieurs groupements hydroxyle ; par un ou plusieurs groupements alcoxy, par un ou plusieurs groupements hydroxyalkyle, par un ou plusieurs groupements amino ou (di)alkylamino, par un ou plusieurs groupements acylamino ; par un ou plusieurs groupements hétérocycloalkyle ou hétéroaryle à 5 ou 6 chaînons; R^{c}, R^{d}, identiques ou différents, représentent un atome d'hydrogène ou un groupement alkyle en C₁-C₄.

7. Procédé selon l'une quelconque des revendications 3 à 6, **caractérisé en ce que** W est un imidazolium, pyridinium, benzopyridinium, benzimidazolium, quinolinium, et pyrazolium.

8. Procédé selon l'une quelconque des revendications précédentes **caractérisé en ce que** le colorant fluorescent disulfure est choisi parmi les composés (III) à (XII): 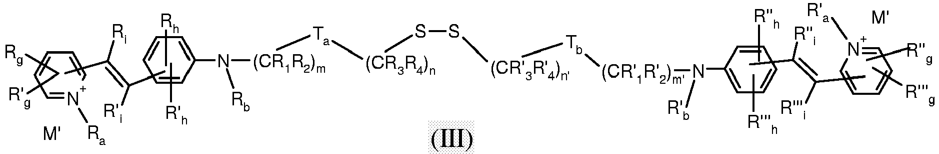 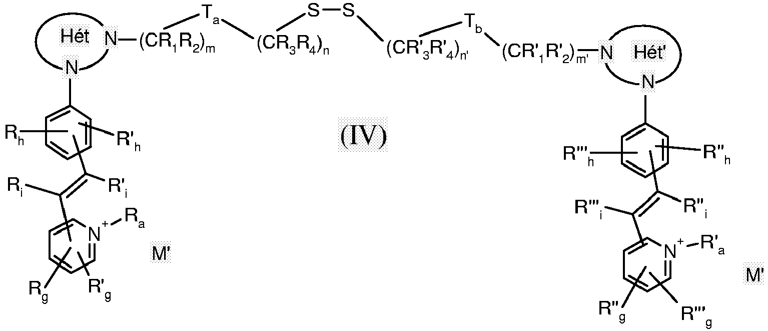 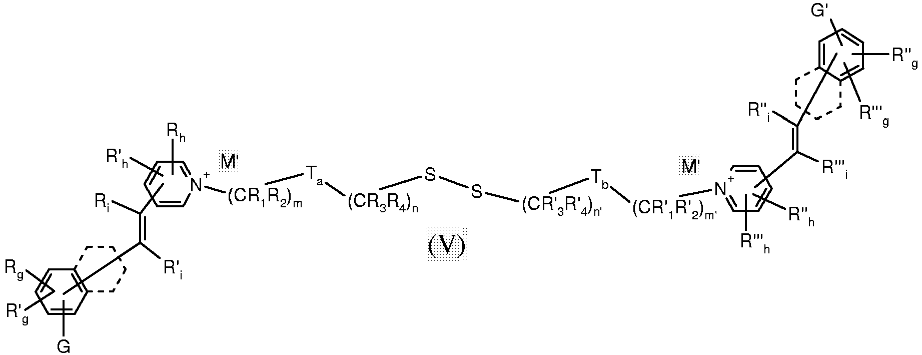 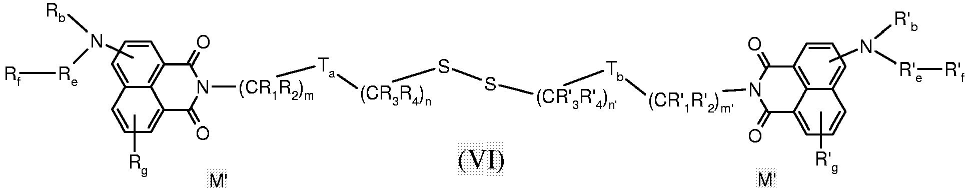 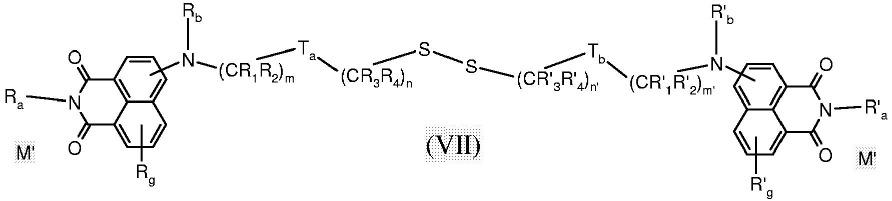 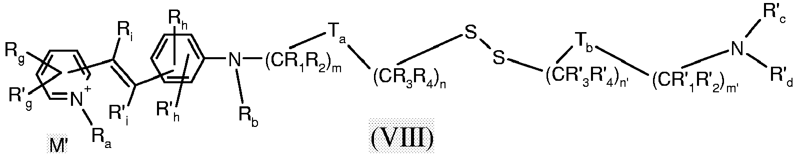 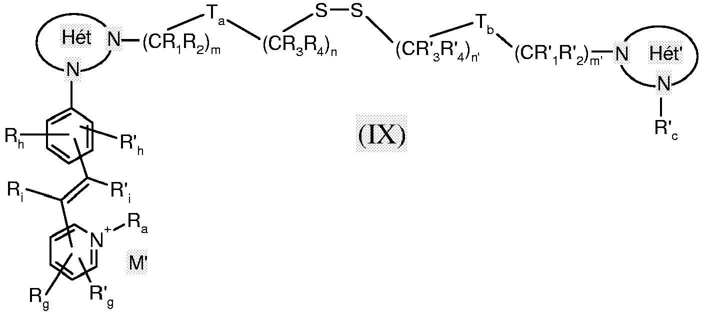 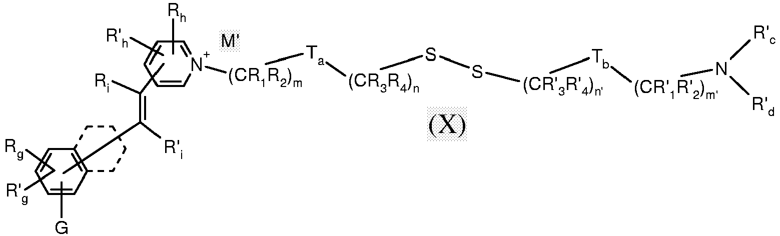 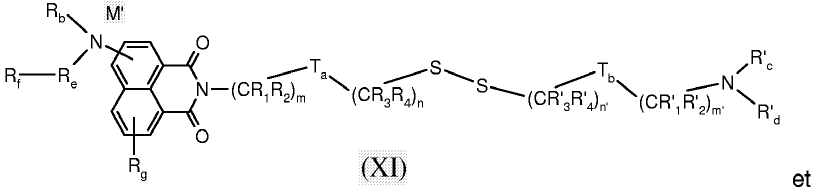 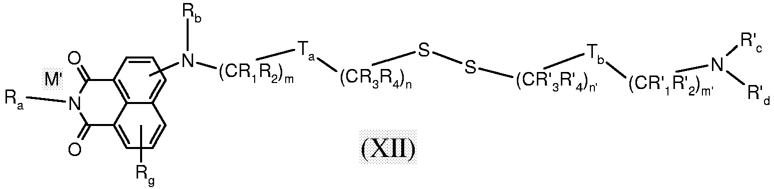 dans lequel
• G et G', identiques ou différents, représentent un groupement -NR_{c}R_{d},-NR'_{c}R'_{d}, ou C₁-C₆ alkoxy éventuellement substitué; préférentiellement G et G' représentent un groupement -NR_{c}R_{d} et -NR'_{c}R'_{d} respectivement ;
• Rₐ et R'ₐ, identiques ou différents, un groupement aryl(C₁-C₄)alkyle ou un groupement C₁-C₆ alkyle éventuellement substitué par un groupement hydroxyle ou amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
• R_{b}, R'_{b}, identiques ou différents, représentent un atome d'hydrogène, un groupement aryl(C₁-C₄)alkyle ou un groupement C₁-C₆ alkyle éventuellement substitué;
• R_{c}, R'_{c}, R_{d} et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ;
ou alors deux radicaux adjacents R_{c} et R_{d}, R'_{c} et R'_{d} portés par le même atome d'azote forment ensemble un groupe hétérocyclique ou hétéroaryle ;
• Rₑ et R'ₑ, identiques ou différents, représentent une chaîne hydrocarbonée en C₁-C₆ alkylènyle divalente, linéaire ou ramifiée, éventuellement insaturée ;
• R_{f} et R'_{f}, identiques ou différents, représentent un groupement ammonium quaternaire (R")(R"')(R"")N⁺- où R", R'" et R"", identiques ou différents, représentent un atome d'hydrogène ou un groupement C₁-C₄ alkyle ou alors (R")(R"')(R"")N⁺- représente un groupement hétéroaryle cationique éventuellement substitué préférentiellement un groupement imidazolinium éventuellement substitué par un groupement C₁-C₃ alkyle ;
• R_{g}, R'_{g}, R"_{g}, R"'_{g}, Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
ou alors deux groupements R_{g} et R'_{g} ; R"_{g} et R"'_{g} ; Rₕ, et R'ₕ ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
• ou alors lorsque G représente -NR_{c}R_{d} et G' représente -NR'_{c}R'_{d} deux groupements R_{c} et R'_{g} ; R'_{c} et R"_{g} ; R_{d} et R_{g} ; R'_{d} et R"'_{g} forment ensemble un hétéroaryle ou hétérocycle saturé, éventuellement substitué par un groupement C₁-C₆ alkyle;
• Rᵢ, R'ᵢ, R"ᵢ, et R'"ᵢ, identiques ou différents, représentent un atome d'hydrogène, ou un groupement C₁-C₄ alkyle ;
• R₁, R₂, R₃, R₄, R'₁, R'₂, R'₃ et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
• Tₐ et T_{b}, identiques ou différents, représentent i) soit une liaison covalent σ, ii) soit un ou plusieurs radicaux ou leurs combinaisons choisis parmi -SO₂-, -O-, -S-, -N(R)-, -N⁺(R)(R^{o})-, -CO-, avec R, R^{o}, identiques ou différents, représentant un atome d'hydrogène, un radical alkyle en C₁-C₄, hydroxyalkyle en C₁-C₄; ou un aryl(C₁-C₄)alkyle, iii) soit un radical hétérocycloalkyle ou hétéroaryle, cationique ou non;
• 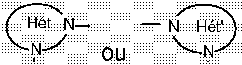 identiques ou différents, représentent une groupement hétérocyclique éventuellement substitué ;
• 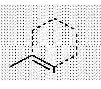 représentent une groupement aryle ou hétéroaryle fusionné au cycle phényle ; ou alors est absent du cycle phényle;
• m, m', n et n', identiques ou différents, représentent un entier compris inclusivement entre 0 et 6 avec m+n, m'+n', identiques ou différents, représente un entier compris inclusivement entre 1 et 10;
M' représentant contre-ion ou un sel d'acide organique ou minéral.

9. Procédé selon l'une quelconque des revendications précédentes, **caractérisé en ce que** le colorant fluorescent disulfure est choisi parmi : 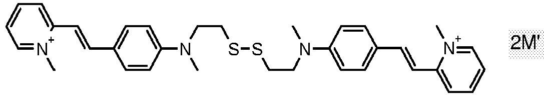 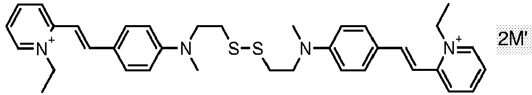 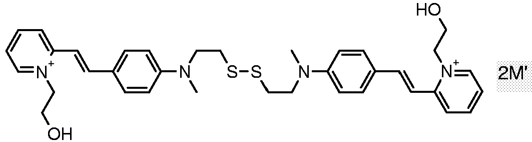 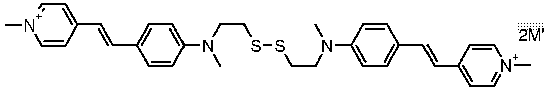 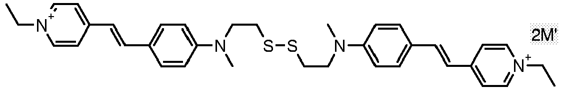 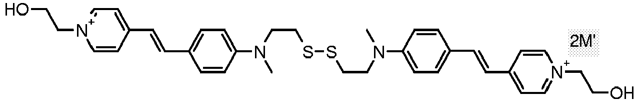 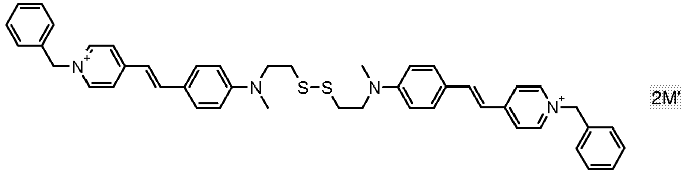 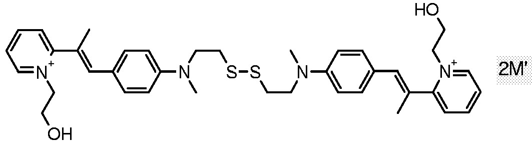 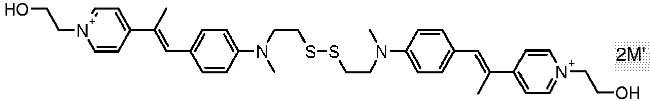 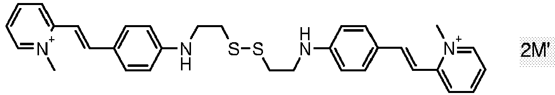 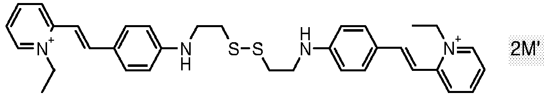 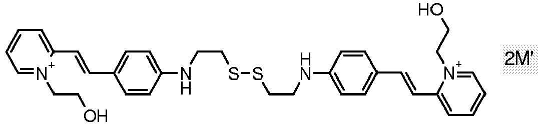 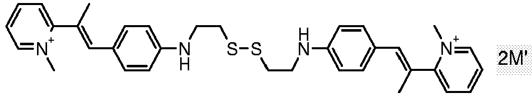 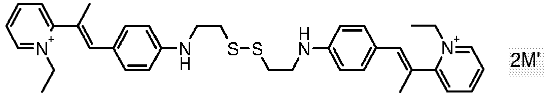 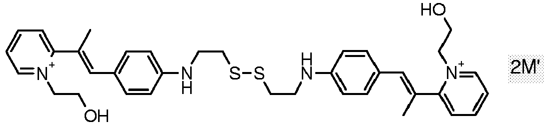 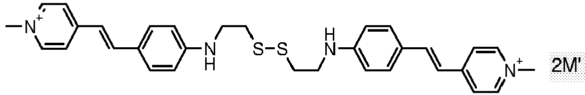 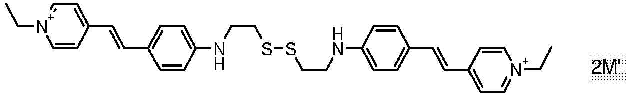 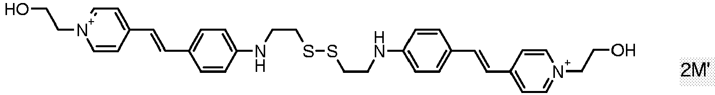 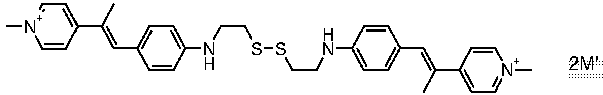 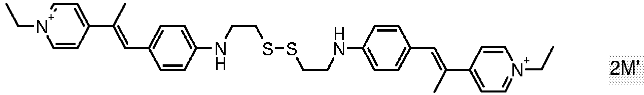 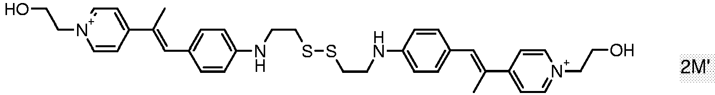 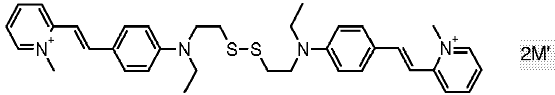 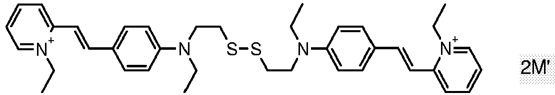 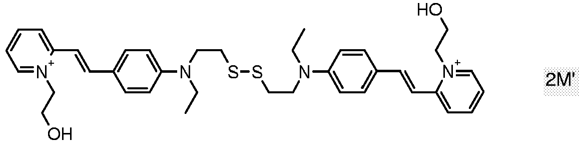 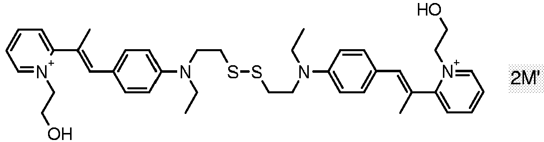 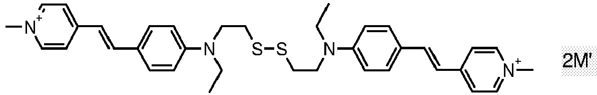 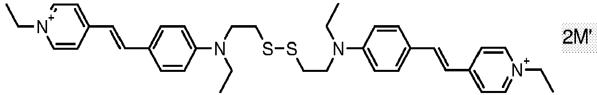 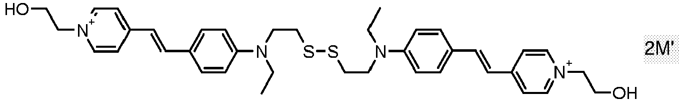 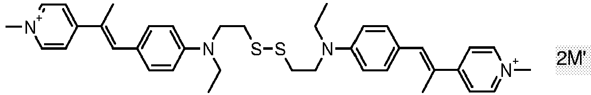 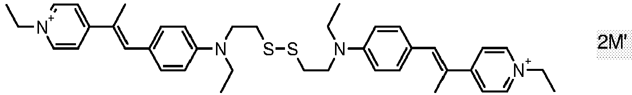 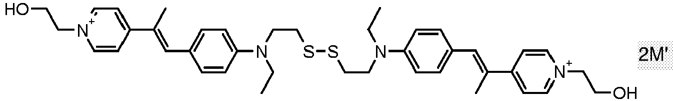 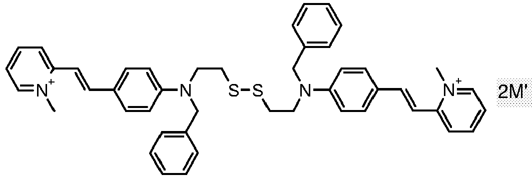 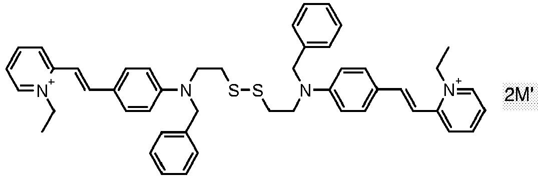 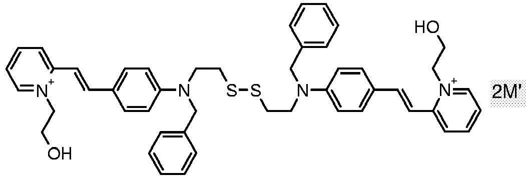 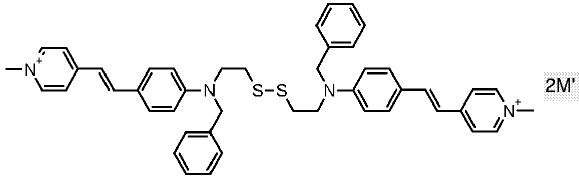 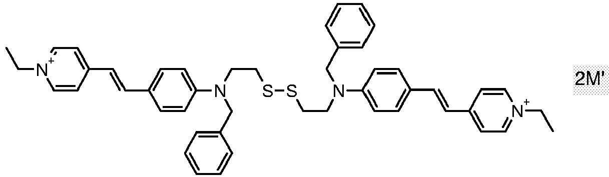 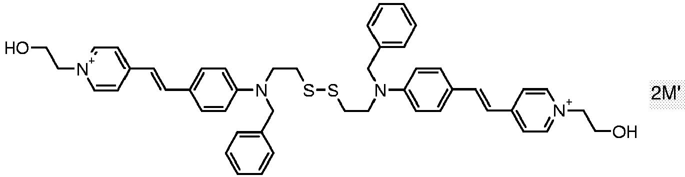 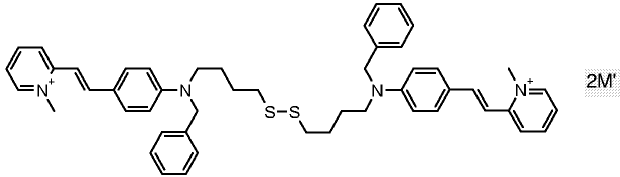 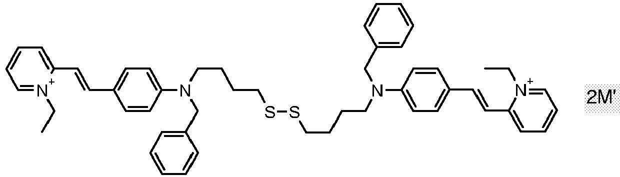 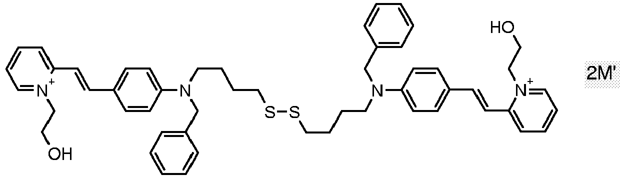 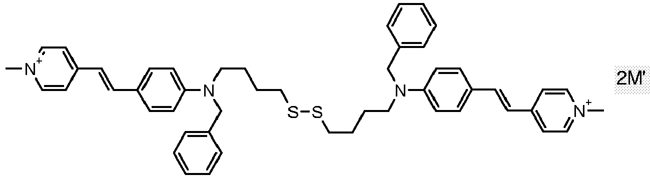 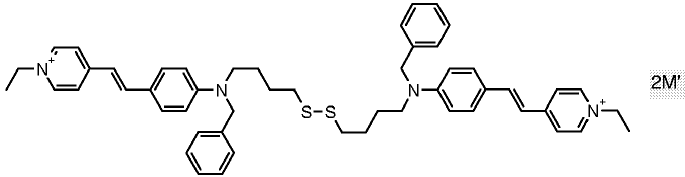 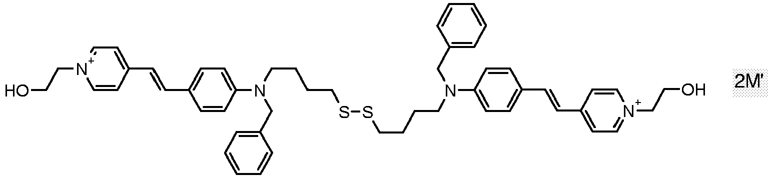 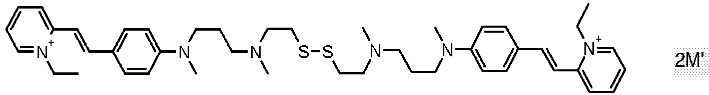 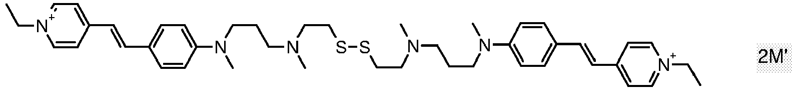 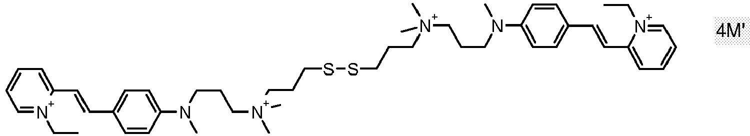 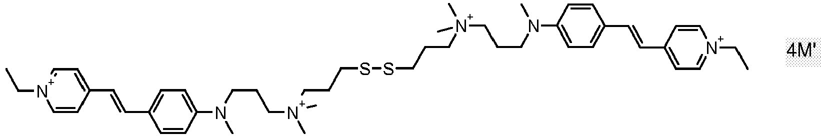 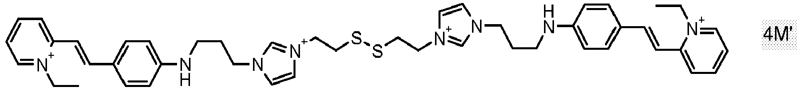 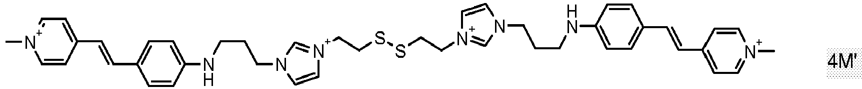 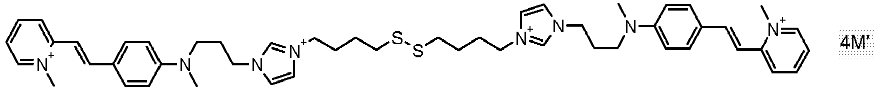 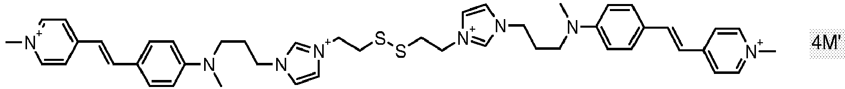 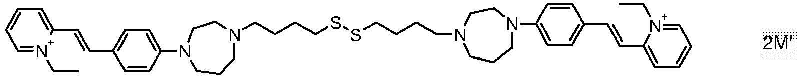 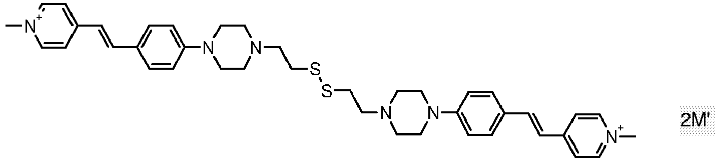 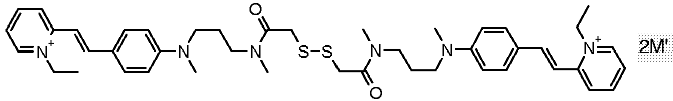 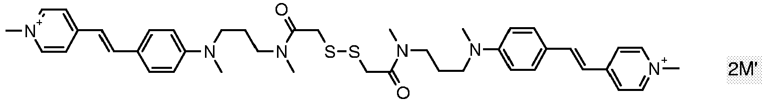 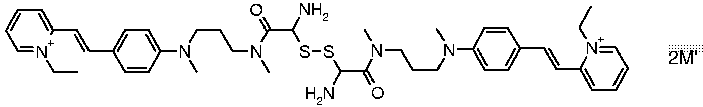 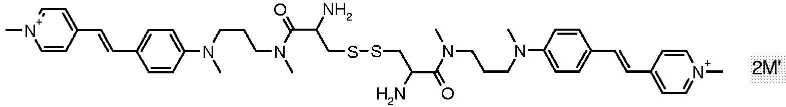 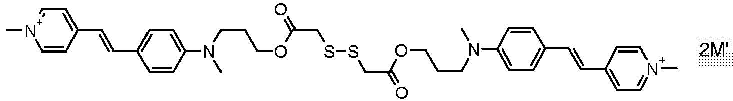 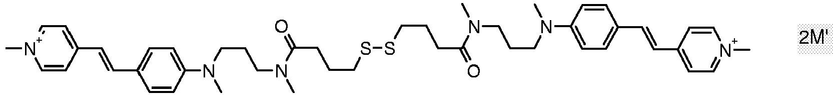 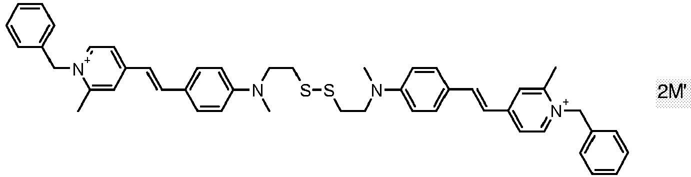 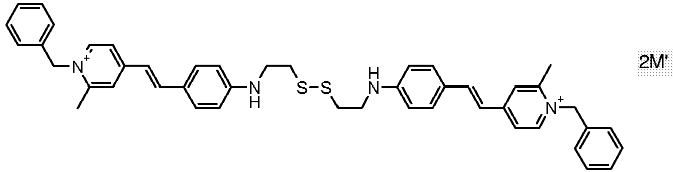 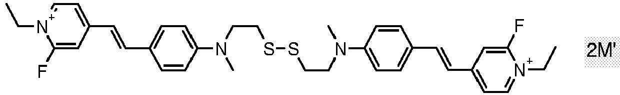 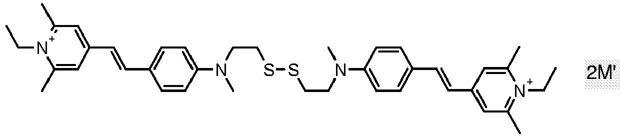 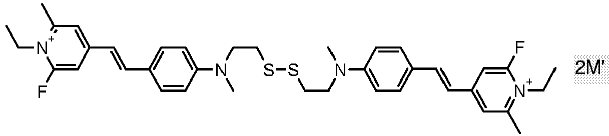 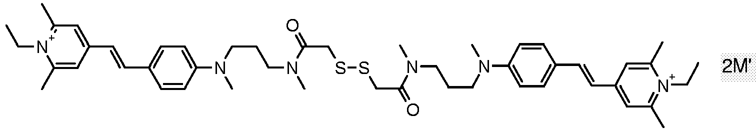 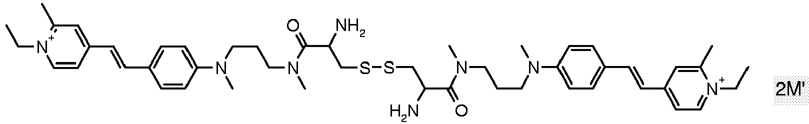 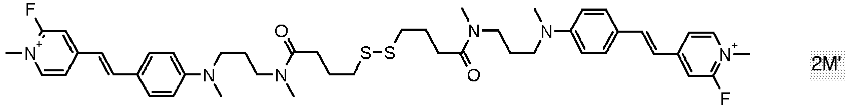 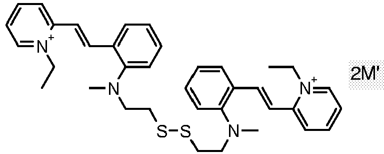 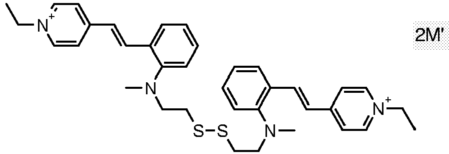 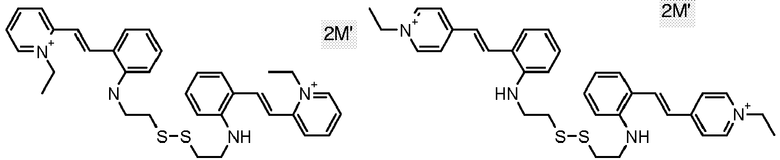 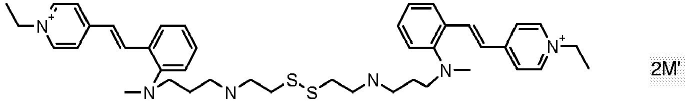 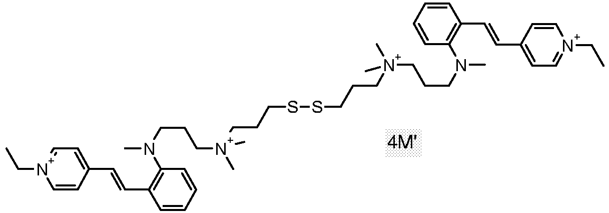 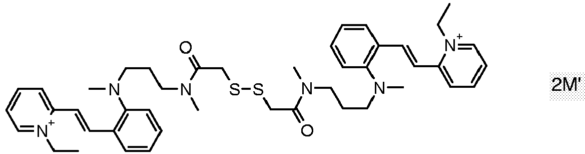 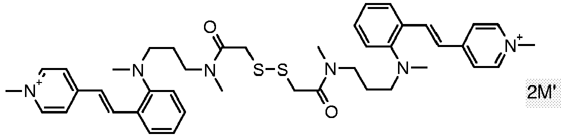 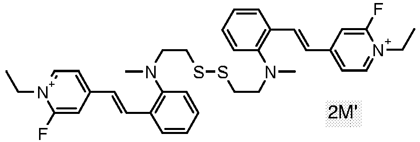 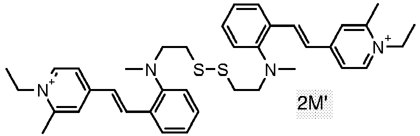 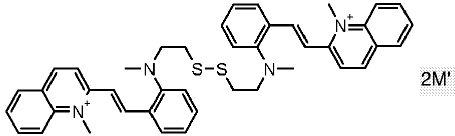 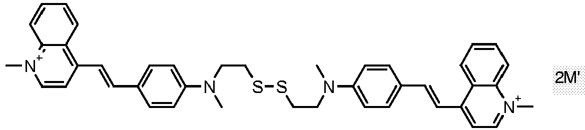 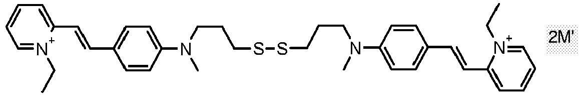 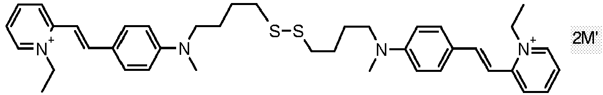 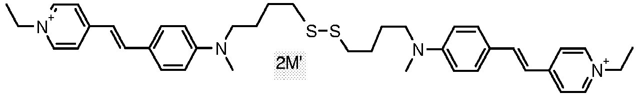 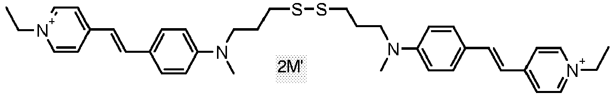 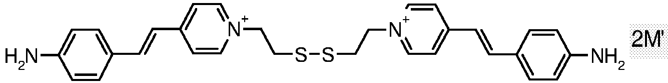 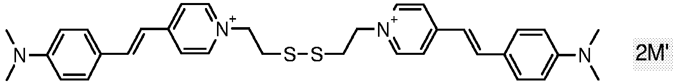 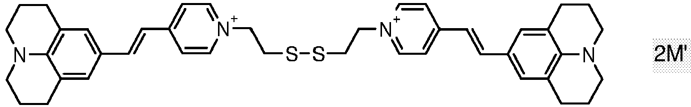 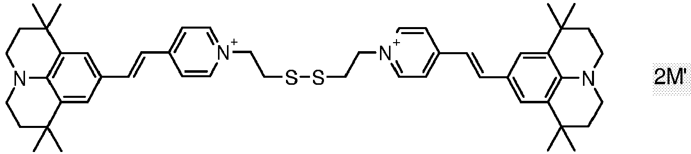 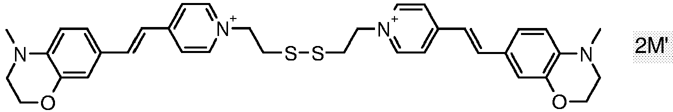 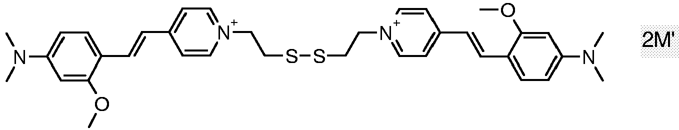 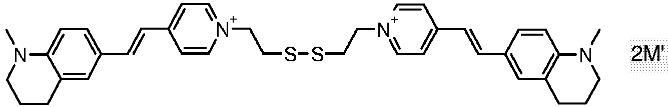 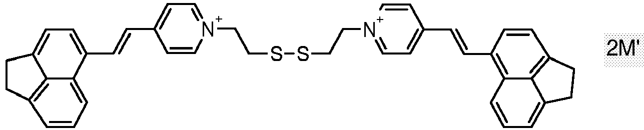 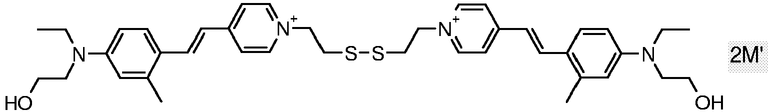 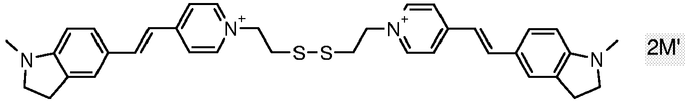 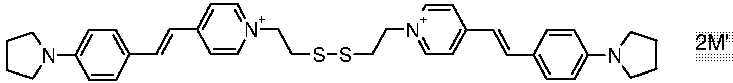 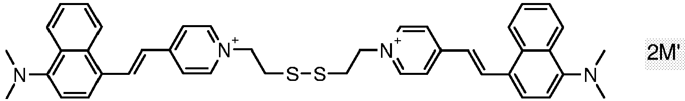 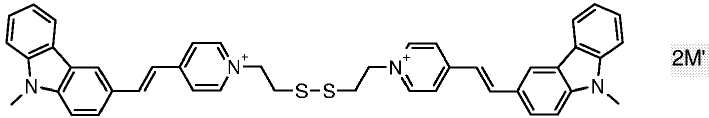 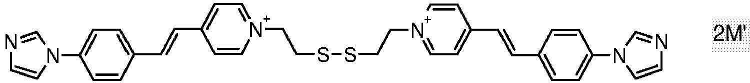 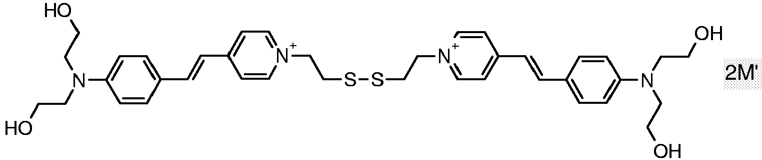 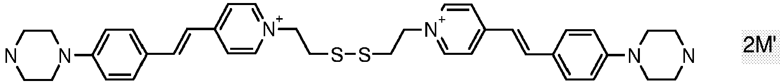 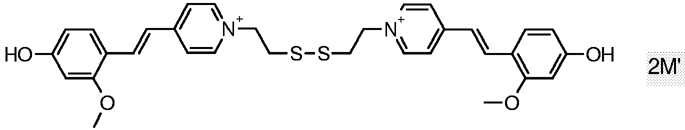 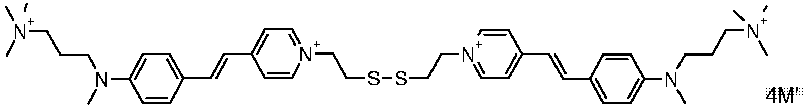 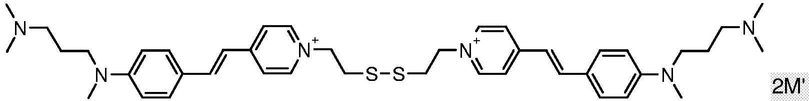 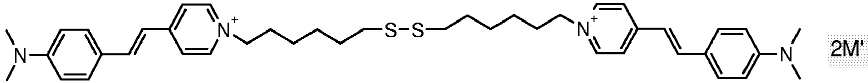 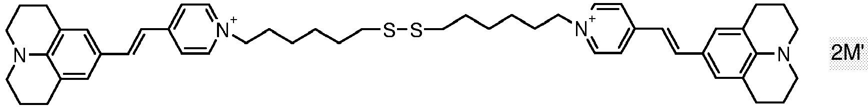 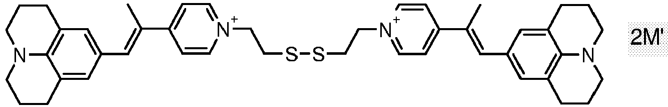 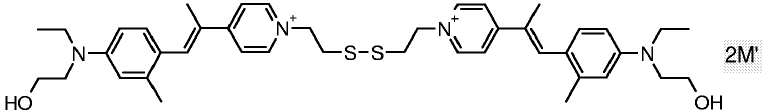 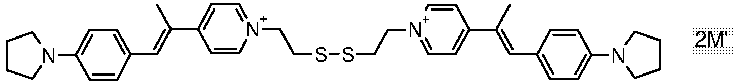 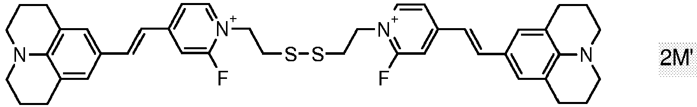 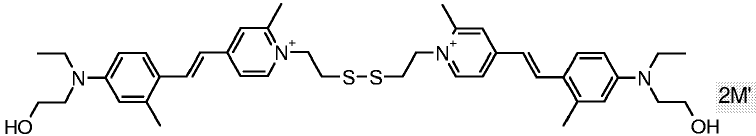 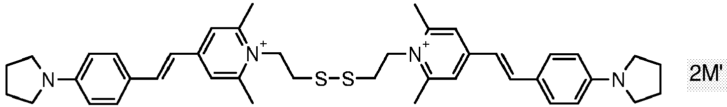 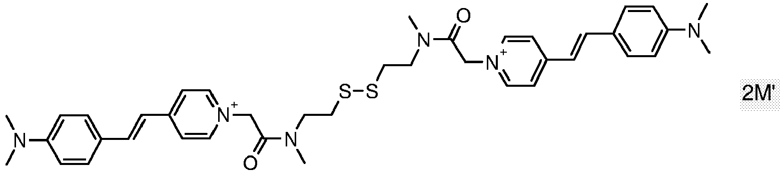 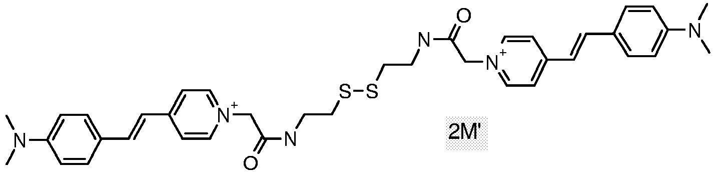 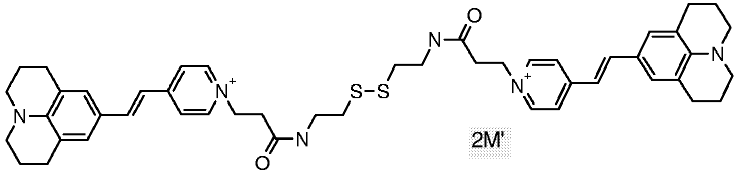 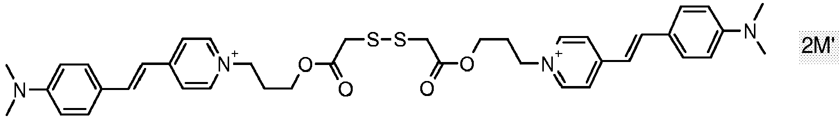 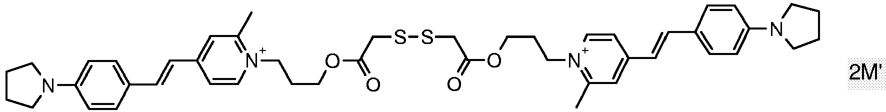 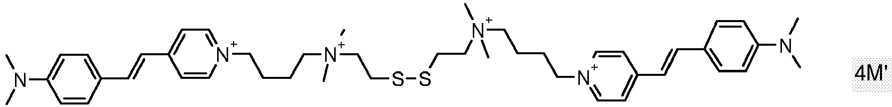 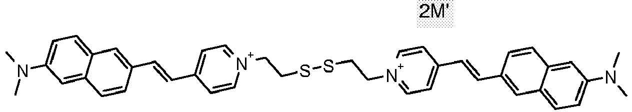 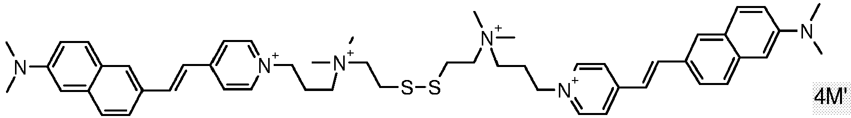 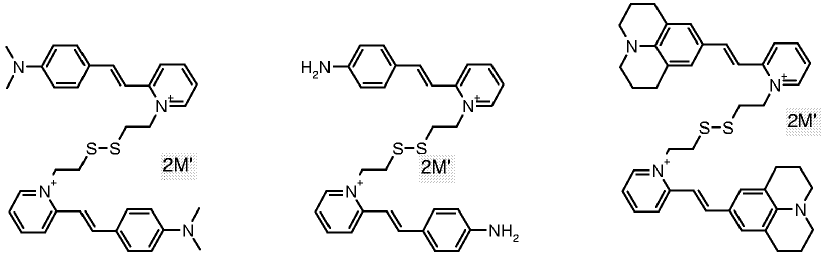 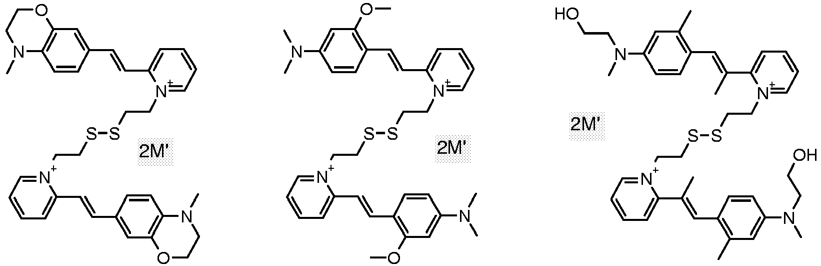 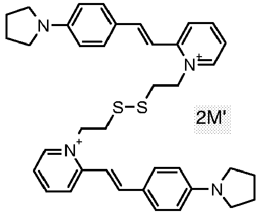 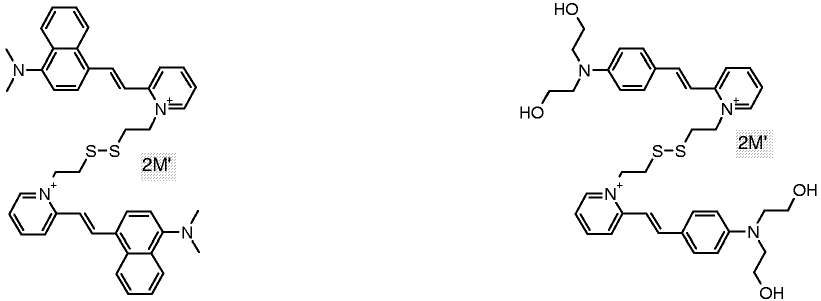 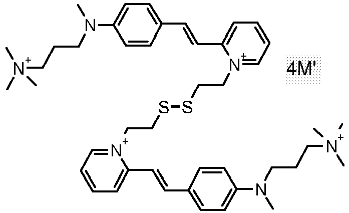 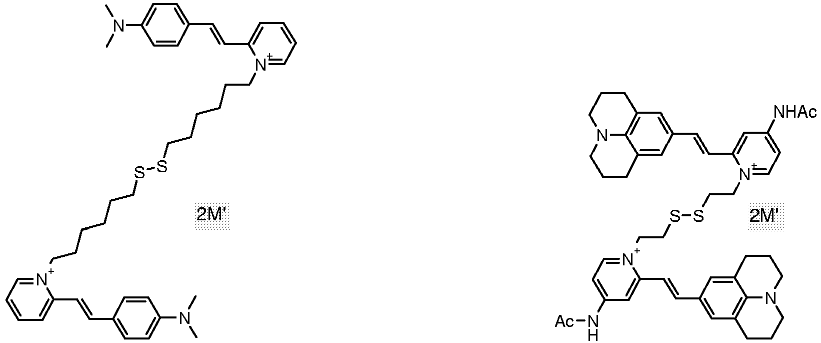 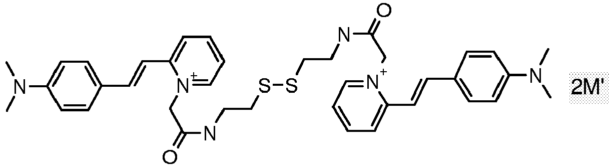 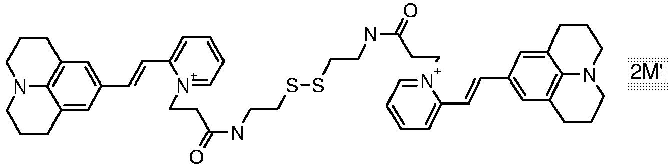 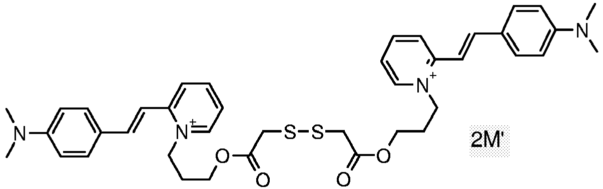 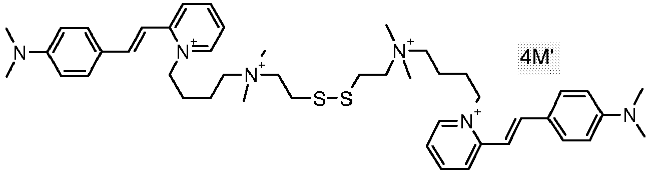 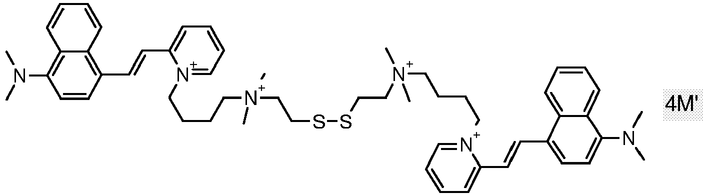 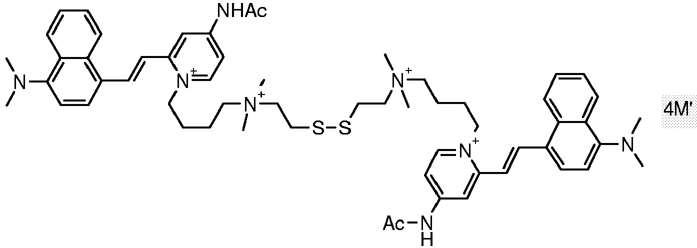 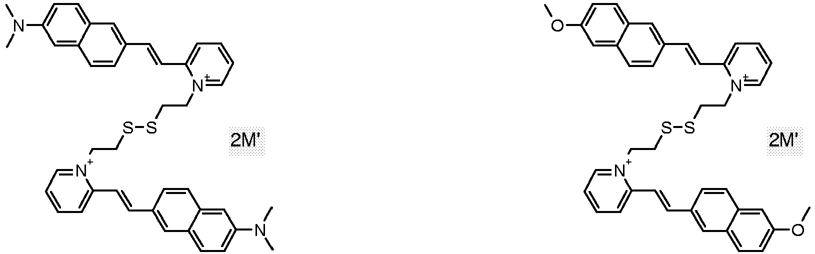 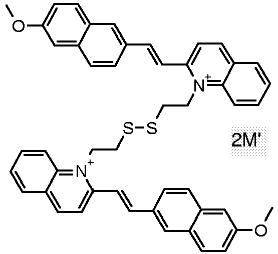 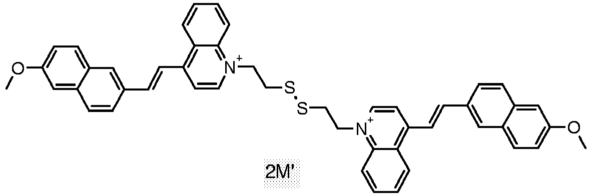 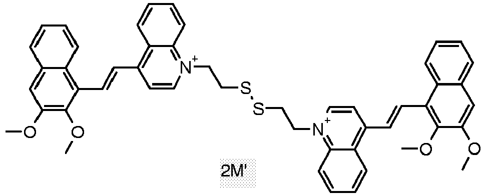 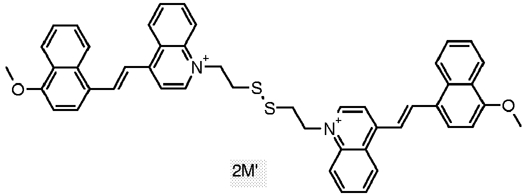 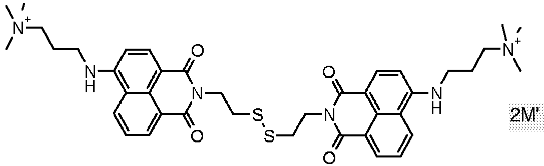 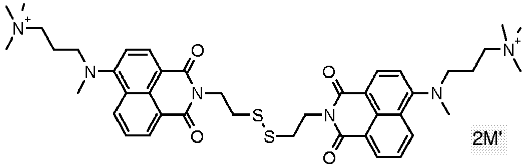 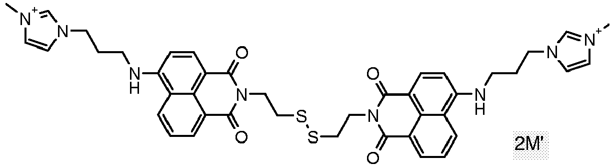 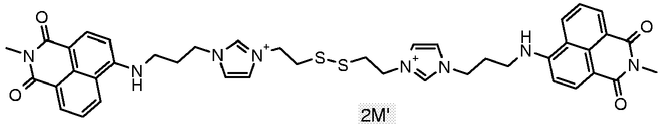 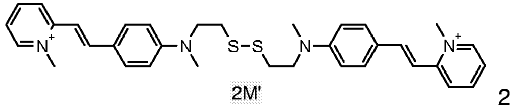 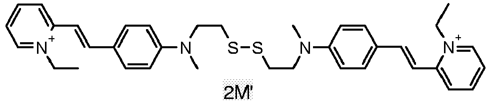 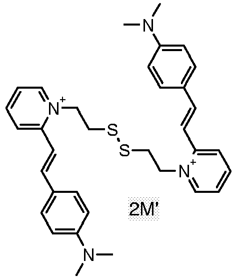 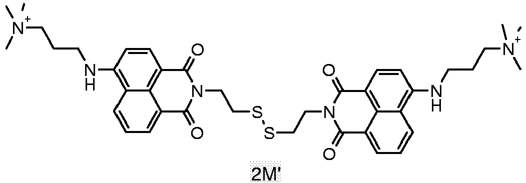 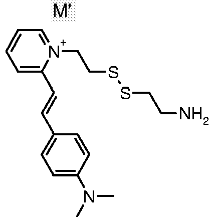 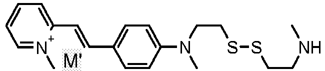 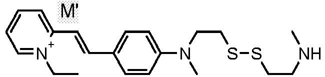 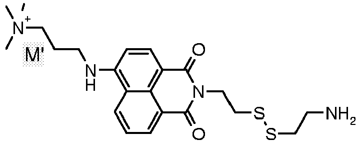 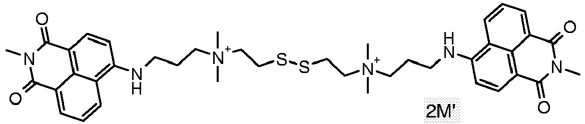 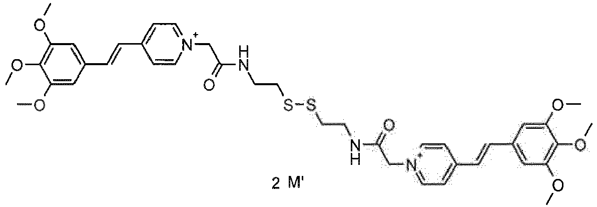 avec M' un contre-ion anionique.

10. Procédé selon une quelconque des revendications précédentes **caractérisé en ce que** l'agent réducteur est choisi parmi la cystéine, l'homocystéine, l'acide thiolactique, les sels de ces thiols, les phosphines, le bisulfite, les sulfites et l'acide thioglycolique, ainsi que les esters de l'acide thioglycolique.

11. Procédé selon une quelconque des revendications précédentes **caractérisé en ce que** les matières kératiniques foncées sont des fibres kératiniques, possédant une hauteur de ton inférieure ou égale à 6, de préférence inférieure ou égale à 4.

12. Procédé selon l'une quelconque des revendications précédentes comprenant une étape supplémentaire consistant à appliquer sur les fibres kératiniques un agent oxydant.

13. Procédé selon l'une quelconque des revendications précédentes, dans lequel le colorant fluorescent disulfure est présent en quantité comprise entre 0,001 et 50% en poids, de préférence, entre 0,005 et 20% en poids et encore plus préférentiellement entre 0,01 et 5% en poids, par rapport au poids total de la composition tinctoriale.

14. Utilisation des colorants fluorescents disulfures tels que définis aux revendications 1 à 9 pour la teinture de fibres kératiniques humaines foncées de hauteur de ton inférieur ou égal à 6, en particulier les cheveux.

15. Utilisation selon la revendication précédente pour l'éclaircissement des fibres kératiniques foncées de hauteur de ton inférieur ou égal à 4.

## Patentansprüche

1. Verfahren zum Färben von dunklen Keratinmaterialien, **dadurch gekennzeichnet, dass** man auf die Materialien mindestens einen Disulfid-Fluoreszenzfarbstoff gleichzeitig mit einem Reduktionsmittel aufbringt.

2. Verfahren nach dem vorhergehenden Anspruch, **dadurch gekennzeichnet, dass** man eine Färbezusammensetzung, die in einem geeigneten kosmetischen Medium mindestens einen Disulfid-Fluoreszenzfarbstoff umfasst, in Gegenwart eines Reduktionsmittels aufbringt.

3. Verfahren nach einem der vorhergehenden Ansprüche, **dadurch gekennzeichnet, dass** der Disulfid-Fluoreszenzfarbstoff aus den Farbstoffen der Formeln (I) und (II) gemäß nachstehender Definition:
A-(X)ₚ-Cₛₐₜ-S-S-C'ₛₐₜ-(X')_{p'}-A' (I)
A-(X)ₚ-Cₛₐₜ-S-S-C'ₛₐₜ-(X')_{p'}-D (II),
organischen oder anorganischen Säuresalzen davon, optischen Isomeren davon, geometrischen Isomeren davon und Solvaten davon wie Hydraten ausgewählt wird,
wobei in den Formeln:
• A und A' gleich oder verschieden sind und für einen Rest mit mindestens einem kationischen oder nichtkationischen Fluoreszenzchromophor stehen;
• X und X' gleich oder verschieden sind und für eine gesättigte oder ungesättigte, lineare oder verzweigte C₁-C₃₀-Kohlenwasserstoffkette, die gegebenenfalls durch eine oder mehrere zweiwertige Gruppen oder Kombinationen davon, die aus:
- -N(R)-; -N⁺(R)(R)-; -O-; -S-; -CO- und -SO₂-, wobei R gleich oder verschieden ist und aus einem Wasserstoff und einem C₁-C₄-Alkyl-, Hydroxyalkyl- und Aminoalkylrest ausgewählt ist;
- einem kondensierten oder nichtkondensierten, gesättigten oder ungesättigten, aromatischen oder nichtaromatischen (hetero)cyclischen Rest, der gegebenenfalls ein oder mehrere gleiche oder verschiedene Heteroatome enthält und gegebenenfalls substituiert ist, ausgewählt sind, unterbrochen und/oder an einem oder beiden ihrer Enden durch diese Gruppen oder Kombinationen davon terminiert ist, stehen;
• die Indices p und p' gleich oder verschieden sind und gleich 0 oder 1 sind;
• Cₛₐₜ und C'ₛₐₜ gleich oder verschieden sind und für eine gegebenenfalls cyclische, gegebenenfalls substituierte, lineare oder verzweigte C₁-C₁₈-Alkylenkette stehen;
• D einem aus Hydroxy-, Hydroxyalkyl-, Alkoxy-, Carboxyl-, Carboxylat-, Amino-, Alkylamino- und Dialkylaminoresten ausgewählten Rest entspricht.

4. Verfahren nach dem vorhergehenden Anspruch, **dadurch gekennzeichnet, dass** der Disulfid-Fluoreszenzfarbstoff der Formel (I) oder (II) einen Rest A oder A', die gleich oder verschieden sind, mit einem Styryl-Chromophor umfasst.

5. Verfahren nach einem der beiden vorhergehenden Ansprüche, **dadurch gekennzeichnet, dass** X und X' gleich oder verschieden sind und für eine Gruppe - N(R)- stehen.

6. Verfahren nach einem der Ansprüche 3 bis 5, **dadurch gekennzeichnet, dass** A und A' gleich oder verschieden, weiter bevorzugt gleich, sind und für W-C(R^{c})=C(R^{d})-Ar- oder -W-C(R^{c})=C(R^{d})-Ar stehen, wobei W für einen Heterocyclus oder ein Heteroaryl mit einem quaternären Ammonium steht; Ar für einen 5- oder 6-gliedrigen (Hetero)arylrest vom Phenyl- oder Pyridium-Typ oder einen (hetero)aromatischen bicyclischen Rest vom Naphthyl-, Benzopyridinium-, Indolinyl- oder Benzoindolinyl-Typ, der gegebenenfalls durch ein oder mehrere Halogenatome, durch eine oder mehrere Alkylgruppen, durch eine oder mehrere Hydroxylgruppen, durch eine oder mehrere Alkoxygruppen, durch eine oder mehrere Hydroxyalkylgruppen, durch eine oder mehrere Amino- oder (Di)alkylaminogruppen; durch eine oder mehrere Acylaminogruppen oder durch eine oder mehrere 5- oder 6-gliedrige Heteroaryl- oder Heterocycloalkylgruppen substituiert ist, steht und R^{c} und R^{d} gleich oder verschieden sind und für ein Wasserstoffatom oder eine C₁-C₄-Alkylgruppe stehen.

7. Verfahren nach einem der Ansprüche 3 bis 6, **dadurch gekennzeichnet, dass** W für ein Imidazolium, Pyridinium, Benzopyridinium, Benzimidazolium, Chinolinium oder Pyrazolium steht.

8. Verfahren nach einem der vorhergehenden Ansprüche, **dadurch gekennzeichnet, dass** der Disulfid-Fluoreszenzfarbstoff aus den Verbindungen (III) bis (XII) ausgewählt ist: 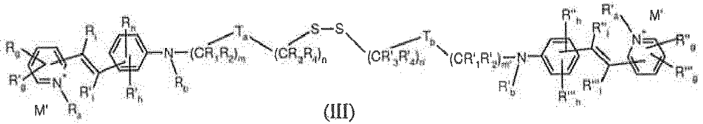 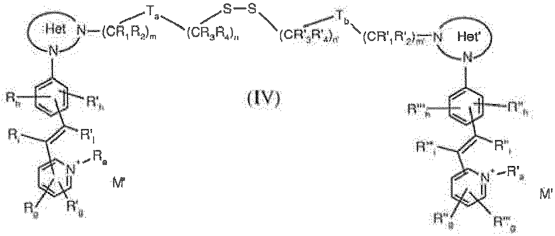 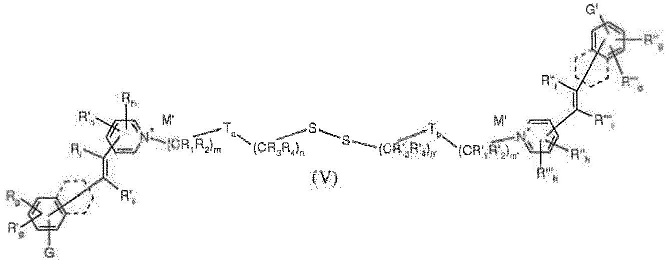 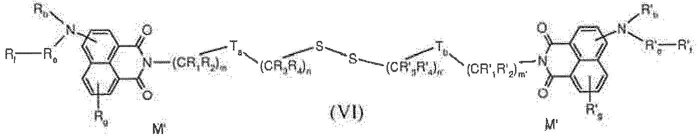 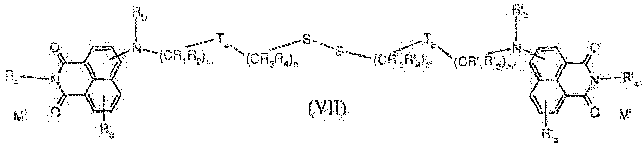 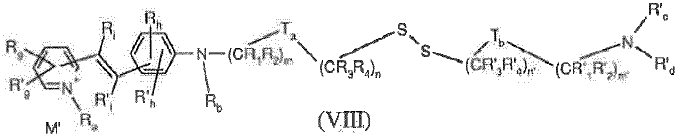 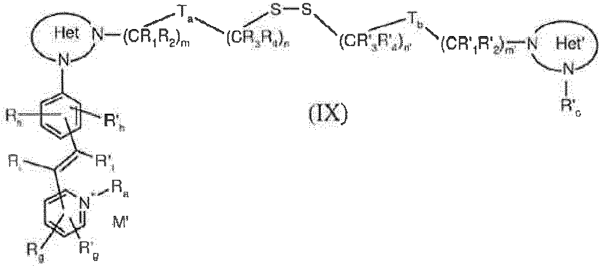 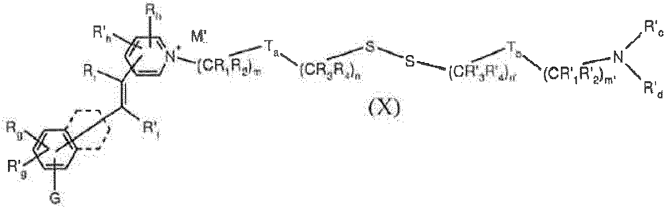 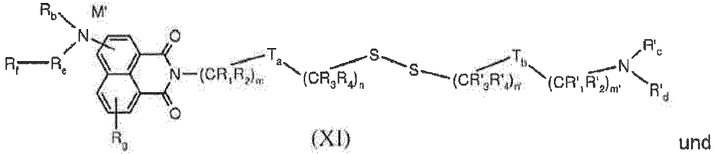 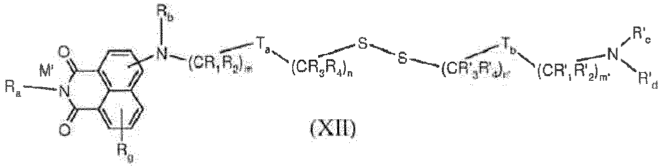 worin
• G und G' gleich oder verschieden sind und für eine -NR_{c}R_{d}-, -NR'_{c}R'_{d}- oder C₁-C₆-Alkoxygruppe, die gegebenenfalls substituiert ist, stehen; G und G' vorzugsweise für eine -NR_{c}R_{d}- bzw. -NR'_{c}R'_{d}-Gruppe stehen;
• Rₐ und R'ₐ gleich oder verschieden sind und für eine Aryl (C₁-C₄) alkylgruppe oder eine C₁-C₆-Alkylgruppe, die gegebenenfalls durch eine Hydroxyl- oder Amino-, C₁-C₄-Alkylamino- oder C₁-C₄-Dialkylaminogruppe substituiert ist, stehen, wobei die Alkylreste mit dem Stickstoffatom, an das sie gebunden sind, einen 5-bis 7-gliedrigen Heterocyclus bilden können, der gegebenenfalls ein weiteres Heteroatom, das mit Stickstoff identisch oder davon verschieden ist, enthält;
• R_{b} und R'_{b} gleich oder verschieden sind und für ein Wasserstoffatom, eine Aryl(C₁-C₄)alkylgruppe oder eine C₁-C₆-Alkylgruppe, die gegebenenfalls substituiert ist, stehen;
• R_{c}, R'_{c}, R_{d} und R'_{d} gleich oder verschieden sind und für ein Wasserstoffatom, eine Aryl(C₁-C₄) alkylgruppe, eine C₁-C₆-Alkoxygruppe oder eine C₁-C₆-Alkylgruppe, die gegebenenfalls substituiert ist, stehen;
oder auch zwei benachbarte Reste R_{c} und R_{d} oder R'_{c} und R'_{d}, die an dasselbe Stickstoffatom gebunden sind, zusammen eine heterocyclische Gruppe oder Heteroarylgruppe bilden;
• Rₑ und R'ₑ gleich oder verschieden sind und für eine gegebenenfalls ungesättigte, lineare oder verzweigte, zweiwertige C₁-C₆-Alkylenyl-Kohlenwasserstoffkette stehen;
• R_{f} und R'_{f} gleich oder verschieden sind und für eine quaternäre Ammoniumgruppe (R")(R"')(R"")N⁺-stehen, wobei R", R"' und R"" gleich oder verschieden sind und für ein Wasserstoffatom oder eine C₁-C₄-Alkylgruppe stehen oder auch (R" (R"')(R"")N⁺- für eine gegebenenfalls substituierte kationische Heteroarylgruppe, vorzugsweise eine gegebenenfalls durch eine C₁-C₃-Alkylgruppe substituierte Imidazoliniumgruppe, steht;
• R_{g}, R'_{g}, R"_{g}, R'"_{g}, Rₕ, R'ₕ, R"ₕ und R"'ₕ gleich oder verschieden sind und für ein Wasserstoffatom, ein Halogenatom, eine Amino-, C₁-C₄-Alkylamino-, C₁-C₄-Dialkylamino-, Cyano-, Carboxy-, Hydroxyl- oder Trifluormethylgruppe, einen Acylamino-, C₁-C₄-Alkoxy-, (Poly)hydroxy-(C₂-C₄)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, stehen oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält;
oder auch zwei Gruppen R_{g} und R'_{g}; R"_{g} und R"'_{g}; Rₕ und R'ₕ; R"ₕ und R"'ₕ, die an zwei benachbarte Kohlenstoffatome gebunden sind, zusammen einen Benzo- oder Indenoring oder eine kondensierte Heterocycloalkyl- oder kondensierte Heteroarylgruppe bilden; wobei der Benzo-, Indeno-, Heterocycloalkyl- oder Heteroarylring gegebenenfalls durch ein Halogenatom, eine Amino-, C₁-C₄-Alkylamino-, C₁-C₄-Dialkylamino-, Nitro-, Cyano-, Carboxy-, Hydroxyl- oder Trifluormethylgruppe, einen Acylamino-, C₁-C₄-Alkoxy-, (Poly)hydroxy(C₂-C₄)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, substituiert ist oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält;
• oder auch dann, wenn G für -NR_{c}R_{d} steht und G' für -NR'_{c}R'_{d} steht, zwei Gruppen R_{c} und R'_{g}; R'_{c} und R"_{g}; R_{d} und R_{g}; R'_{d} und R"'_{g} zusammen ein Heteroaryl oder einen gesättigten Heterocyclus, das bzw. der gegebenenfalls durch eine C₁-C₆-Alkylgruppe substituiert ist, bilden;
• Rᵢ, R'ᵢ, R"ᵢ und R"'ᵢ gleich oder verschieden sind und für ein Wasserstoffatom oder eine C₁-C₄-Alkylgruppe stehen;
• R₁, R₂, R₃, R₄, R'₁, R'₂, R'₃ und R'₄ gleich oder verschieden sind und für ein Wasserstoffatom oder eine C₁-C₄-Alkyl-, C₁-C₁₂-Alkoxy-, Hydroxy-, Cyano-, Carboxy-, Amino-, C₁-C₄-Alkylamino- oder C₁-C₄-Dialkylaminogruppe stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält;
• Tₐ und T_{b} gleich oder verschieden sind und für i) eine kovalente σ-Bindung, ii) einen oder mehrere Reste oder Kombinationen davon, die aus -SO₂-, -O-, -S-, -N(R)-, -N⁺(R)(R^{o})- und -CO-, wobei R und R^{o} gleich oder verschieden sind und für ein Wasserstoffatom oder einen C₁-C₄-Alkyl-, C₁-C₄-Hydroxyalkyl- oder Aryl (C₁-C₄) alkylrest stehen, ausgewählt sind, iii) oder einen kationischen oder nichtkationischen Heterocycloalkyl- oder Heteroarylrest stehen;
• 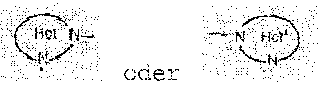 gleich oder verschieden sind und für eine gegebenenfalls substituierte heterocyclische Gruppe stehen;
• 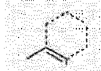 für eine mit einem Phenylring kondensierte Aryl- oder Heteroarylgruppe steht oder auch an dem Phenylring fehlt;
• m, m', n und n' gleich oder verschieden sind und für eine ganze Zahl zwischen 0 und 6 inklusive stehen, wobei m+n, m'+n' gleich oder verschieden sind und für eine ganze Zahl zwischen 1 und 10 inklusive stehen;
wobei M' für ein Gegenion oder ein organisches oder anorganisches Säuresalz steht.

9. Verfahren nach einem der vorhergehenden Ansprüche, **dadurch gekennzeichnet, dass** der Disulfid-Fluoreszenzfarbstoff ausgewählt ist aus: 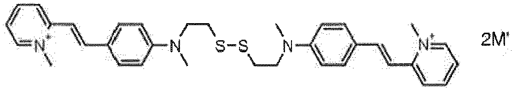 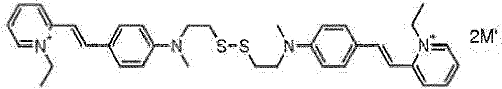 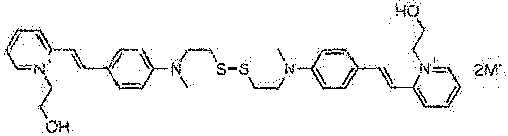 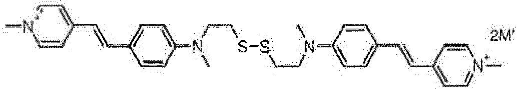 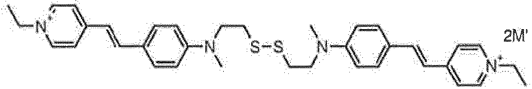 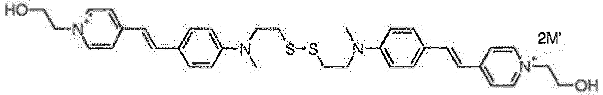 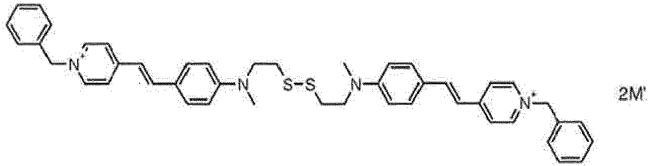 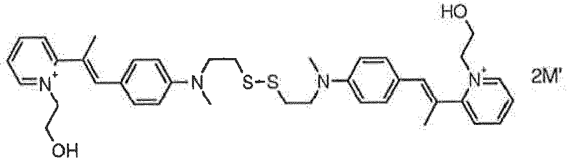 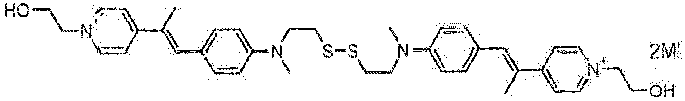 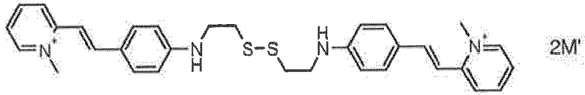 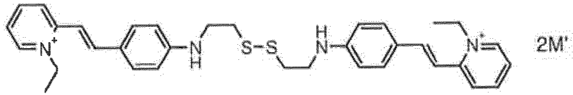 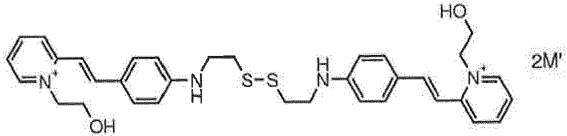 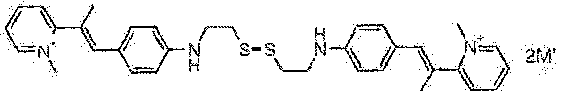 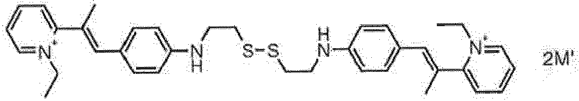 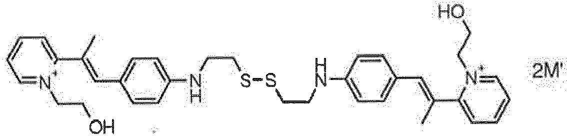 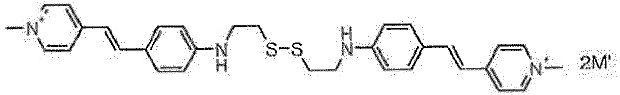 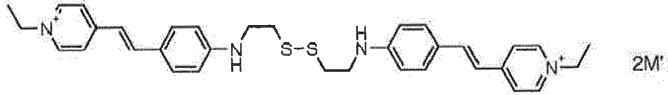 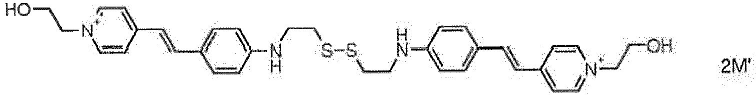 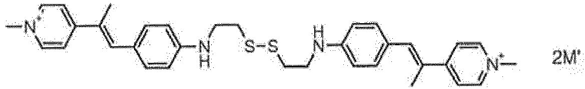 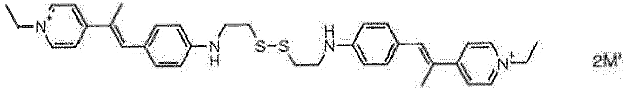 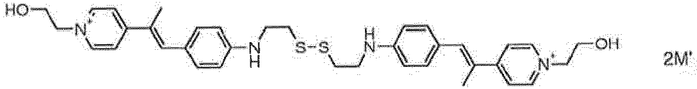 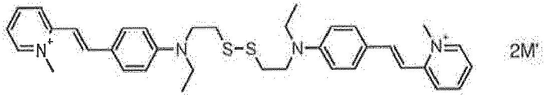 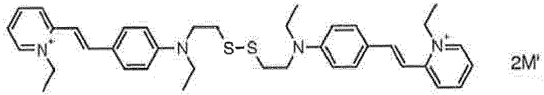 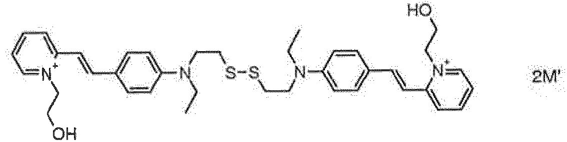 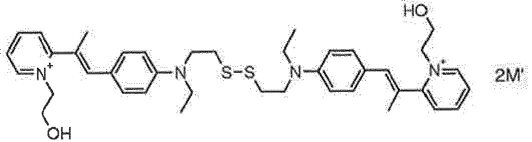 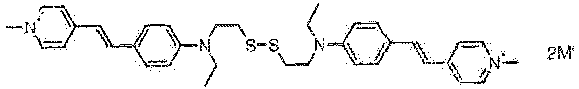 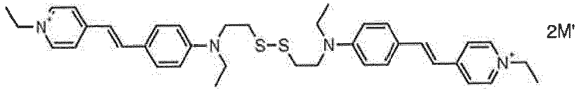 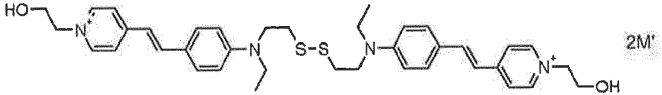 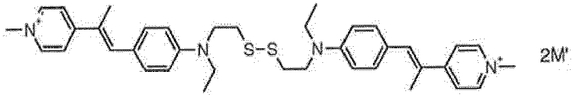 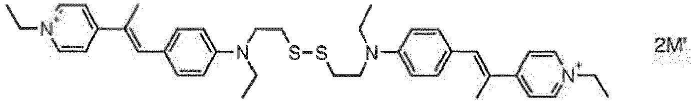 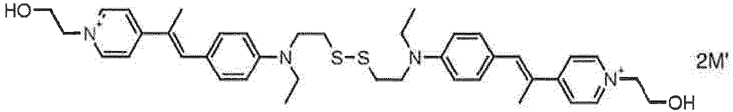 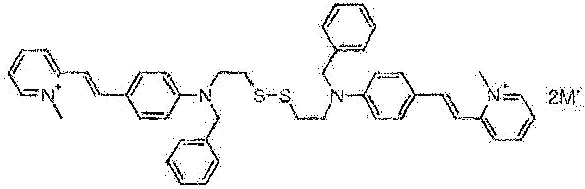 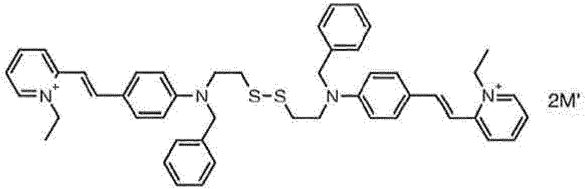 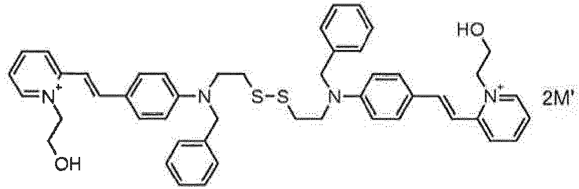 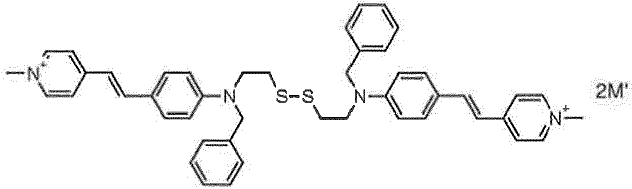 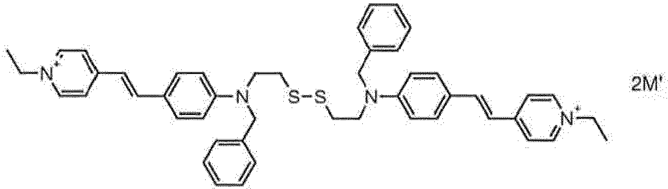 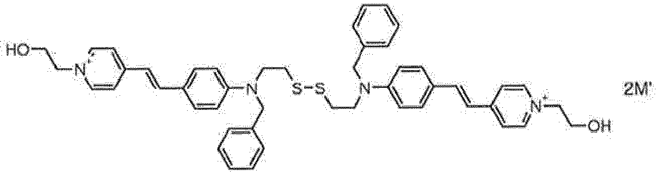 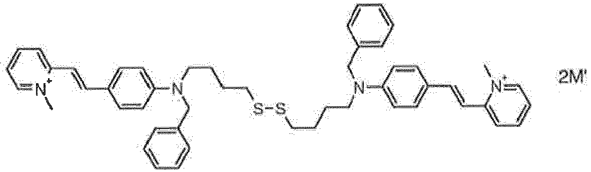 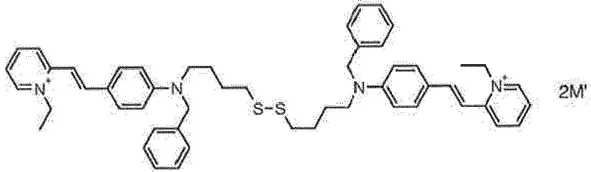 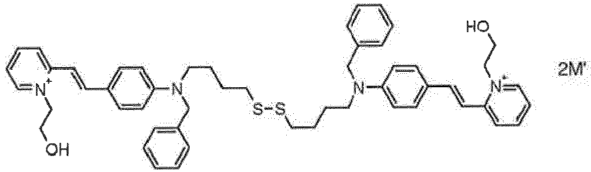 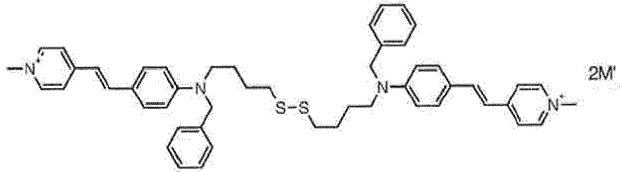 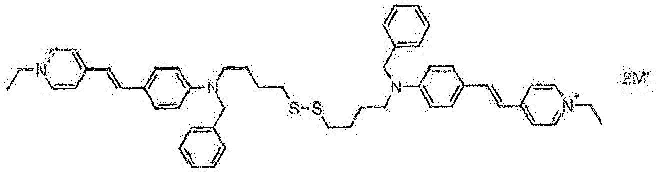 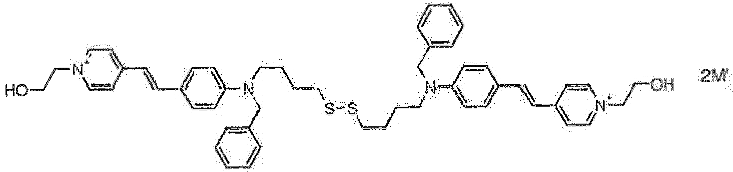 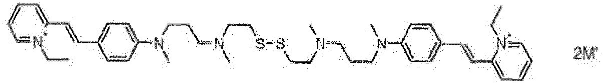 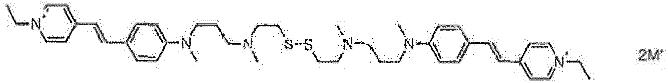 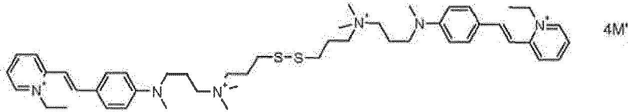 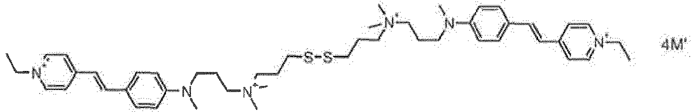 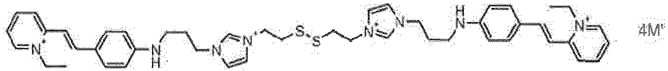 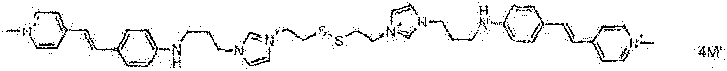 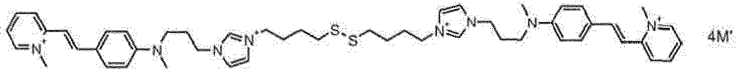 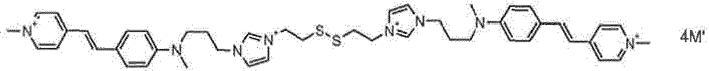 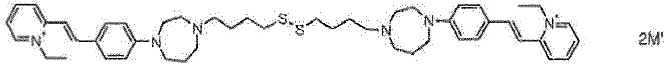 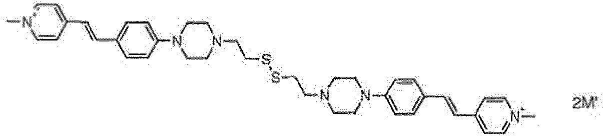 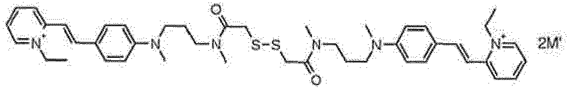 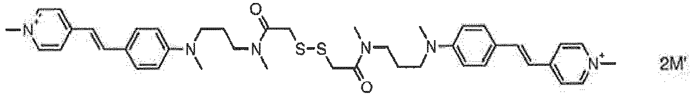 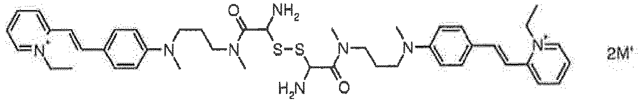 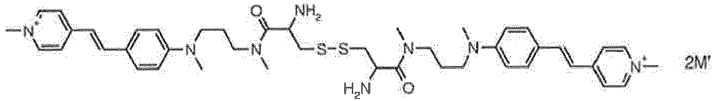 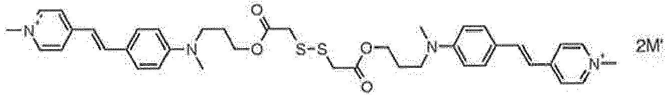 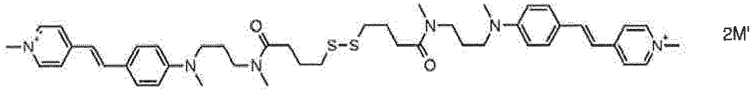 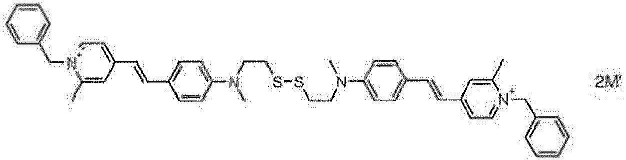 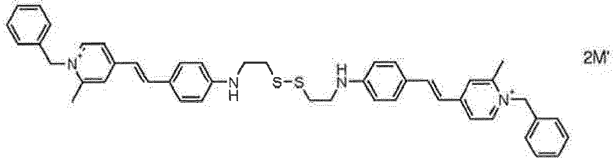 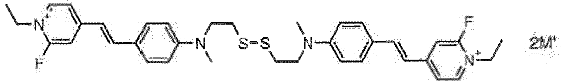 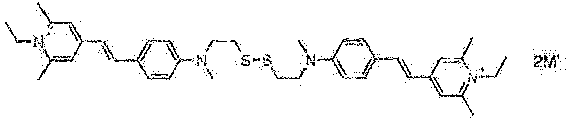 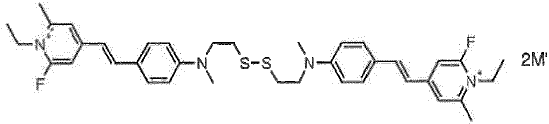 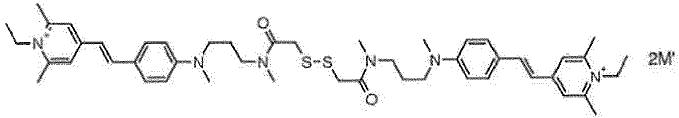 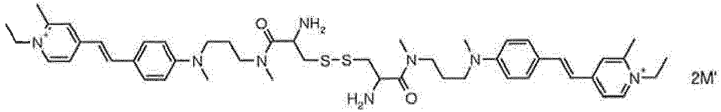 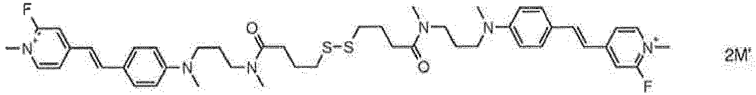 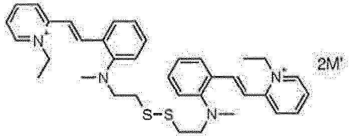 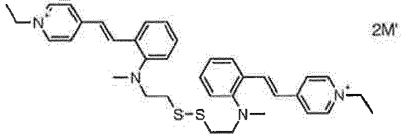 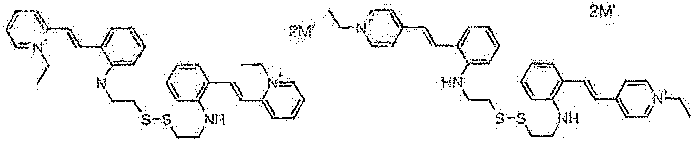 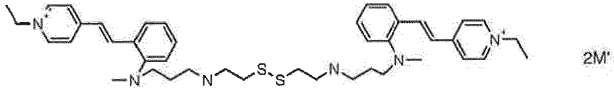 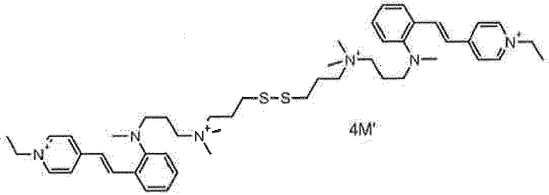 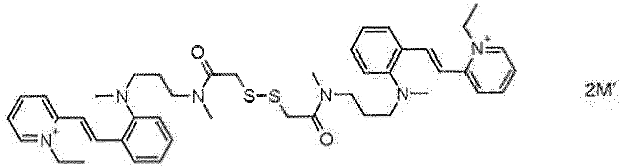 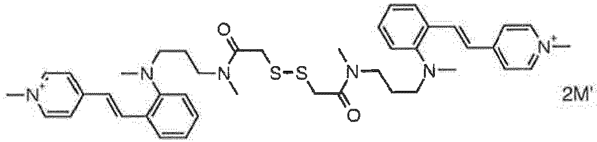 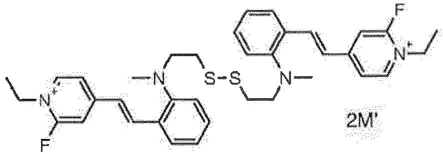 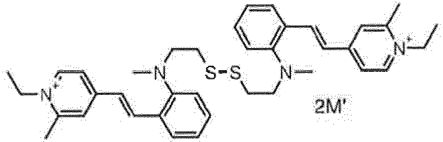 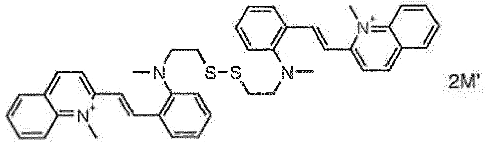 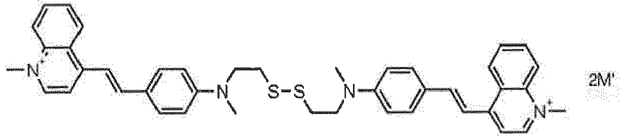 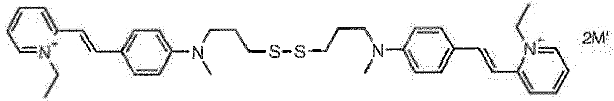 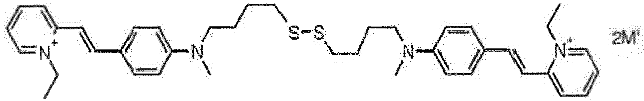 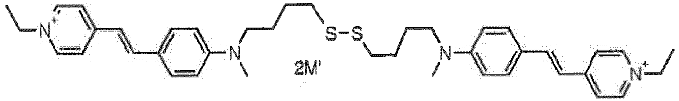 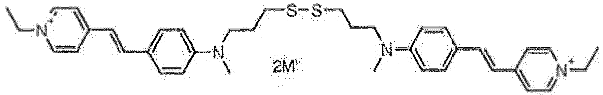 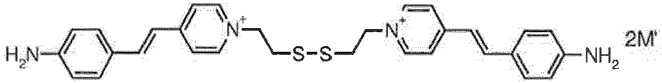 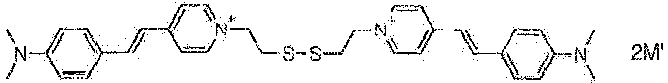 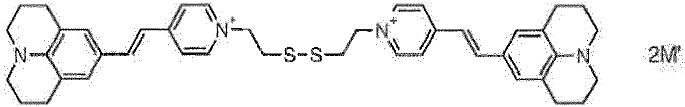 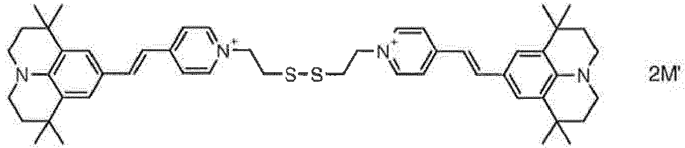 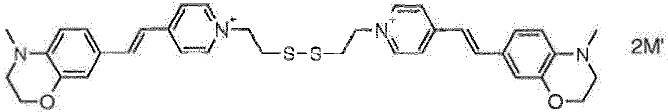 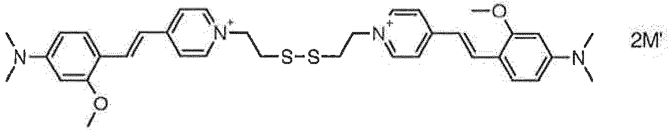 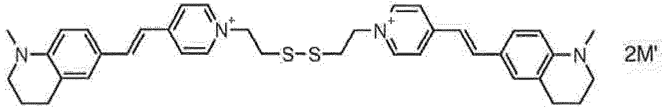 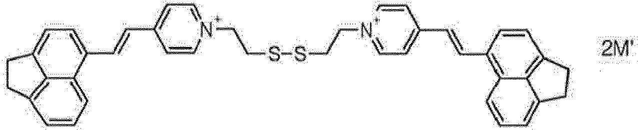 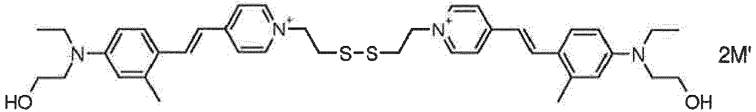 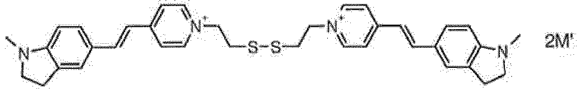 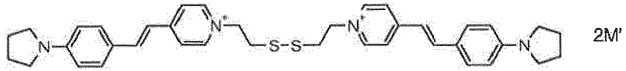 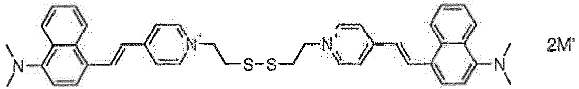 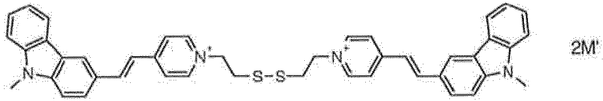 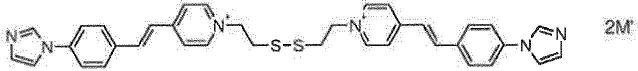 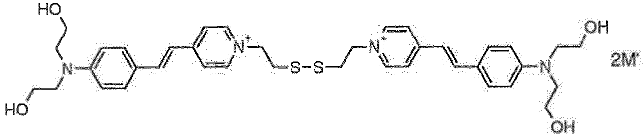 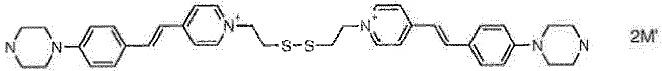 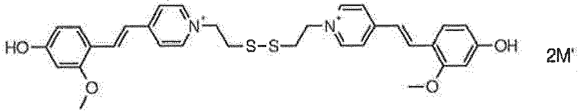 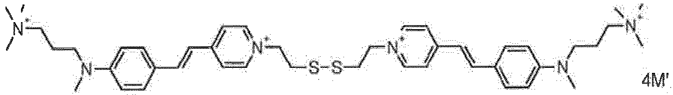 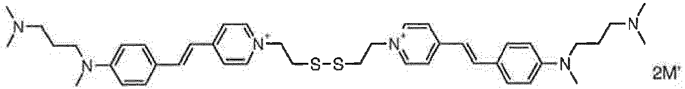 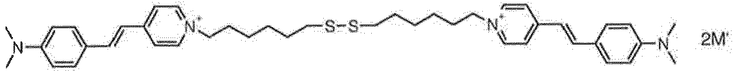 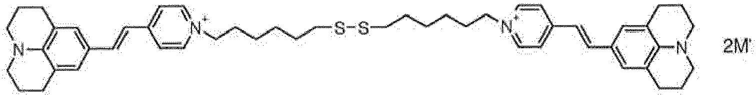 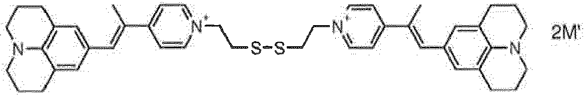 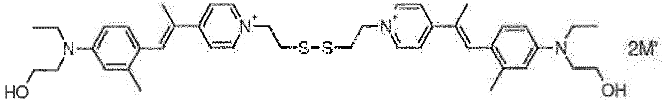 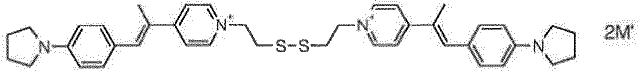 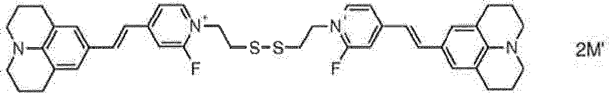 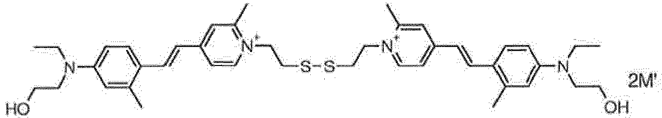 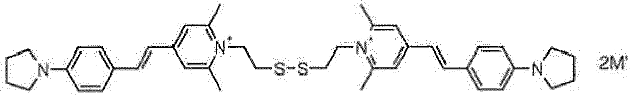 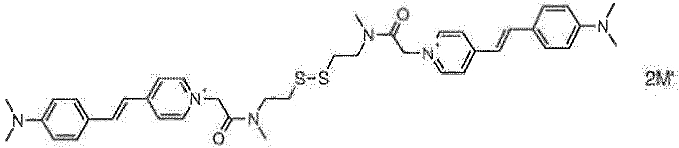 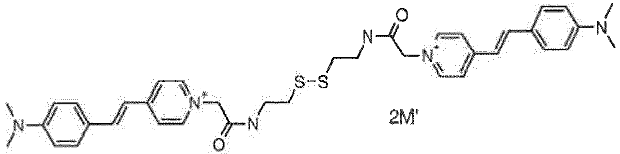 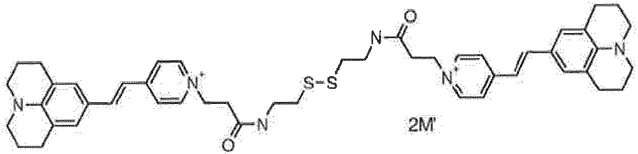 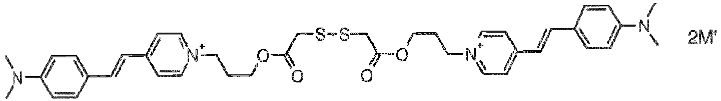 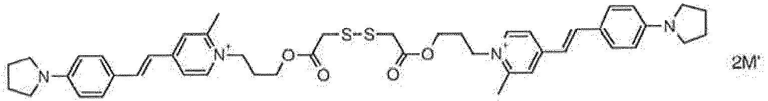 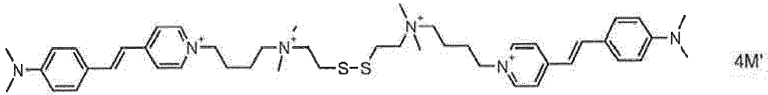 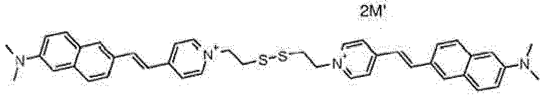 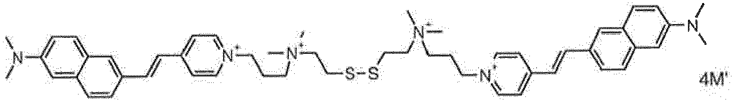 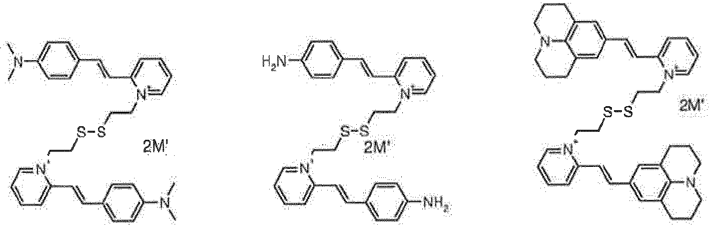 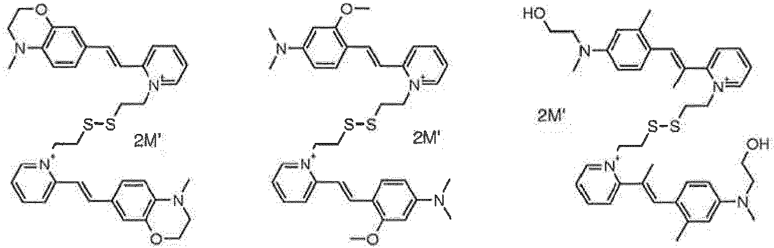 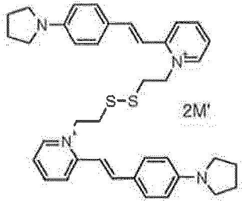 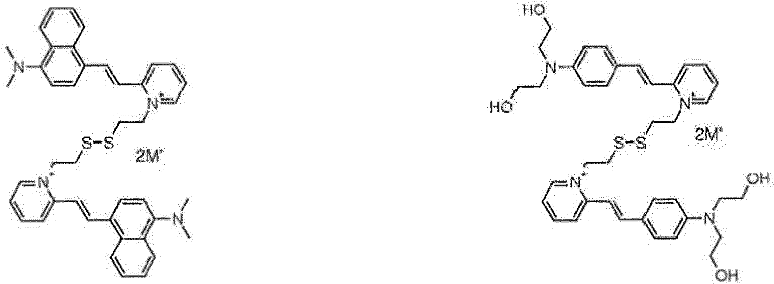 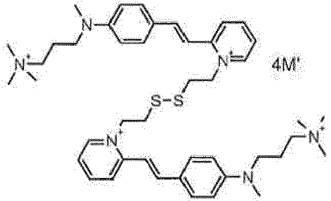 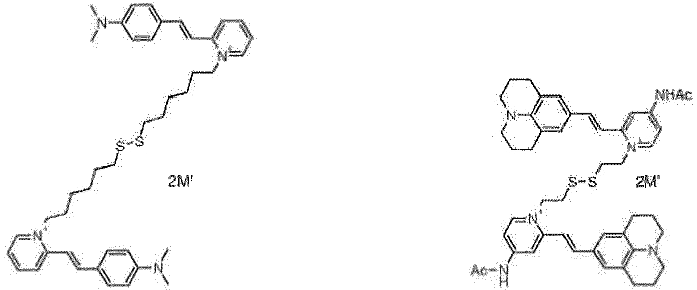 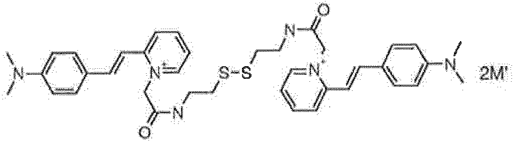 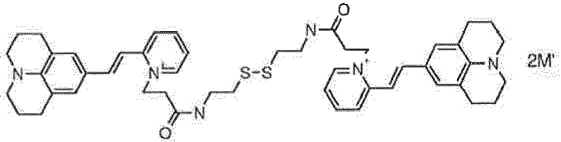 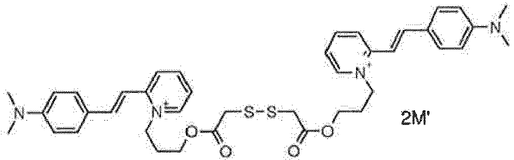 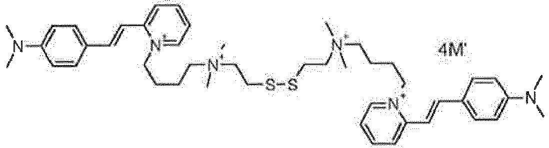 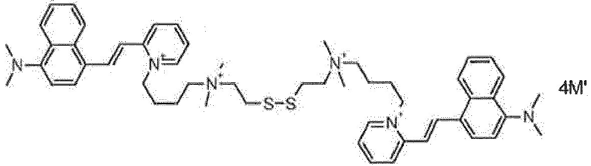 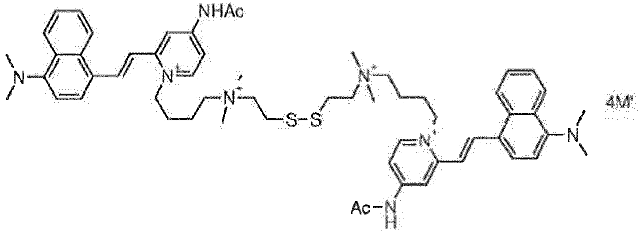 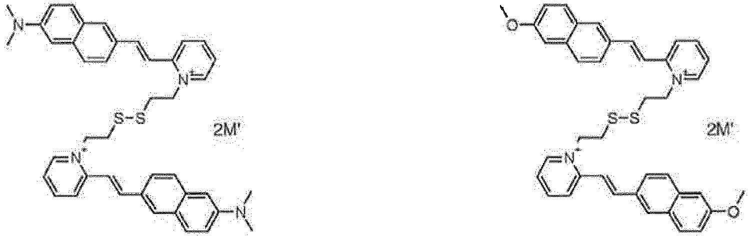 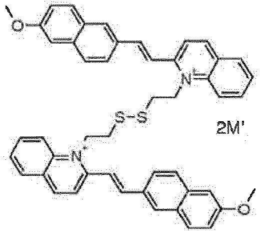 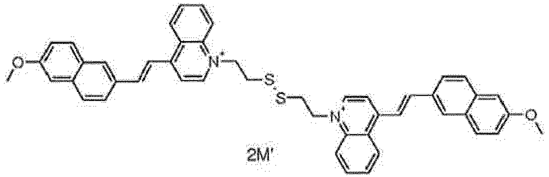 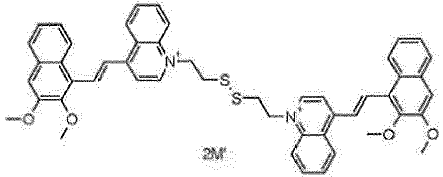 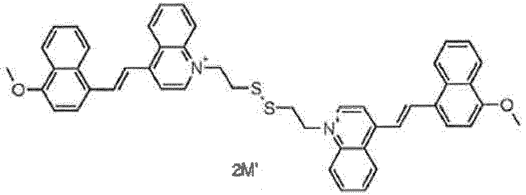 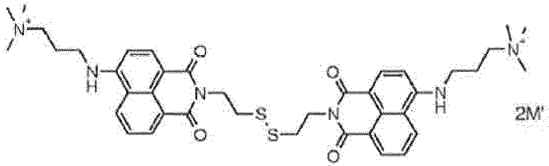 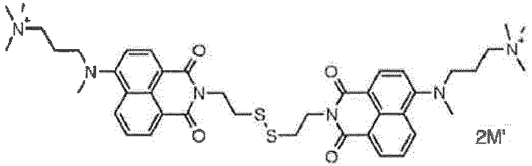 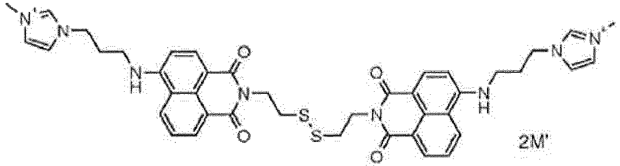 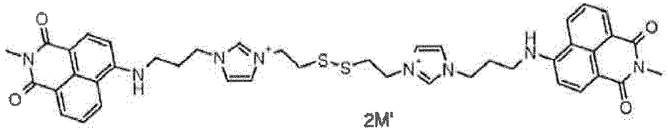 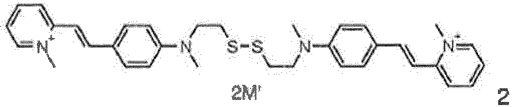 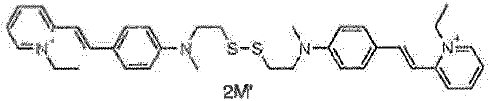 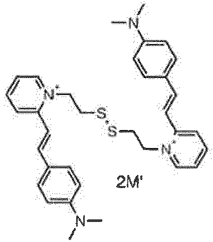 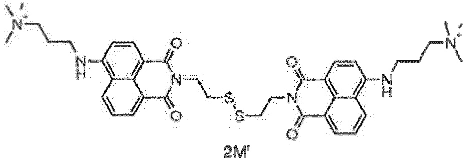 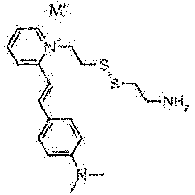 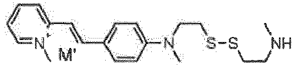 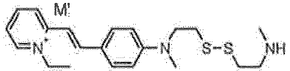 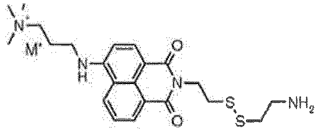 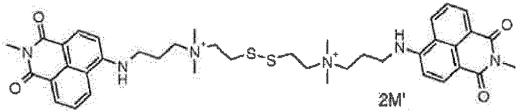 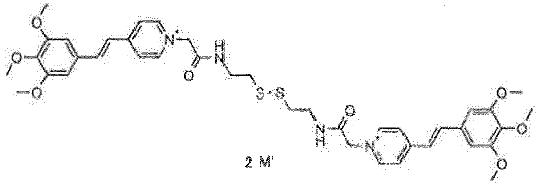 wobei M' für ein anionisches Gegenion steht.

10. Verfahren nach einem der vorhergehenden Ansprüche, **dadurch gekennzeichnet, dass** das Reduktionsmittel aus Cystein, Homocystein, Thiomilchsäure, den Salzen dieser Thiole, Phosphinen, Hydrogensulfit, Sulfiten und Thioglykolsäure sowie den Estern von Thioglykolsäure ausgewählt wird.

11. Verfahren nach einem der vorhergehenden Ansprüche, **dadurch gekennzeichnet, dass** es sich bei den dunklen Keratinmaterialien um Keratinfasern mit einer Tonhöhe kleiner gleich 6, vorzugsweise kleines gleich 4, handelt.

12. Verfahren nach einem der vorhergehenden Ansprüche mit einem zusätzlichen Schritt, der darin besteht, dass man auf die Keratinfasern ein Oxidationsmittel aufbringt.

13. Verfahren nach einem der vorhergehenden Ansprüche, bei dem der Disulfid-Fluoreszenzfarbstoff in einer Menge zwischen 0,001 und 50 Gew.-%, vorzugsweise zwischen 0,005 und 20 Gew.-% und noch weiter bevorzugt zwischen 0,01 und 5 Gew.-%, bezogen auf das Gesamtgewicht der Färbezusammensetzung, vorliegt.

14. Verwendung von Disulfid-Fluoreszenzfarbstoffen gemäß den Ansprüchen 1 bis 9 zum Färben von dunklen menschlichen Keratinfasern mit einer Tonhöhe kleiner gleich 6, insbesondere Haaren.

15. Verwendung nach dem vorhergehenden Anspruch zum Aufhellen von dunklen Keratinfasern mit einer Tonhöhe kleiner gleich 4.

## Claims

1. Method of colouring dark keratin materials, **characterized in that** at least one fluorescent disulphide dye is applied to the materials simultaneously with a reducing agent.

2. Method according to the preceding claim, **characterized in that** a dyeing composition is applied which comprises in an appropriate cosmetic medium at least one fluorescent disulphide dye in the presence of a reducing agent.

3. Method according to either of the preceding claims, **characterized in that** the fluorescent disulphide dye is selected from the dyes of formulae (I) and (II) as defined below:
A-(X)ₚ-Cₛₐₜ-S-S-C'ₛₐₜ-(X')ₚ'-A' (I)
A-(X)ₚ-Cₛₐₜ-S-S-C'ₛₐₜ-(X')ₚ'-D (II),
their organic or inorganic acid salts, optical isomers, geometrical isomers, and the solvates such as hydrates, in which formulae:
• A and A', which are identical or different, each represent a radical containing at least one cationic or non-cationic fluorescent chromophore;
• X and X', which are identical or different, each represent a saturated or unsaturated, linear or branched C₁-C₃₀ hydrocarbon chain which is optionally interrupted and/or optionally terminated at one or two of its ends by one or more divalent groups or combinations thereof selected from:
- -N(R)-, -N⁺(R)(R)-, -O-, -S-, -CO-, -SO₂-where radicals R, which are identical or different, are each selected from a hydrogen or a C₁-C₄ alkyl, hydroxyalkyl or aminoalkyl radical;
- a saturated or unsaturated, fused or non-fused, aromatic or non-aromatic (hetero)cyclic radical optionally comprising one or more identical or non-identical heteroatoms and optionally substituted;
• the indices p and p', which are identical or different, are each 0 or 1;
• Cₛₐₜ and C'ₛₐₜ, which are identical or different, each represent an optionally cyclic, optionally substituted, linear or branched C₁-C₁₈ alkylene chain;
• D corresponds to a radical selected from hydroxyl, hydroxyalkyl, alkoxy, carboxyl, carboxylate, amino, alkylamino and dialkylamino radicals.

4. Method according to the preceding claim, **characterized in that** the fluorescent disulphide dye of formula (I) or (II) comprises a radical A or A', which are identical or different, containing a styryl chromophore.

5. Method according to any one of the two preceding claims, **characterized in that** X and X', which are identical or different, represent a group -N(R)-.

6. Method according to any one of Claims 3 to 5, **characterized in that** A and A', which are identical or different, more preferably identical, represent W-C(R^{c})=C(R^{d})-Ar- or -W-C(R^{c})=C(R^{d}) -Ar, where W represents a heterocycle or a heteroaryl containing a quaternary ammonium; Ar represents a 5- or 6-membered (hetero)aryl radical of phenyl or pyridinium type, or a (hetero)aromatic bicyclic radical of naphthyl, benzopyridinium, indolinyl or benzoindolinyl type, optionally substituted by one or more halogen atoms; by one or more alkyl groups; by one or more hydroxyl groups; by one or more alkoxy groups; by one or more hydroxyalkyl groups; by one or more amino or (di)alkylamino groups; by one or more acylamino groups; or by one or more 5- or 6-membered heteroaryl or heterocycloalkyl groups; and R^{c} and R^{d}, which are identical or different, each represent a hydrogen atom or a C₁-C₄ alkyl group.

7. Method according to any one of Claims 3 to 6, **characterized in that** W is an imidazolium, pyridinium, benzopyridinium, benzimidazolium, quinolinium or pyrazolium.

8. Method according to any one of the preceding claims, **characterized in that** the fluorescent disulphide dye is selected from compounds (III) to (XII) : 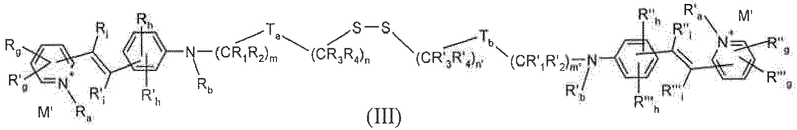 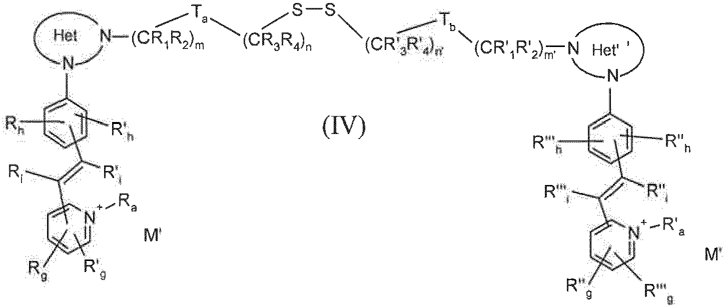 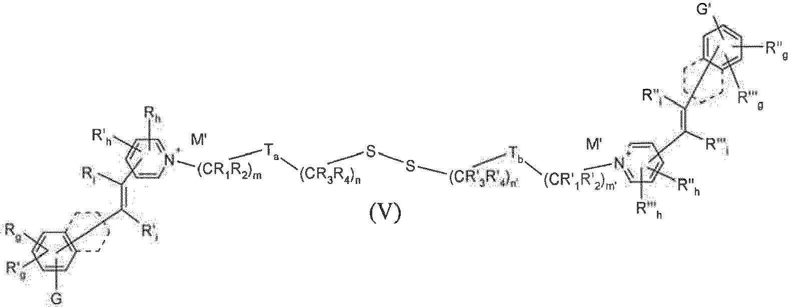 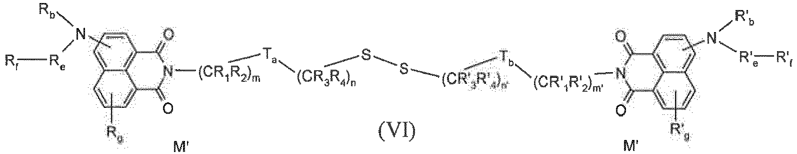 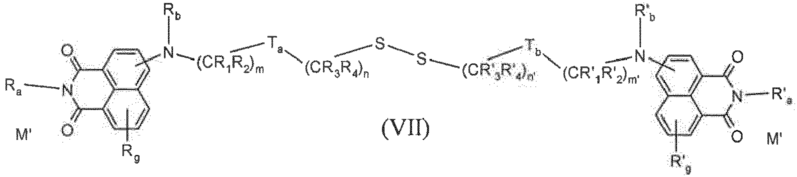 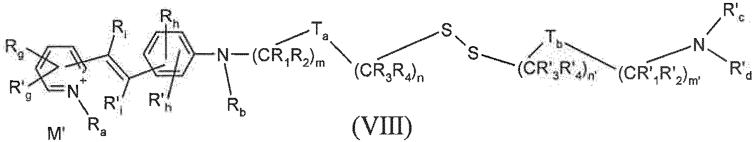 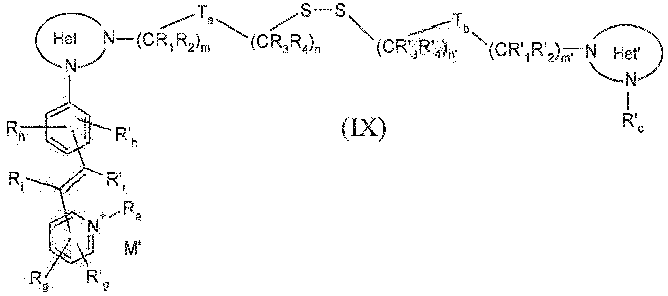 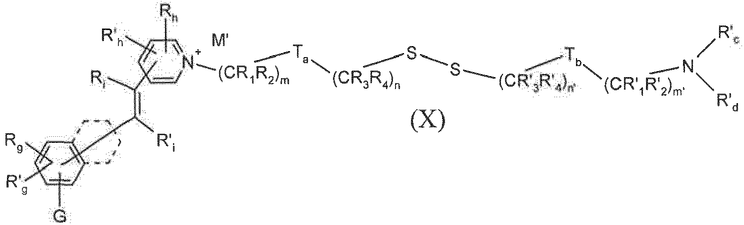 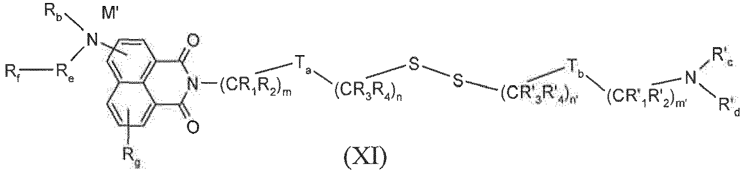 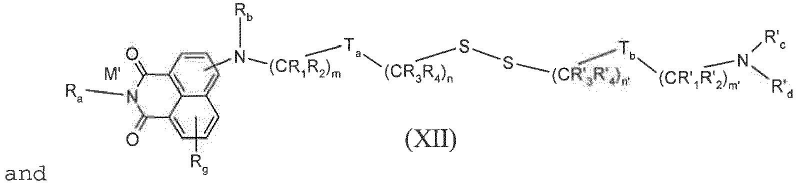 in which
• G and G', which are identical or different, each represent a group -NR_{c}R_{d}, -NR'_{c}R'_{d} or optionally substituted C₁-C₆ alkoxy; preferably G and G' represent respectively a group -NR_{c}R_{d} and -NR'_{c}R'_{d};
• Rₐ and R'ₐ, which are identical or different, represent an aryl(C₁-C₄)alkyl group or a C₁-C₆ alkyl group which is optionally substituted by a hydroxyl or amino group, C₁-C₄ alkylamino or C₁-C₄ dialkylamino, it being possible for said alkyl radicals to form, with the nitrogen atom which carries them, a heterocycle containing from 5 to 7 members, optionally comprising another heteroatom different or not different from nitrogen;
• R_{b} and R'_{b}, which are identical or different, each represent a hydrogen atom, an aryl(C₁-C₄)alkyl group or a C₁-C₆ alkyl group which is optionally substituted;
• R_{c}, R'_{c}, R_{d} and R'_{d}, which are identical or different, each represent a hydrogen atom, an aryl(C₁-C₄)alkyl group, C₁-C₆ alkoxy or a C₁-C₆ alkyl group which is optionally substituted; or two adjacent radicals R_{c} and R_{d} or R'_{c} and R'_{d} which are carried by the same nitrogen atom together form a heterocyclic or heteroaryl group;
• Rₑ and R'ₑ, which are identical or different, each represent a divalent, linear or branched, optionally unsaturated C₁-C₆ alkylenyl hydrocarbon chain;
• R_{f} and R'_{f}, which are identical or different, each represent a quaternary ammonium group (R")(R"')(R"")N⁺- where R", R"' and R"", which are identical or different, each represent a hydrogen atom or a C₁-C₄ alkyl group, or (R")(R"')(R"")N⁺- represents an optionally substituted cationic heteroaryl group, preferably an imidazolinium group which is optionally substituted by a C₁-C₃ alkyl group;
• R_{g}, R'_{g}, R"_{g}, R"'_{g}, Rₕ, R'ₕ, R"ₕ and R"'ₕ, which are identical or different, each represent a hydrogen atom, a halogen atom, an amino, C₁-C₄ alkylamino, C₁-C₄ dialkylamino, cyano, carboxyl, hydroxyl or trifluoromethyl group, an acylamino, C₁-C₄ alkoxy, C₂-C₄ (poly)hydroxyalkoxy, alkylcarbonyloxy, alkoxycarbonyl or alkylcarbonylamino radical, an acylamino, carbamoyl or alkylsulphonylamino radical, an aminosulphonyl radical, or a C₁-C₁₆ alkyl radical optionally substituted by a group selected from C₁-C₁₂ alkoxy, hydroxyl, cyano, carboxyl, amino, C₁-C₄ alkylamino and C₁-C₄ dialkylamino, or the two alkyl radicals carried by the nitrogen atom of the amino group form a heterocycle containing 5 to 7 members and optionally comprising another heteroatom which is identical to or different from that of the nitrogen atom;
or two groups R_{g} and R'_{g}; R"_{g} and R"'_{g}; Rₕ and R'ₕ; or R"ₕ and R"'ₕ, carried by two adjacent carbon atoms, together form a benzo or indeno ring or a fused heteroaryl or fused heterocycloalkyl group, the benzo, indeno, heterocycloalkyl or heteroaryl ring being optionally substituted by a halogen atom, an amino, C₁-C₄ alkylamino, C₁-C₄ dialkylamino, nitro, cyano, carboxyl, hydroxyl or trifluoromethyl group, an acylamino, C₁-C₄ alkoxy, C₂-C₄ (poly)hydroxyalkoxy, alkylcarbonyloxy, alkoxycarbonyl, or alkylcarbonylamino radical, an acylamino, carbamoyl or alkylsulphonylamino radical, an aminosulphonyl radical, or a C₁-C₁₆ alkyl radical which is optionally substituted by a group selected from C₁-C₁₂ alkoxy, hydroxyl, cyano, carboxyl, amino, C₁-C₄ alkylamino or C₁**-**C₄ dialkylamino, or the two alkyl radicals carried by the nitrogen atom of the amino group form a heterocycle containing 5 to 7 members and optionally containing another heteroatom identical to or different from that of the nitrogen atom;
• or, when G represents -NR_{c}R_{d} and G' represents - NR'_{c}R'_{d}, two groups R_{c} and R'_{g}; R'_{c} and R"_{g}; R_{d} and R_{g}; or R'_{d} and R"'_{g} together form a saturated heterocycle or heteroaryl which is optionally substituted by a C₁-C₆ alkyl group;
• Rᵢ, R'ᵢ, R"ᵢ and R"'ᵢ, which are identical or different, each represent a hydrogen atom or a C₁-C₄ alkyl group;
• R₁, R₂, R₃, R₄, R'₁, R'₂, R'₃ and R'₄, which are identical or different, each represent a hydrogen atom or a C₁-C₄ alkyl, C₁-C₁₂ alkoxy, hydroxyl, cyano, carboxyl, amino, C₁-C₄ alkylamino or C₁-C₄ dialkylamino group, it being possible for said alkyl radicals to form, with the nitrogen atom which carries them, a heterocycle containing 5 to 7 members that optionally comprises another heteroatom which is different or not different from the nitrogen;
• Tₐ and T_{b}, which are identical or different, represent i) a covalent □□ bond,
ii) one or more radicals or combinations thereof selected from -SO₂-, -O-,
-S-, -N(R)-, -N⁺(R)(R^{o})-, -CO-, with R and R^{o}, which are identical or different, representing a hydrogen atom or a C₁-C₄ alkyl or C₁-C₄ hydroxyalkyl radical, or an aryl(C₁-C₄) alkyl, iii) or a cationic or non-cationic heteroaryl or heterocycloalkyl radical;
• 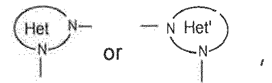 which are identical or different, each represent an optionally substituted heterocyclic group;
• 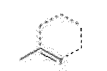 represents an aryl or heteroaryl group which is fused to the phenyl ring; or is absent from the phenyl ring;
• m, m', n and n', which are identical or different, represent an integer of between 0 and 6 inclusive, with m+n and m'+n', which are identical or different, each representing an integer between 1 and 10 inclusive;
M' represents a counterion or an organic or inorganic acid salt.

9. Method according to any one of the preceding claims, **characterized in that** the fluorescent disulphide dye is selected from: 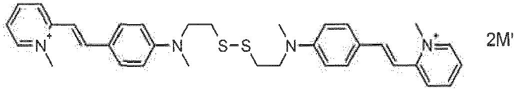 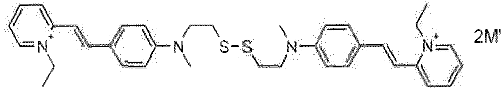 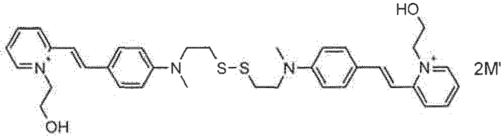 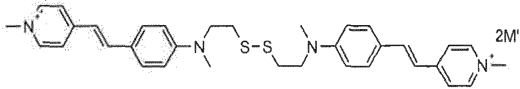 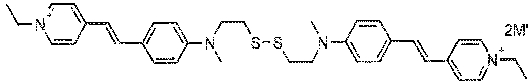 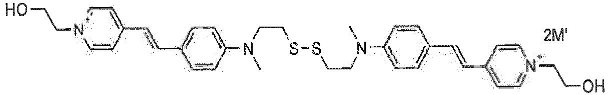 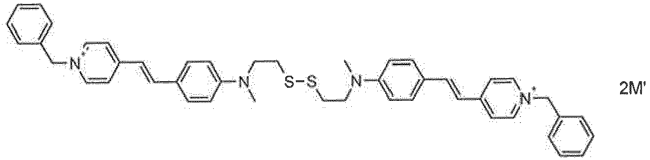 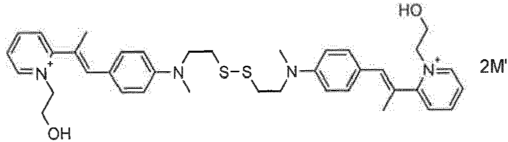 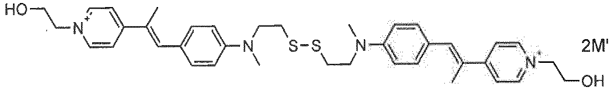 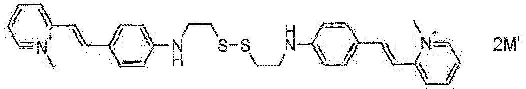 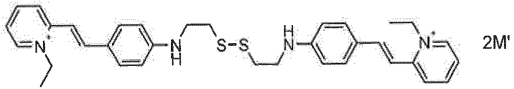 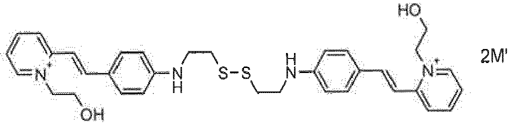 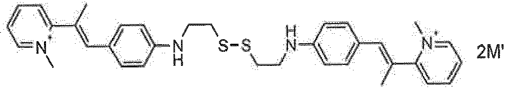 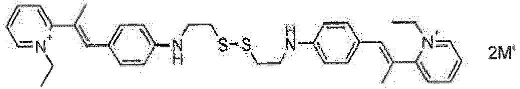 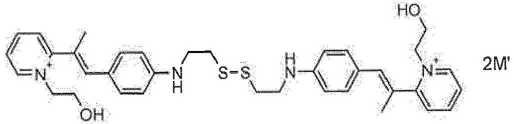 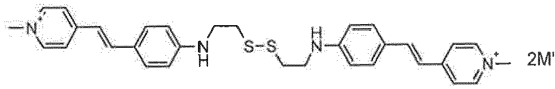 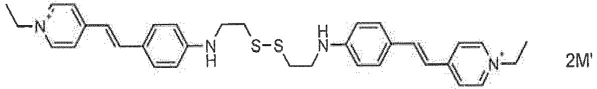 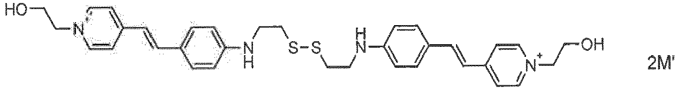 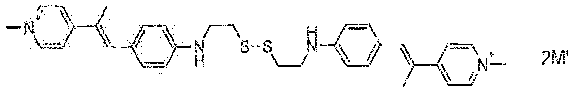 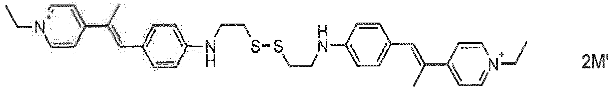 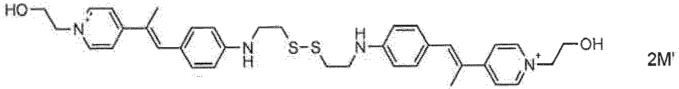 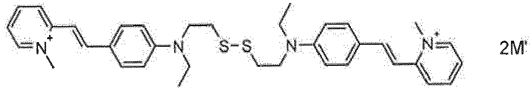 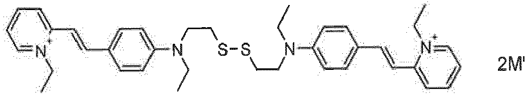 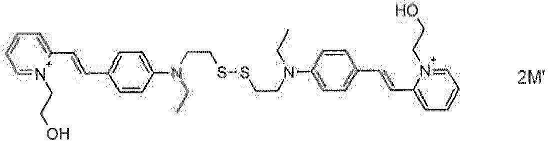 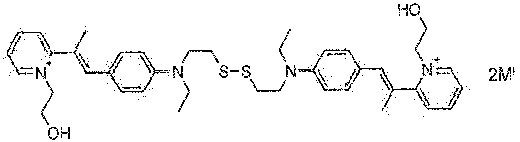 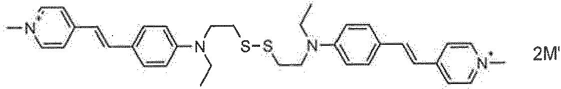 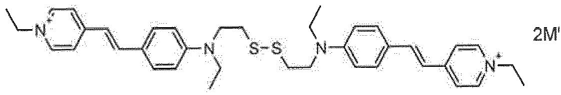 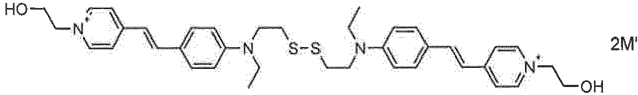 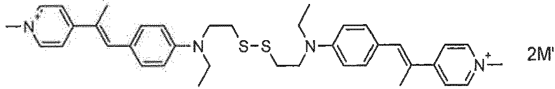 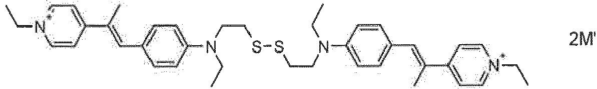 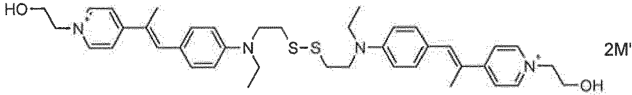 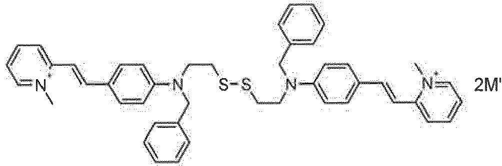 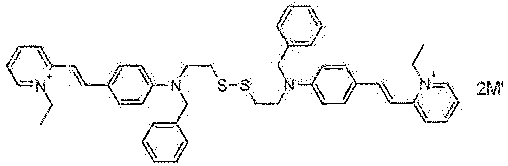 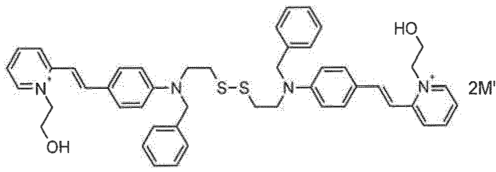 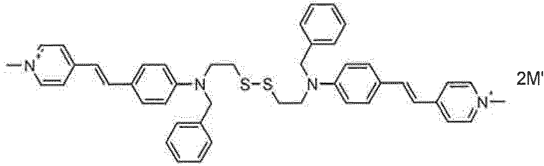 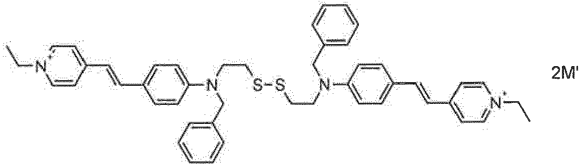 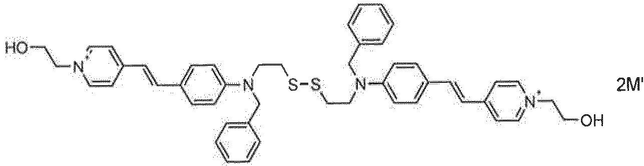 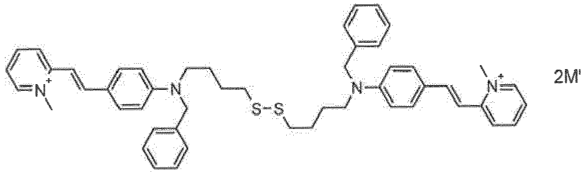 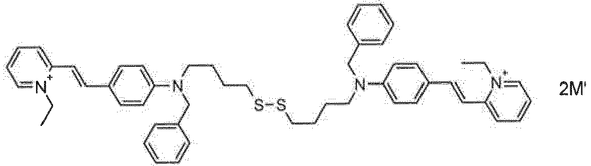 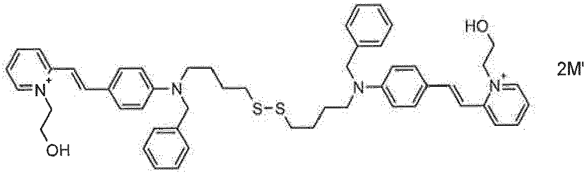 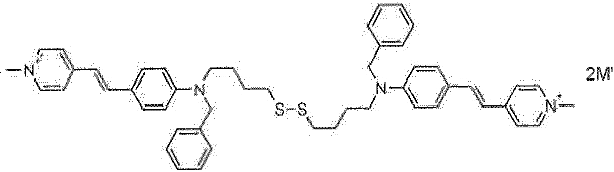 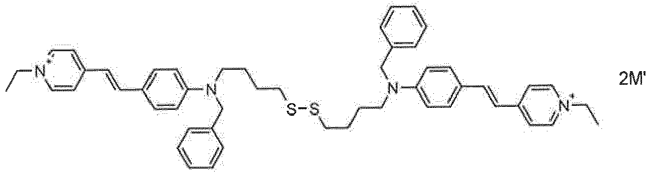 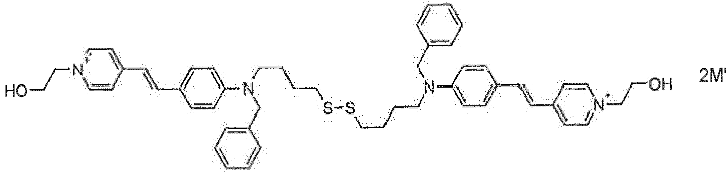 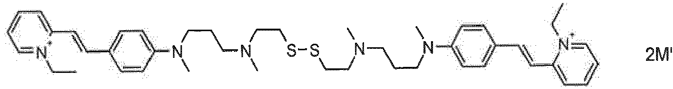 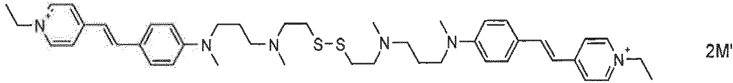 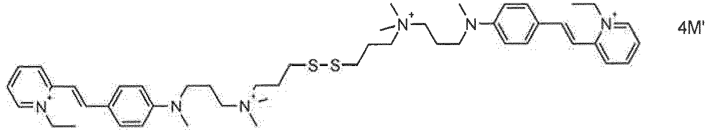 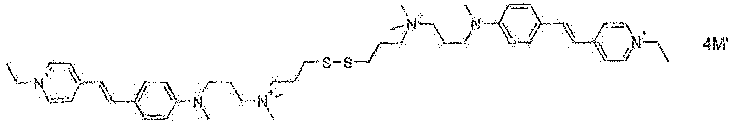 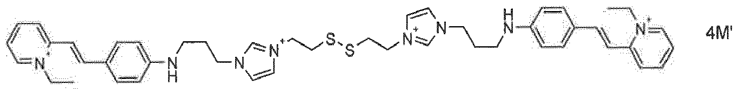 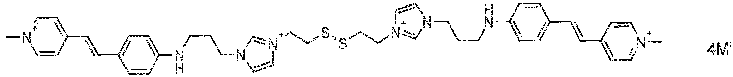 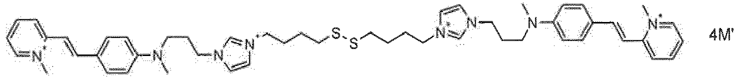 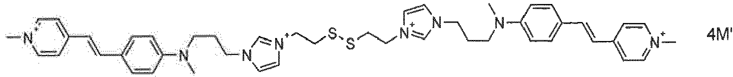 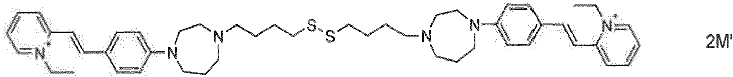 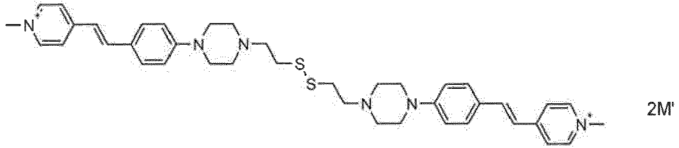 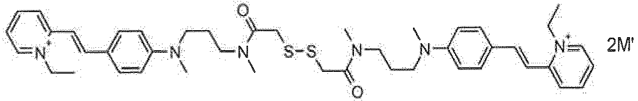 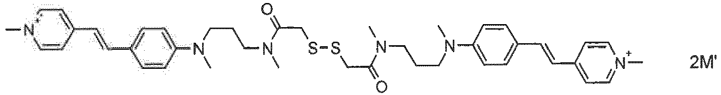 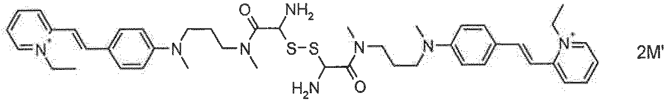 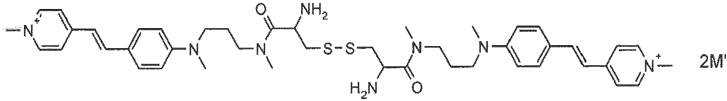 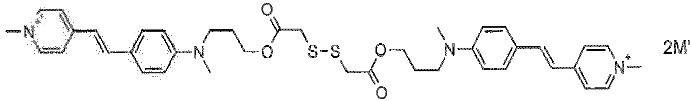 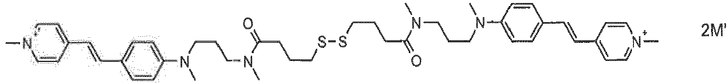 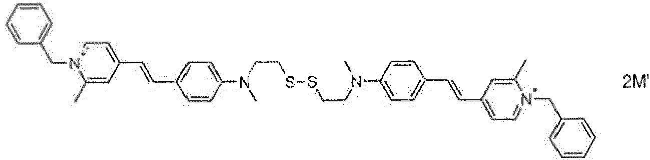 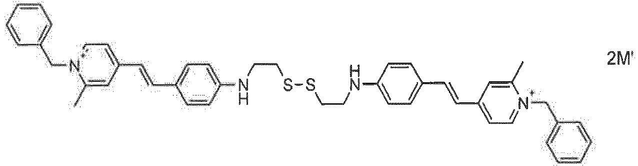 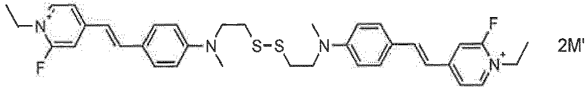 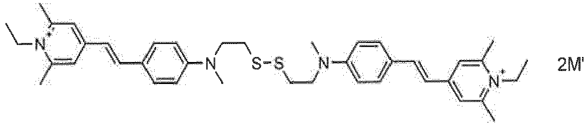 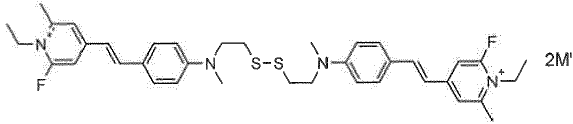 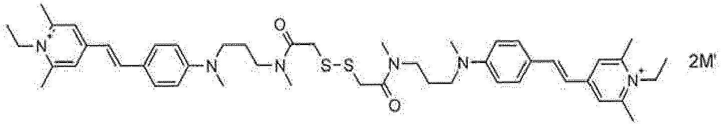 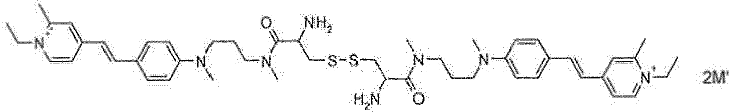 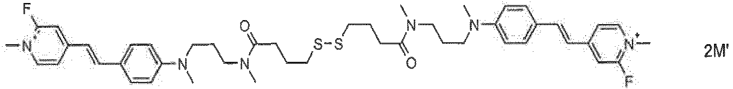 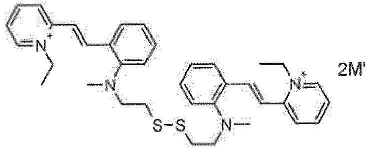 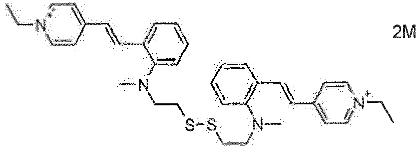 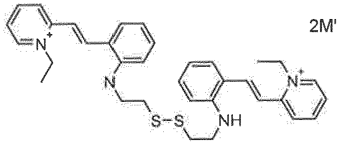 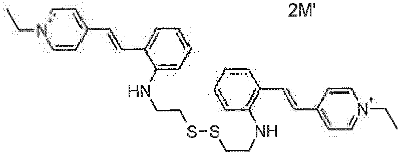 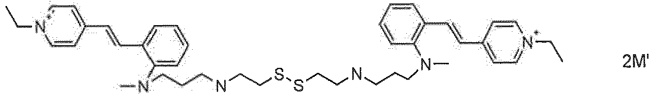 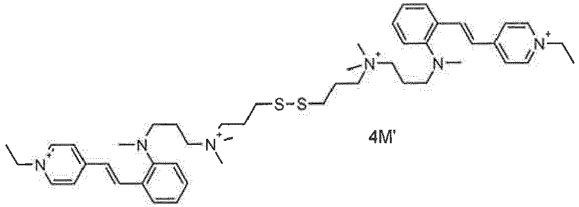 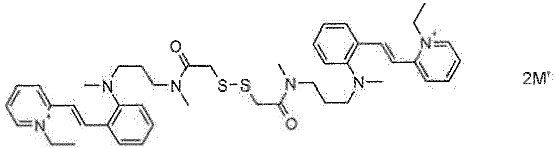 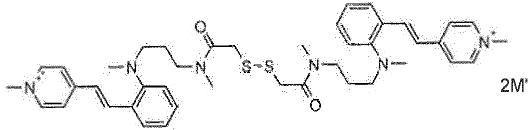 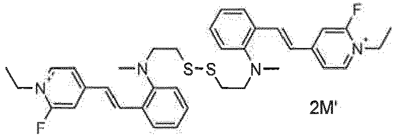 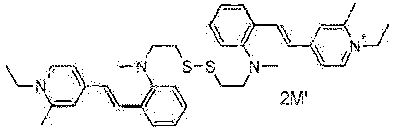 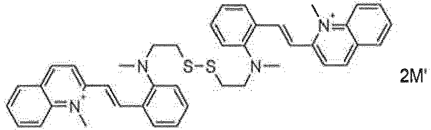 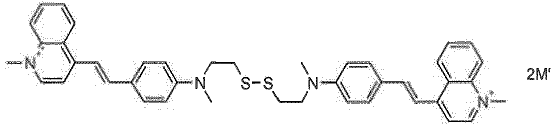 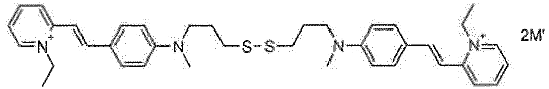 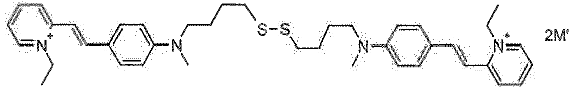 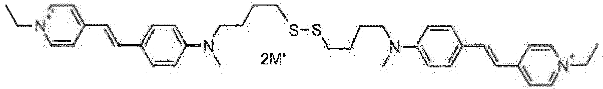 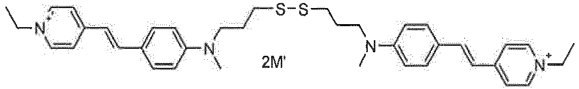 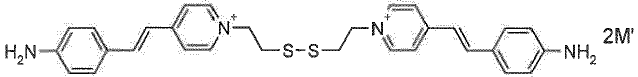 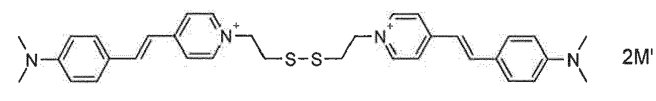 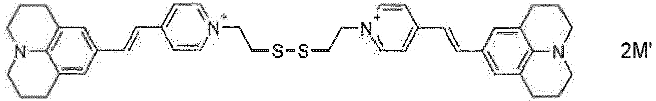 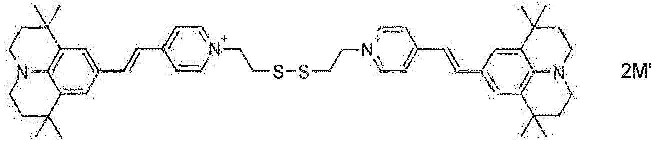 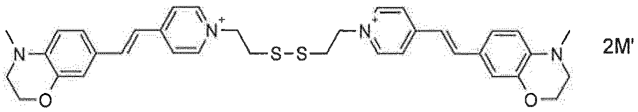 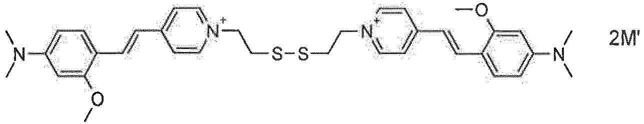 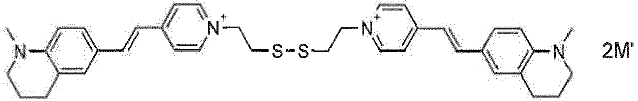 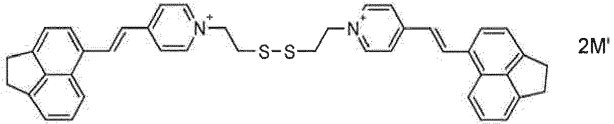 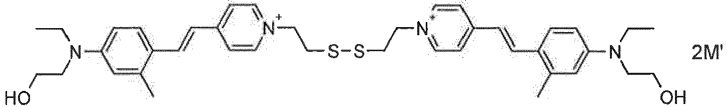 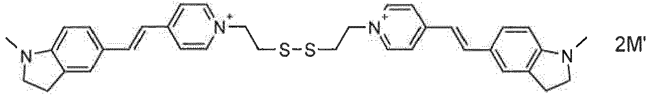 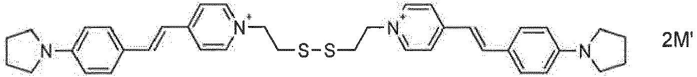 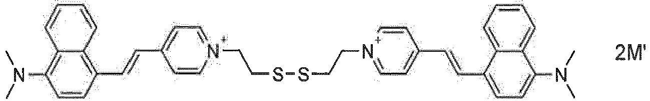 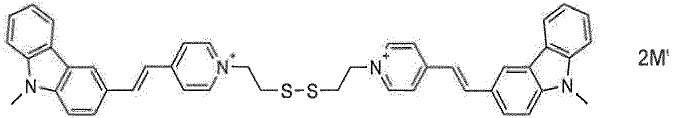 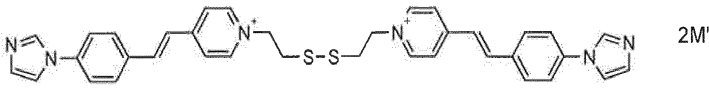 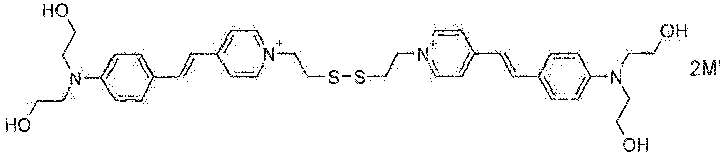 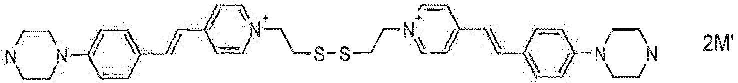 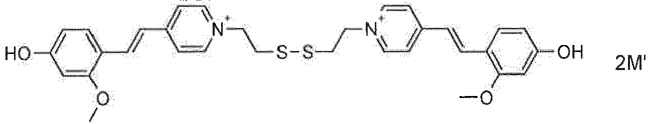 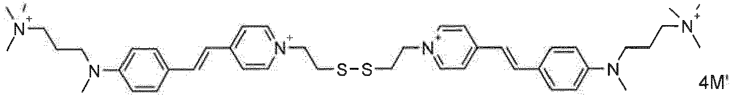 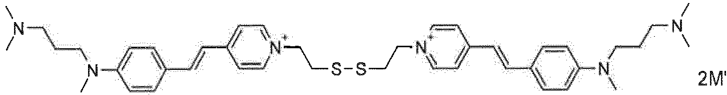 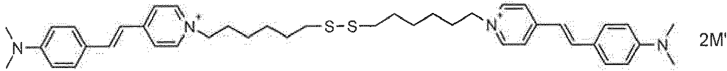 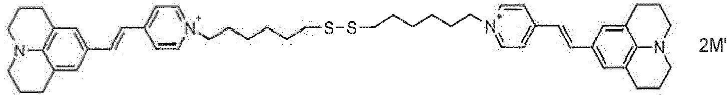 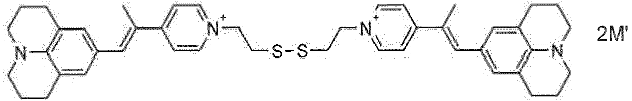 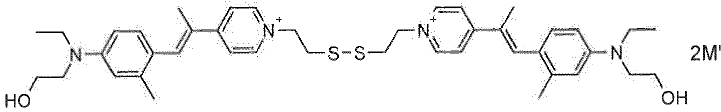 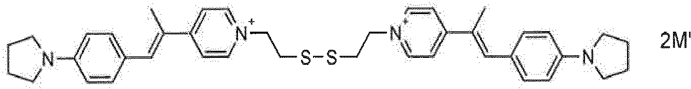 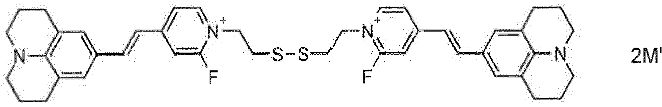 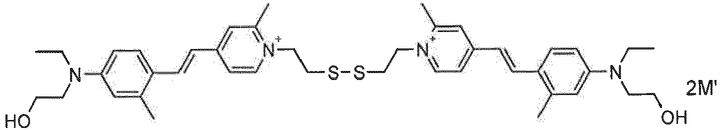 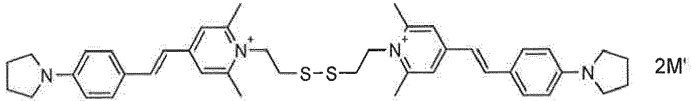 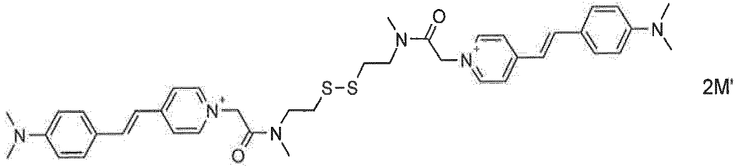 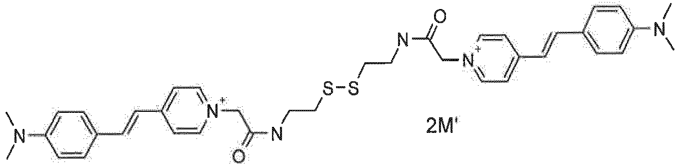 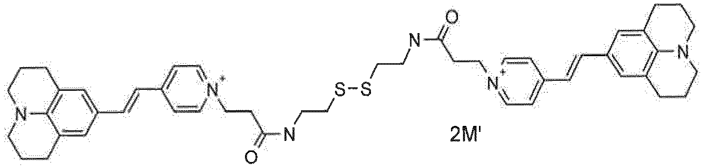 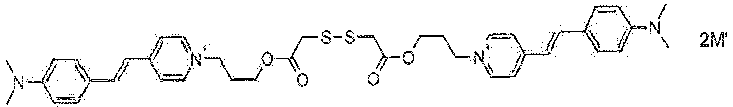 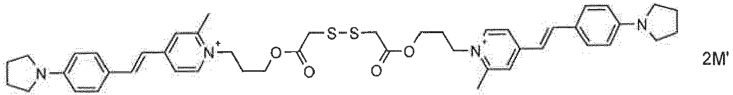 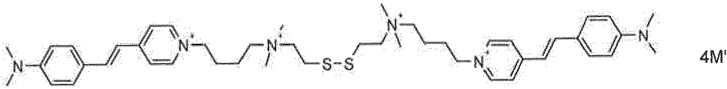 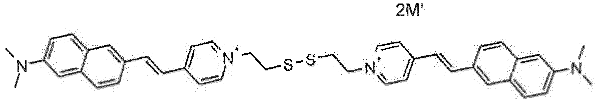 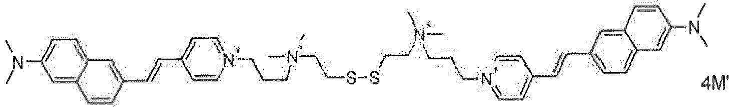 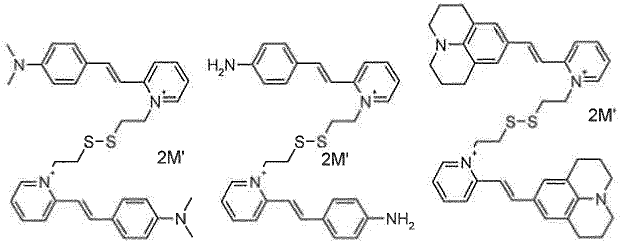 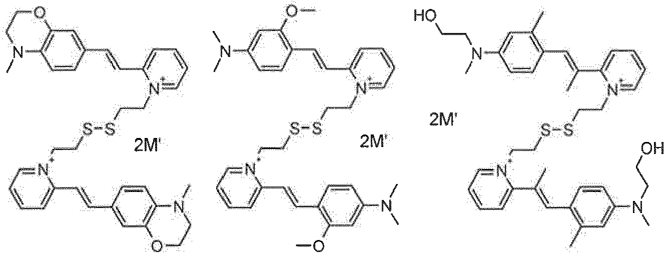 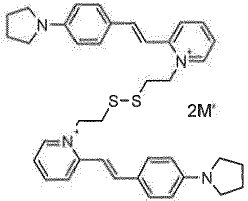 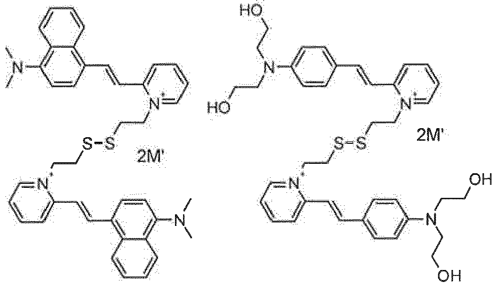 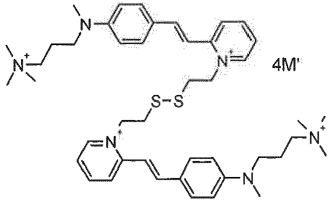 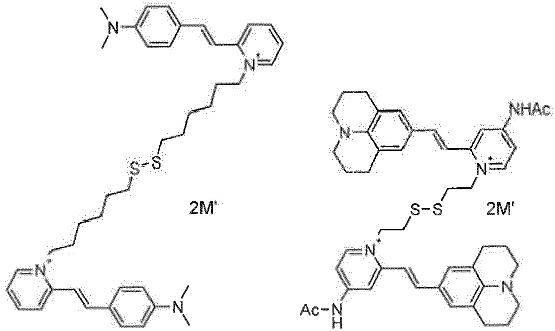 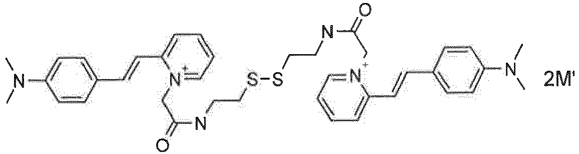 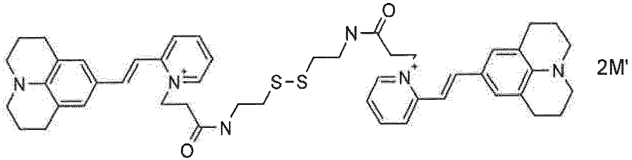 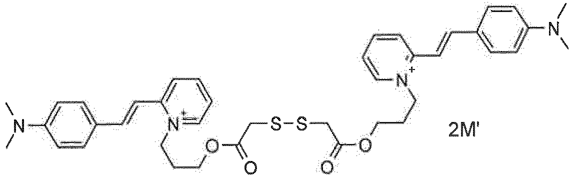 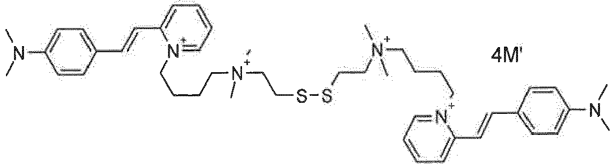 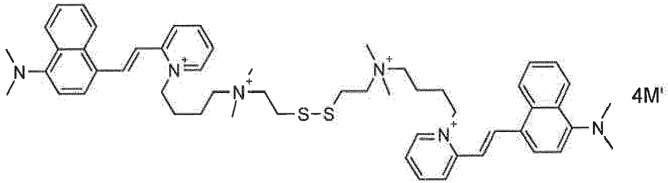 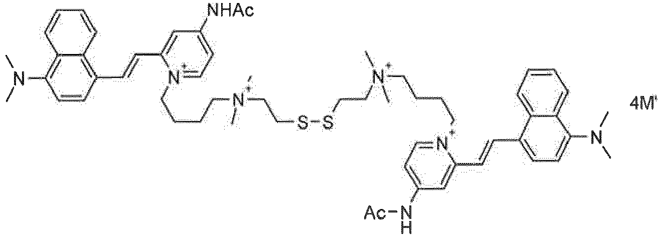 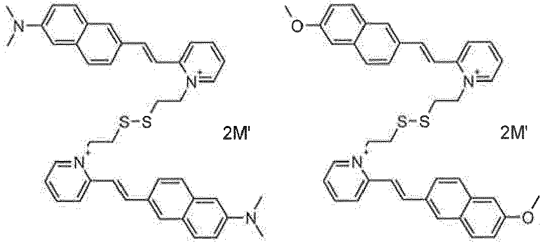 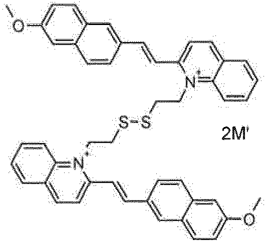 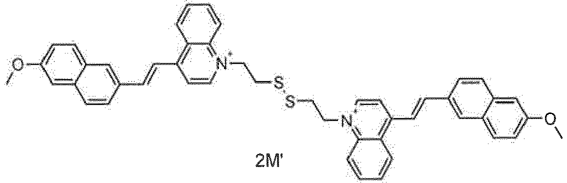 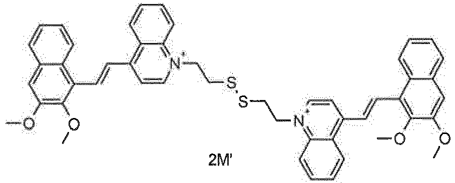 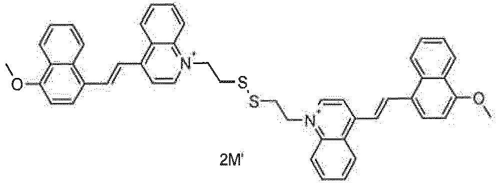 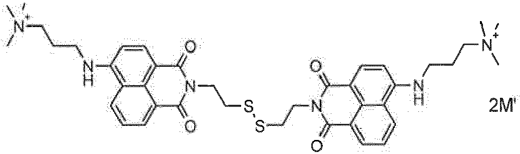 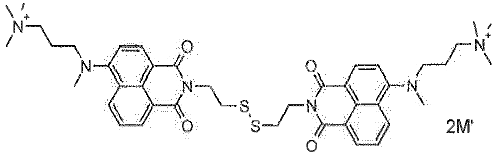 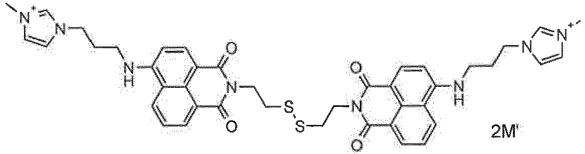 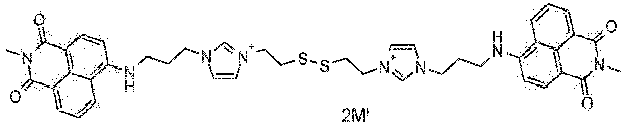 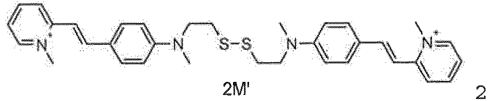 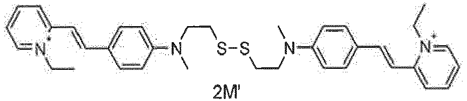 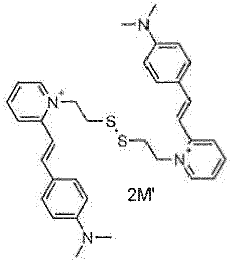 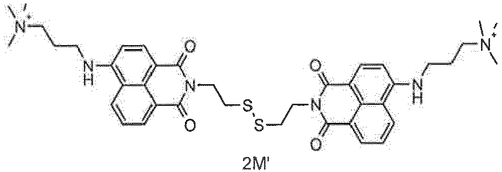 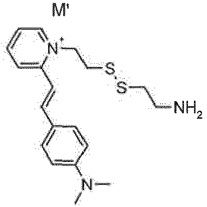 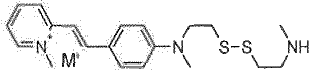 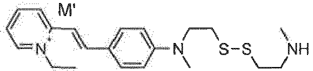 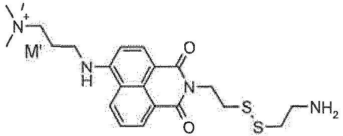 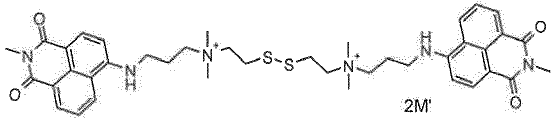 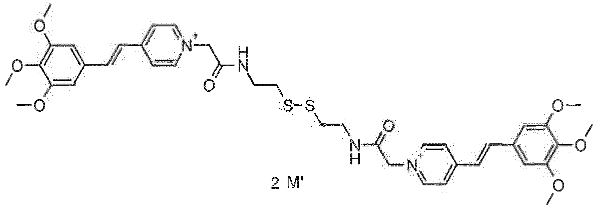 where M' is an anionic counterion.

10. Method according to any one of the preceding claims, **characterized in that** the reducing agent is selected from cysteine, homocysteine, thiolactic acid, the salts of these thiols, phosphines, bisulphite, sulphites and thioglycolic acid, and also the esters of thioglycolic acid.

11. Method according to any one of the preceding claims, **characterized in that** the dark keratin materials are keratin fibres which possess a tone level of less than or equal to 6, preferably less than or equal to 4.

12. Method according to any one of the preceding claims, including an additional step of applying an oxidizing agent to the keratin fibres.

13. Method according to any one of the preceding claims, wherein the fluorescent disulphide dye is present in an amount of between 0.001% and 50% by weight, preferably between 0.005% and 20% by weight and more preferably between 0.01% and 5% by weight, relative to the total weight of the dyeing composition.

14. Use of fluorescent disulphide dyes as defined in Claims 1 to 9 for dyeing dark human keratin fibres with a tone level of less than or equal to 6, especially the hair.

15. Use according to the preceding claim for lightening dark keratin fibres with a tone level of less than or equal to 4.
